# EUROPEAN PATENT APPLICATION

(11) **EP 4 582 428 A1**
(43) Date of publication of application: **09.07.2025**
(21) Application number: 23860281.7
(22) Date of filing: 28.08.2023
(51) Int. Cl.: C07D 487/04, A61K 31/519, A61P 1/04, A61P 1/16, A61P 3/06, A61P 9/10, A61P 19/02, A61P 19/06, A61P 21/02, A61P 25/00, A61P 25/16, A61P 25/28, A61P 29/00, A61P 37/02, A61P 43/00, C07D 519/00

(54) **PYRAZOLOPYRIMIDINE COMPOUND AND PHARMACEUTICAL USE THEREOF**

(30) Priority: 29.08.2022 JP 2022135949; 24.02.2023 JP 2023027426
(71) Applicant: Japan Tobacco Inc., Tokyo 105-6927 (JP)
(72) Inventor: OHBA, Yusuke, Takatsuki-shi, Osaka 569-1125 (JP); ADACHI, Kaoru, Takatsuki-shi, Osaka 569-1125 (JP); SAKURAI, Kentaro, Takatsuki-shi, Osaka 569-1125 (JP); SATO, Shimpei, Takatsuki-shi, Osaka 569-1125 (JP); ITO, Shunya, Takatsuki-shi, Osaka 569-1125 (JP)
(74) Representative: Vossius & Partner Patentanwälte Rechtsanwälte mbB
(86) International application number: PCT/JP2023/030980
(87) International publication number: WO 2024/048519

(57) **Abstract**

A pyrazolopyrimidine compound, or a pharmaceutically acceptable salt thereof, having NLRP3 inflammasome inhibitory activity, a pharmaceutical composition comprising the same, and their medical use, etc., are provided.

A compound of Formula [IA]: 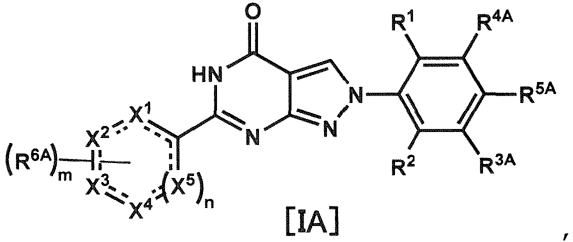 or a pharmaceutically acceptable salt thereof,
wherein each symbol in the formula is defined in the specification.

## Description

### TECHNICAL FIELD

The present invention relates to pyrazolopyrimidine compounds, or pharmaceutically acceptable salts thereof, having NLRP3 inflammasome inhibitory activity, pharmaceutical compositions comprising the same, and medical use thereof, etc.

### BACKGROUND ART

NLRP3 (NOD-, LRR-, and pyrin domain-containing protein 3) is a pattern recognition receptor that belongs to an NLR (NOD-like receptors) family, and is also expressed in non-immune cells such as glomerular epithelial cells and tubular epithelial cells as well as phagocytes such as macrophage and microglia.

NLRP3 recognizes DAMPs (Danger Associated Molecular Patterns) which are a molecular pattern specific to cellular damage factors, such as ATP, HMGB1, S100, urate crystals, and silica, and PAMPs (Pathogen Associated Molecular Patterns) which are a molecular pattern specific to pathogenic microorganisms, such as viruses, bacteria, and fungi, and binds to these molecules to be activated.

Activated NLRP3 associates with an adaptor protein, ASC (Apoptosis-associated speck-like protein containing a caspase recruitment domain), and a cysteine protease, caspase 1, by protein-protein interaction to form an NLRP3 inflammasome, which is a cellular protein complex. The formation of an NLRP3 inflammasome converts caspase 1 in the complex into its activated form, and the activated caspase 1 converts proIL-1β, which is a precursor of a proinflammatory cytokine, IL-1β, into an activated form of IL-1β, while it also converts proIL-18, which is a precursor of IL-18, into an activated form of IL-18. The activated IL-1β secreted outside the cell induces proinflammatory cytokine-chemokine production by surrounding cells, and activates immune cells such as T cells, which causes inflammatory reactions.

In multiple sclerosis patients, the increase of the amount of DAMPs was observed in the brain and cerebral spinal fluid (Non Patent Literature 1), and the increase of the expression level of caspase 1 in involved sites and the increase of the amount of IL-1β in cerebral spinal fluid were also observed (Non Patent Literature 2). It has been reported that activated microglia was present in involved sites during the chronic progressive phase of this disease (Non Patent Literature 3), and the activated microglia stimulated by DAMPs produced proinflammatory cytokine such as IL-1β, which induced nerve inflammation and nerve disorder (Non Patent Literature 4). Thus, an NLRP3 inflammasome is considered to get involved in the expression of disease states of multiple sclerosis.

MOG₃₅₋₅₅EAE model mice prepared by sensitization of Myelin Oligodendrocyte Glycoprotein (MOG) expressed impairment of motor function as seen in multiple sclerosis. The onset of the impairment of motor function was inhibited in NLRP3-knockout mice in the MOG₃₅₋₅₅EAE model (Non Patent Literature 5). Demyelination of central nerve as seen in multiple sclerosis was expressed in cuprizone-model mice prepared by administration of a copper-chelate compound, cuprizone, to mice, while the progress of demyelination was delayed in NLRP3-knockout mice in the cuprizone model (Non Patent Literature 6). Administration of an NLPR3 inflammasome inhibitor, JC-171, after the onset inhibited the impairment of motor function in the MOG₃₅₋₅₅EAE model (Non Patent Literature 7). Thus, an NLRP3 inflammasome inhibitor is considered to become a drug for treating multiple sclerosis.

The increase of the expression of NLRP3 inflammasome-related genes has been reported in the kidney of patients suffering from chronic kidney disease (Non Patent Literatures 8, 9). Further, the inhibitory activity of proteinuria and tubulointerstitial fibrosis by NLRP3-knockout has been reported in a non-clinical chronic kidney disease model, i.e., a 5/6 kidney-enucleated model (Non Patent Literature 10). Accordingly, an NLRP3 inflammasome inhibitor is considered to become a drug for treating chronic kidney disease.

The increase of the expression of NLRP3 inflammasome-related genes has been reported in the intestine of patients suffering from inflammatory bowel disease (for example, ulcerative colitis and Crohn's disease) (Non Patent Literature 11). It has been reported that IL-1β produced by the activation of NLRP 3 was increased in the intestinal mucosa of IBD patients, and that the increased IL-1β secretion from the colonic region was positively correlated with the deterioration of the disease state (Non Patent Literature 11). It has also been reported that the dysfunction of CARD8, which negatively regulates inflammasome activity, increases susceptibility to Crohn's disease, and that the activation of NLRP3 inflammasome enhances IL-1β production from monocytes (Non Patent Literature 12). The suppression of intestinal pathology by NLRP3 deficiency has been reported in TNBS-induced colitis model, a colitis model (Non Patent Literature 13). Accordingly, an NLRP3 inflammasome inhibitor is considered to become a drug for treating inflammatory bowel disease.

The increase of the expression of NLRP3 inflammasome-related genes has been reported in the arteriosclerotic region of coronary arteries of patients suffering from myocardial infarction (Non Patent Literature 14). In addition, the suppressed lesion formation by NLRP3-knockout has been reported in low-density lipoprotein receptor (LDL) receptor-deficient mice fed high-fat diet, an arteriosclerosis model (Non Patent Literature 15). Accordingly, an NLRP3 inflammasome inhibitor is considered to become a drug for treating arteriosclerosis.

Cryopyrin-associated periodic syndrome (CAPS), a generic name of autoinflammatory diseases caused by activating mutation of NLRP3 gene, is classified into 3 disease types as follows: a mild disease type of familial cold autoinflammatory syndrome (FCAS), a moderate disease type of Muckle-Wells syndrome (MWS), a severe disease type of chronic infantile neurologic cutaneous and articular syndrome (CINCA) or Neonatal onset multisystem inflammatory disease (NOMID) (Non Patent Literature 16). More than 200 mutations in NLRP3 gene have been reported in CAPS (Non Patent Literature 17). These NLRP3 gene mutations cause the formation and activation of NLRP3 inflammasome even in the absence of an activation signal. Mice expressing CAPS-related NLRP3 mutations exhibit systemic lethal inflammation dependent on IL-1β and IL-18 which are NLRP3 inflammasome and a downstream signal transduction molecule (Non Patent Literature 18). In a mouse strain expressing CAPS-related NLRP3 mutations, CY-09, an NLRP3 inflammasome inhibitor, suppressed systemic lethal inflammation and improved the survival (Non Patent Literature 19). Accordingly, an NLRP3 inflammasome inhibitor is considered to become a drug for treating CAPS.

The increase of the expression of NLRP3 inflammasome-related genes has been reported in liver tissues of patients suffering from nonalcoholic steato-hepatitis (NASH) (Non Patent Literature 20). In addition, the suppressed hepatic fibrogenesis by NLRP3-knockout has been reported in a choline deficient amino acid defined diet fed model, an NASH model (Non Patent Literature 20). Accordingly, an NLRP3 inflammasome inhibitor is considered to become a drug for treating NASH.

In gout and gouty arthritis, urate crystals deposited in the joint and periarticular tissues induce inflammation (Non Patent Literature 21). Urate crystals activate macrophage NLRP3 to produce IL-1β and IL-18 (Non Patent Literature 22). OLT1177, an NLRP3 inflammasome inhibitor, suppressed arthritis in an intra-articular urate-injected arthritis model (Non Patent Literature 23). Accordingly, an NLRP3 inflammasome inhibitor is considered to become a drug for treating gout and gouty arthritis.

The increase of the expression of NLRP3 inflammasome-related genes has been reported in joint synovium, peripheral-blood mononuclear cells of patients suffering from rheumatoid arthritis (Non Patent Literature 24). In addition, the increase of the expression of NLRP3 inflammasome-related genes in synovium has been reported in collagen-induced arthritis, a model of rheumatoid arthritis (Non Patent Literature 25). Accordingly, an NLRP3 inflammasome inhibitor is considered to become a drug for treating rheumatoid arthritis.

It has been reported that trinitrochlorobenzene, which induces contact dermatitis, increased IL-1β production from human skin keratinocytes via NLRP3 activation, and that NLRP3 knockout inhibits development of dermatitis in a trinitrochlorobenzene-induced dermatitis model, a model of contact dermatitis (Non Patent Literature 26). Accordingly, an NLRP3 inflammasome inhibitor is considered to become a drug for treating contact dermatitis.

The increase of the expression of NLRP3 inflammasome-related genes has been reported in the tear fluid and ocular surface of patients suffering from dry eye (Non Patent Literatures 27 and 28). In addition, it has been reported that increased expression of NLRP3 inflammasome-related genes and increased IL-1β production were observed when hypertonic stress was applied to cultured human corneal epithelial cells to induce a dry eye condition, and that IL-1β production was suppressed by knockdown of NLRP3 gene (Non Patent Literature 28). Accordingly, an NLRP3 inflammasome inhibitor is considered to become a drug for treating dry eye.

The increase of the expression of ASC domain of NLRP3 inflammasome has been reported in macrophages and neutrophils infiltrated into myocardial tissue of patients suffering from acute myocardial infarction (Non Patent Literature 29). In addition, it has been reported that the increased expression of NLRP3 inflammasome-related genes were observed in the infarct site in an ischemia-reperfusion model, a model of myocardial infarction, and that knockdown of NLRP3 gene decreased the infarct area and suppressed the reduction of myocardial contractility (Non Patent Literature 30). Accordingly, an NLRP3 inflammasome inhibitor is considered to become a drug for treating ischemic heart disease such as acute myocardial infarction.

It has been reported that the expression of IL-1β or IL-18 was increased in sera and glomeruli of patients with systemic lupus erythematosus (SLE) (Non Patent Literature 31, 32), and that the expression of NLRP3 gene and the production of IL-1β were increased in the macrophages (Non Patent Literature 33). In Nlrp3-R258W mice, which have an activating mutation of NLRP3 gene, lupus nephritis-like symptoms caused by pristane administration were exacerbated (Non Patent Literature 34). Accordingly, an NLRP3 inflammasome inhibitor is considered to become a drug for treating SLE.

In addition to the above diseases, diseases for which an NLRP3 inflammasome inhibitor is expected to be effective include systemic juvenile idiopathic arthritis (Non Patent Literature 35), recurrent pericarditis (Non Patent Literature 36), adult onset Still's disease (for example, hemophagocytic lymphohistiocytosis and macrophage activation syndrome) (Non Patent Literature 37), Schnitzler syndrome (Non Patent Literature 38), deficiency of the IL-1 receptor antagonist (Non Patent Literature 39), familial Mediterranean fever (Non Patent Literature 40), mevalonate kinase deficiency (Non Patent Literature 40), hyper IgD syndrome (Non Patent Literature 40), TNF receptor-associated periodic syndrome (Non Patent Literature 40), Behcet's disease (Non Patent Literature 41), lung cancer (Non Patent Literature 42) and the like. It has been reported that anti-IL-1β antibody such as canakinumab and IL-1 inhibitor such as rilonacept are effective for the treatment of these diseases. Since NLRP3 inflammasome is involved in the production of proinflammatory cytokines such as IL-1β, an NLRP3 inflammasome inhibitor is considered to become a drug for treating these diseases.

It is has been reported that the NLRP3 rs10733113 genotype is significantly increased in patients with psoriasis and increases psoriasis susceptibility (Non Patent Literature 43). In addition, NLRP3 deficiency has been reported to suppress psoriatic symptoms in an IL-23 induced psoriasis model, a psoriasis model (Non-patent document 44). Accordingly, an NLRP3 inflammasome inhibitor is considered to become a drug for treating psoriasis.

Gout, atherosclerosis (arteriosclerosis), and chronic kidney disease, which are associated with NLRP3 inflammasome activation, involve hypertension. NLRP3 deficiency has been reported to suppress hypertension in a mouse model of left renal artery stenosis (Non Patent Literature 45). In addition, MCC950, an NLRP3 inflammasome inhibitor, has been reported to suppress hypertension in a mouse model of deoxycorticosterone acetate-salt (Non Patent Literature 46). Accordingly, an NLRP3 inflammasome inhibitor is considered to become a drug for treating hypertension.

It has been reported that NLRP3 expression is enhanced in fibrovascular membranes of patients with diabetic retinopathy (Non Patent Literature 47). In addition, NLRP3 expression is increased in a STZ-induced retinopathy model, a model of diabetic retinopathy (Non Patent Literature 48). In this model, it has been reported that decreased NLRP3 expression by NLRP3 shRNA exhibits decreased secretions of IL-1β and VEGF, increased ganglion cell mass, and recovery of retinal damage (Non Patent Literature 49). Thus, an NLRP3 inflammasome inhibitor is considered to become a drug for treating diabetic retinopathy.

NLRP3 inflammasome activation occurs in the brain of Alzheimer's disease patients, MCI (mild cognitive impairment) patients, and APP/PS1 mice, a model mouse of Alzheimer's disease. NLRP3 deficiency in APP/PS1 mice suppresses the development of spatial memory impairment (Non Patent Literature 50). MCC950, an NLRP3 inhibitor, suppresses NLRP3 activation in microglia and improves cognitive dysfunction in APP/PS1 mice (Non Patent Literature 51). Thus, an NLRP3 inflammasome inhibitor is considered to become a drug for treating Alzheimer's disease and MCI.

In the substantia nigra of Parkinson's disease patients and mice injected with α-synuclein PFF (pre-formed fibril), a pathological model of Parkinson's disease, increased expression of NLRP3 inflammasome-related molecules and NLRP3 inflammasome activation occur in microglia (Non Patent Literature 52). In α-synuclein PFF injected mice, MCC950, an NLRP3 inhibitor, inhibits NLRP3 activation in the substantia nigra and suppresses neuronal death of dopamine neurons in the substantia nigra (Non Patent Literature 52). Thus, an NLRP3 inflammasome inhibitor is considered to become a drug for treating Parkinson's disease.

In patients with Huntington's disease, cerebrospinal fluid levels of IL-1β, an NLRP3 inflammasome-associated cytokine, are increased (Non Patent Literature 53). The expression level of NLRP3 inflammasome is increased in the striatum of R6/2 mice, a model of Huntington's disease (Non Patent Literature 54). MCC950, an NLRP3 inhibitor, inhibits NLRP3 inflammasome activation in the striatum of R6/2 mice, suppresses neuronal death in the striatum, and suppresses symptom progression (Non Patent Literature 55). Thus, an NLRP3 inflammasome inhibitor is considered to become a drug for treating Huntington's disease.

The expressions of the NLRP3 inflammasome, IL-18, and active caspase 1 are increased in the spinal cord of patients with amyotrophic lateral sclerosis (ALS) (Non Patent Literature 56). In the spinal cord of SOD1G93A mice and TDP-43Q331K mice, which are ALS model mice, mRNA expressions of IL-1β, Nlrp3, Pycard, and Casp1 are increased (Non Patent Literature 57). MCC950, an NLRP3 inhibitor, inhibits SOD1G93A and TDP-43 protein-induced NLRP3 activation in microglia and decreases IL-1β production (Non-patent Document 57). In SOD1G93A mice, deficiency of IL-1β or caspase 1 prolongs survival time, and administration of IL-1β receptor antibody suppresses disease progression and prolongs survival time (Non Patent Literature 58). Thus, an NLRP3 inflammasome inhibitor is considered to become a drug for treating ALS.

The expression level of NLRP3 inflammasome is increased in brain tissue and cerebrospinal fluid of patients with traumatic brain injury (TBI) (Non Patent Literatures 59 and 60). In the brain tissue of TBI model rats, the expression level of NLRP3 inflammasome is increased, and the expression levels of IL-1β and IL-18 are also increased (Non Patent Literature 61). MCC950, an NLRP 3 inhibitor, inhibits IL-1β production in TBI model mice and suppresses the development of neurological symptoms after brain injury (Non Patent Literature 62). Thus, an NLRP3 inflammasome inhibitor is considered to become a drug for treating TBI.

In cerebral infarct patients; middle cerebral artery occlusion (MCAO) mice, a model of cerebral infarct; and intracerebral bleeding model rats, the expressions of NLRP3 inflammasome, IL-1β, and IL-18 are increased in the brain tissue (Non Patent Literatures 63 and 64). In addition, MCC950, an NLRP 3 inhibitor, showed neuroprotective effects in the MCAO model and intracerebral bleeding model rats. Thus, an NLRP3 inflammasome inhibitor is considered to become a drug for treating cerebral infarct and intracerebral bleeding.

NLRP inflammasome expression is increased in brain tissue of patients with temporal lobe epilepsy and in Pilocarpine-induced epileptic model mice (Non Patent Literatures 65 and 66). In addition, in the pilocarpine-induced epilepsy model mice, NLRP3 inflammasome deficiency and administration of MCC950, an NLRP3 inhibitor, suppress apoptosis of hippocampal neurons, which causes the development of epilepsy (Non Patent Literature 66). Thus, an NLRP3 inflammasome inhibitor is considered to become a drug for treating epilepsy.

In peripheral blood of depressive illness patients, the expression level of NLRP3 inflammasome, the IL-1β level, and the IL-18 level are increased, and the IL-1β level correlates with the depression symptom score (Non Patent Literature 67). In an LPS-induced model, a chronic stress-induced model, or a social defeat model, which are pathological models of depressive illness, the expression level of NLRP3 inflammasome, IL-1β, or IL-18 in brain tissue is increased, and NLRP3 inflammasome is activated (Non Patent Literatures 68, 69 and 70). In the pathological models, administration of MCC950, an NLRP3 inhibitor, or NLRP3 deficiency improves depressive symptoms (Non Patent Literatures 69 and 70). Thus, an NLRP3 inflammasome inhibitor is considered to become a drug for treating depressive illness.

NLRP3 inflammasome expression and IL-1β and IL-18 levels are increased in peripheral blood of patients with autism spectrum disorder (ASD) (Non Patent Literature 71). In a maternal immune activation (MIA) model, administration of PolyIC to pregnant animals causes ASD symptoms in offspring. The expression of IL-1β is increased in the fetal brain of this model, and administration of MCC950, an NLRP 3 inhibitor, to the mother suppresses ASD symptoms in offspring (Non Patent Literature 72). Thus, an NLRP3 inflammasome inhibitor is considered to become a drug for treating ASD.

In the spinal cord of mice with spinal cord injury, NLRP3 inflammasome or IL-1β expression is increased and NLRP3 activation is observed (Non Patent Literatures 73 and 74). When MCC950, an NLRP3 inhibitor, is administered to mice after spinal cord injury, NLRP3 activation and IL-1β expression in the spinal cord are suppressed, and the recovery of motor function is promoted (Non Patent Literature 73). Thus, an NLRP3 inflammasome inhibitor is considered to become a drug for treating spinal cord injury.

In an intestinal perforation model, an animal model of sepsis, increased expression and activation of NLRP3 inflammasome or IL-1β occur in the brain, resulting in damage to hippocampal neurons and memory impairment, a symptom of septic encephalopathy (Non Patent Literatures 75 and 76). When MCC950, an NLRP3 inhibitor, is administered to the intestinal perforation model, NLRP3 inflammasome activation is suppressed and the memory impairment is improved (Non Patent Literature 76). Thus, an NLRP3 inflammasome inhibitor is considered to become a drug for treating septic encephalopathy.

In a chronic constriction injury (CCI) model, an animal model of neuropathic pain, the expression levels of IL-1β and NLRP3 inflammasome-related molecules are increased in glial cells and neurons in the spinal cord (Non Patent Literature 77). In a paclitaxel-induced pain model, a neuropathic pain model of anticancer drug-induced neuropathy, the expression level of NLRP3 inflammasome-related molecules is increased in the dorsal root ganglion and sciatic nerve (Non Patent Literature 78). In a trigeminal neuralgia model animal, the expression level of NLRP3 inflammasome in the spinal cord dorsal horn is increased, and silencing NLRP3 in the spinal cord inhibits the NLRP3 inflammasome activation in the spinal cord and mechanical allodynia (Non Patent Literature 79). Thus, an NLRP3 inflammasome inhibitor is considered to become a drug for treating neuropathic pain.

Mice infected with SARS-CoV-2 show the increased expression levels of IL-1β and NLRP3 inflammasome-related molecules in lung tissue. NLRP3 knockout mice, on the other hand, do not show an increase in their expression levels and the severe respiratory inflammation caused by SARS-CoV-2 is reduced. In addition, administration of the NLRP3 inhibitor MCC950 to mice infected with SARS-CoV-2 inhibits NLRP3 inflammasome activation in the lung and suppresses the dysregulated immune response (Non Patent Literature 80). Thus, an NLRP3 inflammasome inhibitor is considered to become a drug for treating COVID-19 caused by SARS-CoV-2.

Increases of the ASC domain of NLRP3 inflammasome and matured IL-1β protein have been reported in the cerebral cortex of patients with frontotemporal dementia having mutations in protein tau (Non Patent Literature 81). Increases of the ASC domain of NLRP3 inflammasome and post-truncation caspase 1 have also been reported in the cerebral cortex of a frontotemporal dementia model, Tau22 mice which are human-mutant tau protein-expressed mice, which indicated that knockout of NLRP3 inhibited formation of tau pathologies and cognitive function decline (Non Patent Literature 81). These results suggest that an NLRP3 inflammasome inhibitor is considered to become a drug for treating frontotemporal dementia.

A possible causative substance, drusen, that is considered to develop age-related macular degeneration (AMD) was confirmed to be formed in patients with NLRP3-associated autoinflammatory disease (NLRP3-AID) caused by activation mutation in the NLRP3 gene (Non Patent Literature 82). An NLRP3 inhibitor also suppressed degeneration of retinal pigment epithelial cells in one of models of age-related macular degeneration, a model with Alu RNA-induced degeneration in retinal pigment epithelial cells (Non Patent Literature 83). An NLRP3 inhibitor suppressed neoangiogenesis in another model of age-related macular degeneration, a model with laser-induced choroidal neovascularization (Non Patent Literature 83). These results suggest that an NLRP3 inflammasome inhibitor is considered to become a drug for treating age-related macular degeneration.

Diabetes increases retinal vascular permeability in patients with diabetic macular edema, which results in leak of blood ingredients into retina (Non Patent Literature 84). An NLRP3 inhibitor, MCC950, alleviated increased vascular permeability in STZ-induced diabetic mice (Non Patent Literature 85). Thus, an NLRP3 inflammasome inhibitor is considered to become a drug for treating diabetic macular edema.

Hereditary transient corneal endotheliitis is one of Cryopyrin-associated periodic syndromes that is occurred by activation mutation in the NLRP3 gene (Non Patent Literature 86). Thus, an NLRP3 inflammasome inhibitor is considered to become a drug for treating hereditary transient corneal endotheliitis.

### CITATION LIST

### NON PATENT LITERATURE

[Non Patent Literature 1] Andersson, A et al., Pivotal advance: HMGB1 expression in active lesions of human and experimental multiple sclerosis. J Leukoc Biol., 2008, Vol 84 (5), p.1248-55
[Non Patent Literature 2] Voet, S et al., A20 critically controls microglia activation and inhibits inflammasome-dependent neuroinflammation. Nat Commun., 2018, Vol 9(1), p2036.
[Non Patent Literature 3] Politis, M et al., Increased PK11195 PET binding in the cortex of patients with MS correlates with disability. Neurology, 2012, Vol 79(6), p523-30.
[Non Patent Literature 4] Hernandez-Pedro, N et al., PAMP-DAMPs interactions mediates development and progression of multiple sclerosis. Front Biosci (Schol Ed), 2016, Vol 8, p13-28.
[Non Patent Literature 5] Denis, G et al., NLRP3 Plays a Critical Role in the Development of Experimental Autoimmune Encephalomyelitis by Mediating Th1 and Th17 Responses. J Immunol., 2010, Vol 185 (2) p974-981
[Non Patent Literature 6] Jha, S et al., The inflammasome sensor, NLRP3, regulates CNS inflammation and demyelination via caspase-1 and interleukin-18. J Neurosci., 2010 Vol 30(47), p15811-20
[Non Patent Literature 7] Guo, C et al., Development and Characterization of a Hydroxyl-Sulfonamide Analogue, 5-Chloro-N-[2-(4-hydroxysulfamoyl-phenyl)-ethyl]-2-methoxybenzamide, as a Novel NLRP3 Inflammasome Inhibitor for Potential Treatment of Multiple Sclerosis. ACS Chem Neurosci. , 2017, Vol 8(10), p2194-2201
[Non Patent Literature 8] Akosua Vilaysane et al., The NLRP3 Inflammasome Promotes Renal Inflammation and Contributes to CKD. J Am Soc Nephrol. 2010 Oct; 21(10): 1732-1744.
[Non Patent Literature 9] Shahzad K et al., Nlrp3-inflammasome activation in non-myeloid-derived cells aggravates diabetic nephropathy. Kidney Int. 2015 Jan;87(1):74-84.
[Non Patent Literature 10] Gong W et al., NLRP3 deletion protects against renal fibrosis and attenuates mitochondrial abnormality in mouse with 5/6 nephrectomy. Am J Physiol Renal Physiol. 2016 May 15;310(10):F1081-8
[Non Patent Literature 11] Ranson N et al., NLRP3-dependent and -independent processing Interleiukin-1β in active Ulcerative colitis. Int J mol Sci 2018: 20pii:E57.
[Non Patent Literature 12] Mao L et al., Loss-of-function CARD8 mutation causes NLRP3 inflammasome activation and Crohn's disease. J Clin Invest 2018: vol 128:1793-1806.
[Non Patent Literature 13] Bauer c. et al., Protective and aggravating effects of NLRP3 inlammasome activation in IBD models: influence of genetic and environmental factors. Dig.Dis 2012 vol 30 suppl 1 82-90.
[Non Patent Literature 14] Paramel V G et al., NLRP3 Inflammasome Expression and Activation in Human Atherosclerosis. J Am Heart Assoc. 2016 May 20;5(5):e003031.
[Non Patent Literature 15] Duewell P et al., NLRP3 inflammasomes are required for atherogenesis and activated by cholesterol crystals. Nature. 2010 Apr 29;464(7293):1357-61.
[Non Patent Literature 16] Broderick L et al., The inflammasomes and autoinflammatory syndromes. Annu Rev Pathol. 2015;10:395-424.
[Non Patent Literature 17] Sarrauste M C et al., INFEVERS: the Registry for FMF and hereditary inflammatory disorders mutations. Nucleic Acids Res. 2003 Jan 1;31(1):282-5.
[Non Patent Literature 18] Brydges SD et al., Divergence of IL-1, IL-18, and cell death in NLRP3 inflammasomopathies. J Clin Invest. 2013 Nov;123(11):4695-705.
[Non Patent Literature 19] Jiang H et al., Identification of a selective and direct NLRP3 inhibitor to treat inflammatory disorders. J Exp Med. 2017 Nov 6;214(11):3219-3238.
[Non Patent Literature 20] Wree A et al., NLRP3 inflammasome activation is required for fibrosis development in NAFLD. J Mol Med (Berl). 2014 Oct;92(10):1069-82.
[Non Patent Literature 21] So AK et al., Inflammation in gout: mechanisms and therapeutic targets. Nat Rev Rheumatol. 2017 Nov;13(11):639-647.
[Non Patent Literature 22] Martinon F et al., Gout-associated uric acid crystals activate the NALP3 inflammasome. Nature. 2006 Mar 9;440(7081):237-41.
[Non Patent Literature 23] Marchetti C et al., NLRP3 inflammasome inhibitor OLT1177 suppresses joint inflammation in murine models of acute arthritis. Arthritis Res Ther. 2018 Aug 3;20(1):169.
[Non Patent Literature 24] Mathews RJ et al., Evidence of NLRP3-inflammasome activation in rheumatoid arthritis (RA); genetic variants within the NLRP3-inflammasome complex in relation to susceptibility to RA and response to anti-TNF treatment. Ann Rheum Dis. 2014 Jun;73(6):1202-10.
[Non Patent Literature 25] Zhang Y et al., NLRP3 Inflammasome Plays an Important Role in the Pathogenesis of Collagen-Induced Arthritis. Mediators Inflamm. 2016;2016:9656270.
[Non Patent Literature 26] Watanabe H et al., Activation of the IL-1beta-processing inflammasome is involved in contact hypersensitivity. J Invest Dermatol. 2007 Aug;127(8):1956-63.
[Non Patent Literature 27] Niu L et al., Upregulation of NLRP3 Inflammasome in the Tears and Ocular Surface of Dry Eye Patients. PLoS One. 2015 May 11;10(5):e0126277.
[Non Patent Literature 28] Zheng Q et al., Reactive oxygen species activated NLRP3 inflammasomes initiate inflammation in hyperosmolarity stressed human corneal epithelial cells and environment-induced dry eye patients. Exp Eye Res. 2015 May;134:133-40.
[Non Patent Literature 29] Kawaguchi M et al., Inflammasome activation of cardiac fibroblasts is essential for myocardial ischemia/reperfusion injury. Circulation. 2011 Feb 15;123(6):594-604.
[Non Patent Literature 30] Sandanger O et al., The NLRP3 inflammasome is up-regulated in cardiac fibroblasts and mediates myocardial ischaemia-reperfusion injury. Cardiovasc Res. 2013 Jul 1;99(1):164-74.
[Non Patent Literature 31] Dellalibera-Joviliano R et al., Kinins and cytokines in plasma and cerebrospinal fluid of patients with neuropsychiatric lupus. J Rheumatol. 2003 Mar;30(3):485-92.
[Non Patent Literature 32] Tucci M et al., Glomerular accumulation of plasmacytoid dendritic cells in active lupus nephritis: role of interleukin-18. Arthritis Rheum. 2008 Jan;58(1):251-62.
[Non Patent Literature 33] Yang CA et al., Sex-dependent differential activation of NLRP3 and AIM2 inflammasomes in SLE macrophages. Rheumatology (Oxford). 2015 Feb;54(2):324-31.
[Non Patent Literature 34] Lu A et al., Hyperactivation of the NLRP3 Inflammasome in Myeloid Cells Leads to Severe Organ Damage in Experimental Lupus. J Immunol. 2017 Feb 1;198(3):1119-1129.
[Non Patent Literature 35] Ruperto N et al., Two randomized trials of canakinumab in systemic juvenile idiopathic arthritis. N Engl J Med. 2012 Dec 20;367(25):2396-406.
[Non Patent Literature 36] Klein AL et al., Phase 3 Trial of Interleukin-1 Trap Rilonacept in Recurrent Pericarditis. N Engl J Med. 2021 Jan 7; 384(1):31-41.
[Non Patent Literature 37] Junge G et al., Adult onset Still's disease-The evidence that anti-interleukin-1 treatment is effective and well-tolerated (a comprehensive literature review). Seminars in Arthritis Rheumatism 2017 Oct; 47(2):295-302.
[Non Patent Literature 38] Krause K et al., Efficacy and safety of canakinumab in Schnitzler syndrome: A multicenter randomized placebo-controlled study. J Allergy Clin Immunol. 2017 Apr;139(4):1311-1320.
[Non Patent Literature 39] Garg M et al., Rilonacept maintains long-term inflammatory remission in patients with deficiency of the IL-1 receptor antagonist. JCI Insight. 2017 Aug 17;2(16).
[Non Patent Literature 40] De Benedetti F et al., Canakinumab for the Treatment of Autoinflammatory Recurrent Fever Syndromes. N Engl J Med. 2018 May 17;378(20):1908-1919.
[Non Patent Literature 41] Emmi G et al., Efficacy and safety profile of anti-interleukin-1 treatment in Behcet's disease: a multicenter retrospective study. Clin. Rheumatol., 35 (2016), pp. 1281-1286.
[Non Patent Literature 42] Ridker P.M. et al., Antiinflammatory Therapy with Canakinumab for Atherosclerotic Disease. Lancet (2017) 390 1833-42.
[Non Patent Literature 43] M Carlstrom et al., Genetic support for the role of the NLRP3 inflammasome in psoriasis susceptibility. Exp Dermatol. (2012) 21:932-7.
[Non Patent Literature 44] J. A Diaz-Perez et al., Extracellular ATP and IL-23 Form a Local Inflammatory Circuit Leading to the Development of a Neutrophil-Dependent Psoriasiform Dermatitis. J Invest Dermatol. (2018) 138:2595-605.
[Non Patent Literature 45] Wang Q et al., Renin-Dependent Hypertension in Mice Requires the NLRP3-Inflammasome. J. Hypertens (2014) 3:187.
[Non Patent Literature 46] Krishnan S M et al., Pharmacological inhibition of the NLRP3 inflammasome reduces blood pressure, renal damage, and dysfunction in salt-sensitive hypertension. Cardiovasc. Res. (2019) 115 4: 776-787.
[Non Patent Literature 47] Zhang Y et al., Protection of Mcc950 against high-glucose-induced human retinal endothelial cell dysfunction. Cell Death Dis. 2017 Jul 20; 8(7):e2941.
[Non Patent Literature 48] Sheng Li et al., Protective effects of sulforaphane on diabetic retinopathy: activation of the Nrf2 pathway and inhibition of NLRP3 inflammasome formation. Exp Anim. 2019 May 8;68(2):221-231.
[Non Patent Literature 49] Guangrui Chai et al., NLRP3 Blockade Suppresses Pro-Inflammatory and Pro-Angiogenic Cytokine Secretion in Diabetic Retinopathy. Diabetes Metab Syndr Obes. 2020 Aug 25;13:3047-3058.
[Non Patent Literature 50] Heneka MT et al., NLRP3 is activated in Alzheimer's disease and contributes to pathology in APP/PS1 mice. Nature. 2013 Jan 31;493(7434):674-8.
[Non Patent Literature 51] Dempsey C et al., Inhibiting the NLRP3 inflammasome with MCC950 promotes non-phlogistic clearance of amyloid-β and cognitive function in APP/PS1 mice. Brain Behav Immun. 2017 Mar; 61:306-316.
[Non Patent Literature 52] Gordon R et al., Inflammasome inhibition prevents α-synuclein pathology and dopaminergic neurodegeneration in mice. Sci Transl Med. 2018 Oct 31;10(465):eaah4066.
[Non Patent Literature 53] Rodrigues FB et al., Cerebrospinal Fluid Inflammatory Biomarkers Reflect Clinical Severity in Huntington's Disease. PLoS One. 2016 Sep 22;11(9):e0163479.
[Non Patent Literature 54] Paldino E et al., Pyroptotic cell death in the R6/2 mouse model of Huntington's disease: new insight on the inflammasome. Cell Death Discov. 2020 Jul 31;6:69.
[Non Patent Literature 55] Chen KP et al., A selective inhibitor of the NLRP3 inflammasome as a potential therapeutic approach for neuroprotection in a transgenic mouse model of Huntington's disease. J Neuroinflammation. 2022 Feb 26;19(1):56.
[Non Patent Literature 56] Johann S et al., NLRP3 inflammasome is expressed by astrocytes in the SOD1 mouse model of ALS and in human sporadic ALS patients. Glia. 2015 Dec;63(12):2260-73.
[Non Patent Literature 57] Deora V et al., The microglial NLRP3 inflammasome is activated by amyotrophic lateral sclerosis proteins. Glia. 2020 Feb;68(2):407-421.
[Non Patent Literature 58] Meissner F et al., A. Mutant superoxide dismutase 1-induced IL-1beta accelerates ALS pathogenesis. Proc Natl Acad Sci U S A. 2010 Jul 20;107(29):13046-50.
[Non Patent Literature 59] Lin C et al., Omega-3 fatty acids regulate NLRP3 inflammasome activation and prevent behavior deficits after traumatic brain injury. Exp Neurol. 2017 Apr;290:115-122.
[Non Patent Literature 60] Wallisch JS et al., Cerebrospinal Fluid NLRP3 is Increased After Severe Traumatic Brain Injury in Infants and Children. Neurocrit Care. 2017 Aug;27(1):44-50.
[Non Patent Literature 61] Liu HD et al., Expression of the NLRP3 inflammasome in cerebral cortex after traumatic brain injury in a rat model. Neurochem Res. 2013 Oct;38(10):2072-83.
[Non Patent Literature 62] Ismael S et al., MCC950, the Selective Inhibitor of Nucleotide Oligomerization Domain-Like Receptor Protein-3 Inflammasome, Protects Mice against Traumatic Brain Injury. J Neurotrauma. 2018 Jun 1;35(11):1294-1303.
[Non Patent Literature 63] Fann DY et al., Intravenous immunoglobulin suppresses NLRP1 and NLRP3 inflammasome-mediated neuronal death in ischemic stroke. Cell Death Dis. 2013 Sep 5;4(9):e790.
[Non Patent Literature 64] Feng L et al., P2X7R blockade prevents NLRP3 inflammasome activation and brain injury in a rat model of intracerebral hemorrhage: involvement of peroxynitrite. J Neuroinflammation. 2015 Oct 17;12:190.
[Non Patent Literature 65] Yue J et al., NLRP3 inflammasome and endoplasmic reticulum stress in the epileptogenic zone in temporal lobe epilepsy: molecular insights into their interdependence. Neuropathol Appl Neurobiol. 2020 Dec;46(7):770-785.
[Non Patent Literature 66] Wu C et al., The Role of NLRP3 and IL-1β in Refractory Epilepsy Brain Injury. Front Neurol. 2020 Feb 7;10:1418.
[Non Patent Literature 67] Alcocer-Gomez E et al., NLRP3 inflammasome is activated in mononuclear blood cells from patients with major depressive disorder. Brain Behav Immun. 2014 Feb;36:111-7.
[Non Patent Literature 68] Zhang Y et al., Involvement of inflammasome activation in lipopolysaccharide-induced mice depressive-like behaviors. CNS Neurosci Ther. 2014 Feb;20(2):119-24.
[Non Patent Literature 69] Iwata M et al., Psychological Stress Activates the Inflammasome via Release of Adenosine Triphosphate and Stimulation of the Purinergic Type 2X7 Receptor. Biol Psychiatry. 2016 Jul 1;80(1):12-22.
[Non Patent Literature 70] Li W et al., Inhibition of the NLRP3 inflammasome with MCC950 prevents chronic social isolation-induced depression-like behavior in male mice. Neurosci Lett. 2021 Nov 20;765:136290.
[Non Patent Literature 71] Saresella M et al., Multiple inflammasome complexes are activated in autistic spectrum disorders. Brain Behav Immun. 2016 Oct;57:125-133.
[Non Patent Literature 72] Szabo D et al., Maternal P2X7 receptor inhibition prevents autism-like phenotype in male mouse offspring through the NLRP3-IL-1β pathway. Brain Behav Immun. 2022 Mar;101:318-332.
[Non Patent Literature 73] Amo-Aparicio J et al., Inhibition of the NLRP3 inflammasome by OLT1177 induces functional protection and myelin preservation after spinal cord injury. Exp Neurol. 2022 Jan;347:113889.
[Non Patent Literature 74] Jiao J et al., MCC950, a Selective Inhibitor of NLRP3 Inflammasome, Reduces the Inflammatory Response and Improves Neurological Outcomes in Mice Model of Spinal Cord Injury. Front Mol Biosci. 2020 Mar 3;7:37.
[Non Patent Literature 75] Ding H et al., Fisetin ameliorates cognitive impairment by activating mitophagy and suppressing neuroinflammation in rats with sepsis-associated encephalopathy. CNS Neurosci Ther. 2022 Feb;28(2):247-258.
[Non Patent Literature 76] Fu Q et al., NLRP3/Caspase-1 Pathway-Induced Pyroptosis Mediated Cognitive Deficits in a Mouse Model of Sepsis-Associated Encephalopathy. Inflammation. 2019 Feb;42(1):306-318.
[Non Patent Literature 77] Xu L et al., MiR-34c Ameliorates Neuropathic Pain by Targeting NLRP3 in a Mouse Model of Chronic Constriction Injury. Neuroscience. 2019 Feb 10;399:125-134.
[Non Patent Literature 78] Jia M et al., Activation of NLRP3 inflammasome in peripheral nerve contributes to paclitaxel-induced neuropathic pain. Mol Pain. 2017 Jan-Dec;13:1744806917719804.
[Non Patent Literature 79] Sun X et al., The NLRP3-related inflammasome modulates pain behavior in a rat model of trigeminal neuropathic pain. Life Sci. 2021 Jul 15;277:119489.
[Non Patent Literature 80] Zeng J et al., Specific inhibition of the NLRP3 inflammasome suppresses immune overactivation and alleviates COVID-19 like pathology in mice. EBioMedicine. 2022 Jan;75:103803.
[Non Patent Literature 81] Ising C, et al., NLRP3 inflammasome activation drives tau pathology. Nature. 2019 Nov; 575(7784): 669-673.
[Non Patent Literature 82] Bing Li, et al., Early onset drusen and RPE dysfunction in a patient with NLRP3-AID, Ocul Immunol Inflamm. 2022 Nov 17;1-4.
[Non Patent Literature 83] Benjamin J Fowler, et al., Nucleoside reverse transcriptase inhibitors possess intrinsic anti-inflammatory activity, Science. 2014 Nov 21;346(6212):1000-3.
[Non Patent Literature 84] Hideharu Funatsu, Development of treatment for macular edema, Diabetes 48(10): 721-723, 2005.
[Non Patent Literature 85] Keke Ge, et al., Down-expression of the NLRP3 inflammasome delays the progression of diabetic retinopathy, Microvasc Res. 2022 Jan;139:104265.
[Non Patent Literature 86] Joni A Turunen, et al., Keratoendotheliitis Fugax Hereditaria: A Novel Cryopyrin-Associated Periodic Syndrome Caused by a Mutation in the Nucleotide-Binding Domain, Leucine-Rich Repeat Family, Pyrin Domain-Containing 3 (NLRP3) Gene, Am J Ophthalmol. 2018 Apr;188:41-50.

### SUMMARY OF INVENTION

The present invention provides pyrazolopyrimidine compounds, or pharmaceutically acceptable salts thereof, having NLRP3 inflammasome inhibitory activity, pharmaceutical compositions comprising the same, and medical use thereof, etc. Specifically, the present invention includes the embodiments illustrated as follows.

### Item 1

A compound of Formula [I]: 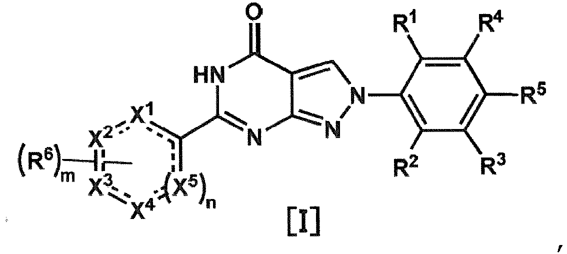 or a pharmaceutically acceptable salt thereof (herein "a compound of Formula [I] or a pharmaceutically acceptable salt thereof" is also referred to as "Compound [I]"), wherein a bond represented by: 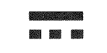
is a single bond or a double bond;
R¹ and R² are each independently
   (1) hydrogen,
   (2) hydroxy,
   (3) cyano,
   (4) C₁₋₆ alkyl, wherein the alkyl may be optionally substituted with 1 or 2 substituents independently selected from the group consisting of:
      (a) hydroxy,
      (b) C₁₋₄ alkoxy, and
      (c) C₃₋₆ cycloalkyl,
   (5) C₁₋₆ alkoxy, wherein the alkoxy may be optionally substituted with C₃₋₆ cycloalkyl,
   (6) halogen,
   (7) C₁₋₄ haloalkyl,
   (8) -CHO,
   (9) -O-C₁₋₄ haloalkyl,
   (10) -O-C₃₋₆ cycloalkyl,
   (11) -CO-C₁₋₄ alkyl,
   (12) -CO-C₃₋₆ cycloalkyl,
   (13) -NR⁷R⁸, wherein R⁷ and R⁸ are each independently hydrogen or 2,4-dimethoxybenzyl, or alternatively, R⁷ and R⁸ may be combined together with the nitrogen atom to which they attach and the -NR⁷R⁸ group may form 5- to 6-membered heterocycloalkyl comprising 1 or 2 heteroatoms independently selected from the group consisting of nitrogen and oxygen atom, or
   (14) C₃₋₆ cycloalkyl,
R³ and R⁴ are each independently
   (1) hydrogen,
   (2) C₁₋₄ alkyl, or
   (3) C₁₋₄ haloalkyl,
R⁵ is
   (1) hydrogen,
   (2) cyano,
   (3) C₁₋₆ alkyl,
   (4) C₂₋₆ alkenyl,
   (5) C₂₋₅ alkynyl,
   (6) C₁₋₄ alkoxy,
   (7) halogen,
   (8) C₁₋₆ haloalkyl,
   (9) C₂₋₆ haloalkenyl,
   (10) -O-C₁₋₄ haloalkyl,
   (11) C₃₋₆ cycloalkyl, wherein the cycloalkyl may be optionally substituted with 1 to 3 halogen or C₁₋₄ haloalkyl,
   (12) C₅₋₆ cycloalkenyl, or
   (13) 4- to 6-membered heterocycloalkyl comprising 1 or 2 heteroatoms independently selected from the group consisting of nitrogen and oxygen atom,
R⁶ is
   (1) C₁₋₄ alkyl, wherein the alkyl may be optionally substituted with C₁₋₄ alkoxy,
   (2) C₁₋₄ alkoxy,
   (3) halogen,
   (4) C₁₋₄ haloalkyl, or
   (5) 4- to 6-membered heterocycloalkyl comprising 1 or 2 heteroatoms independently selected from the group consisting of nitrogen and oxygen atom,
m is 0, 1, or 2,
n is 0 or 1,
provided that when n is 0, then X¹, X², X³, and X⁴ are each independently carbon, nitrogen, oxygen, or sulfur atom, wherein a total number of nitrogen, oxygen, and sulfur atoms as X¹, X², X³, or X⁴ is 1, 2, or 3, and a total number of oxygen and sulfur atoms is 0 or 1, and X¹, X², X³, and X⁴ are combined together with the carbon atom that is adjacent to X¹ and X⁴ to form heteroaryl, and
provided that when n is 1, then X¹, X², X³, X⁴, and X⁵ are each independently carbon or nitrogen atom, wherein a total number of nitrogen atoms as X¹, X², X³, X⁴, or X⁵ is 1 or 2, and X¹, X², X³, X⁴, and X⁵ are combined together with the carbon atom that is adjacent to X¹ and X⁵ to form heteroaryl.

### Item 2

The compound according to Item 1, or a pharmaceutically acceptable salt thereof, wherein R³ and R⁴ are hydrogen.

### Item 3

The compound according to Item 1 or 2, or a pharmaceutically acceptable salt thereof, wherein the compound is represented by Formula [II]: 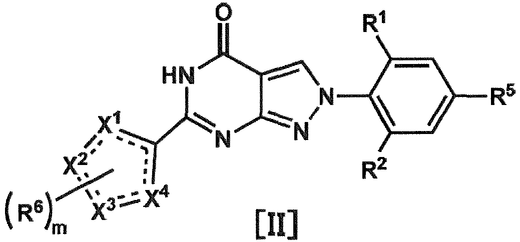 wherein each symbol is as defined in Item 1.

### Item 4

The compound according to Item 1 or 2, or a pharmaceutically acceptable salt thereof, wherein the compound is represented by Formula [III]: 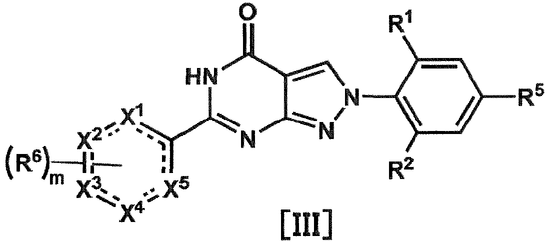 wherein each symbol is as defined in Item 1.

### Item 5

A pharmaceutical composition comprising the compound according to any one of Items 1 to 4, or a pharmaceutically acceptable salt thereof, and a pharmaceutically acceptable carrier.

### Item 6

An NLRP3 inflammasome inhibitor comprising the compound according to any one of Items 1 to 4, or a pharmaceutically acceptable salt thereof.

### Item 7

A medicament for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, and TNF receptor-associated periodic syndrome, comprising the compound according to any one of Items 1 to 4, or a pharmaceutically acceptable salt thereof.

### Item 8

The medicament according to Item 7, wherein the inflammatory bowel disease is ulcerative colitis or Crohn's disease. Item 9

The medicament according to Item 7, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Item 10

A method for inhibiting NLRP3 inflammasome, comprising administering a therapeutically effective amount of the compound according to any one of Items 1 to 4, or a pharmaceutically acceptable salt thereof, to a mammal.

### Item 11

A method for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, and TNF receptor-associated periodic syndrome, comprising administering a therapeutically effective amount of the compound according to any one of Items 1 to 4, or a pharmaceutically acceptable salt thereof, to a mammal.

### Item 12

The method according to Item 11, wherein the inflammatory bowel disease is ulcerative colitis or Crohn's disease.

### Item 13

The method according to Item 11, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Item 14

Use of the compound according to any one of Items 1 to 4, or a pharmaceutically acceptable salt thereof, in the manufacture of an NLRP3 inflammasome inhibitor.

### Item 15

Use of the compound according to any one of Items 1 to 4, or a pharmaceutically acceptable salt thereof, in the manufacture of a medicament for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, and TNF receptor-associated periodic syndrome.

### Item 16

The use according to Item 15, wherein the inflammatory bowel disease is ulcerative colitis or Crohn's disease.

### Item 17

The use according to Item 15, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Item 18

A compound according to any one of Items 1 to 4, or a pharmaceutically acceptable salt thereof, for use in inhibiting NLRP3 inflammasome.

### Item 19

A compound according to any one of Items 1 to 4, or a pharmaceutically acceptable salt thereof, for use in treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, and TNF receptor-associated periodic syndrome.

### Item 20

The compound, or a pharmaceutically acceptable salt thereof, for use according to Item 19, wherein the inflammatory bowel disease is ulcerative colitis or Crohn's disease.

### Item 21

The compound, or a pharmaceutically acceptable salt thereof, for use according to Item 19, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Item 22

A package for commercial use, comprising the pharmaceutical composition according to Item 5 and a written document associated with the pharmaceutical composition, the document describing that the composition may be used for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, and TNF receptor-associated periodic syndrome.

### Item 23

A kit for commercial use, comprising the pharmaceutical composition according to Item 5 and a written document associated with the pharmaceutical composition, the document describing that the composition may be used for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, and TNF receptor-associated periodic syndrome.

### Item 1A

A compound of Formula [IA]: 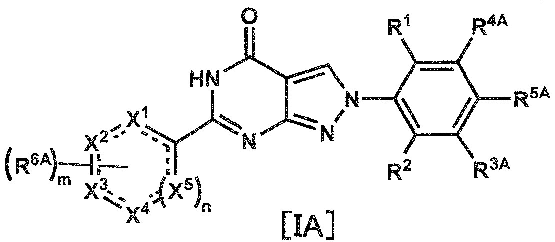 or a pharmaceutically acceptable salt thereof (herein "a compound of Formula [IA] or a pharmaceutically acceptablt salt thereof" is also referred to as "Compound [IA]"),
wherein a bond represented by: 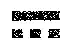
is a single bond or a double bond;
R¹ and R² are each independently
   (1) hydrogen,
   (2) hydroxy,
   (3) cyano,
   (4) C₁₋₆ alkyl, wherein the alkyl may be optionally substituted with 1 or 2 substituents independently selected from the group consisting of:
      (a) hydroxy,
      (b) C₁₋₄ alkoxy, and
      (c) C₃₋₆ cycloalkyl,
   (5) C₁₋₆ alkoxy, wherein the alkoxy may be optionally substituted with C₃₋₆ cycloalkyl,
   (6) halogen,
   (7) C₁₋₄ haloalkyl,
   (8) -CHO,
   (9) -O-C₁₋₄ haloalkyl,
   (10) -O-C₃₋₆ cycloalkyl,
   (11) -CO-C₁₋₄ alkyl,
   (12) -CO-C₃₋₆ cycloalkyl,
   (13) -NR⁷R⁸, wherein R⁷ and R⁸ are each independently hydrogen or 2,4-dimethoxybenzyl, or alternatively, R⁷ and R⁸ may be combined together with the nitrogen atom to which they attach and the -NR⁷R⁸ group may form 5- to 6-membered heterocycloalkyl comprising 1 or 2 heteroatoms independently selected from the group consisting of nitrogen and oxygen atom, or
   (14) C₃₋₆ cycloalkyl,
R^{3A} and R^{4A} are each independently
   (1) hydrogen,
   (2) C₁₋₄ alkyl, or
   (3) C₁₋₄ haloalkyl,
R^{5A} is
   (1) hydrogen,
   (2) cyano,
   (3) C₁₋₆ alkyl,
   (4) C₂₋₆ alkenyl,
   (5) C₂₋₅ alkynyl,
   (6) C₁₋₄ alkoxy,
   (7) halogen,
   (8) C₁₋₆ haloalkyl,
   (9) C₂₋₆ haloalkenyl,
   (10) -O-C₁₋₄ haloalkyl,
   (11) C₃₋₆ cycloalkyl, wherein the cycloalkyl may be optionally substituted with 1 to 3 substituents independently selected from the group consisting of:
      (a) hydroxy,
      (b) halogen, and
      (c) C₁₋₄ haloalkyl,
   (12) C₅₋₆ cycloalkenyl, or
   (13) 4- to 6-membered heterocycloalkyl comprising 1 or 2 heteroatoms independently selected from the group consisting of nitrogen and oxygen atom, or alternatively
R^{5A} may be combined together with R^{3A} or R^{4A} and the carbon atom to which they attach to form:
   (1) C₅₋₆ cycloalkene, or
   (2) 5- to 7-membered heterocycloalkene comprising 1 or 2 oxygen atoms,
R^{6A} is independently
   (1) C₁₋₄ alkyl, wherein the alkyl may be optionally substituted with C₁₋₄ alkoxy,
   (2) C₁₋₄ alkoxy,
   (3) halogen,
   (4) C₁₋₄ haloalkyl, or
   (5) 4- to 6-membered heterocycloalkyl comprising 1 or 2 heteroatoms independently selected from the group consisting of nitrogen and oxygen atom,
m is 0, 1, or 2,
provided that when m is 2, then two adjacent R^{6A}s may be combined together with the two adjacent atoms among X¹, X², X³, X⁴, and X⁵ to which they attach to form 5- to 7-membered heterocycloalkane or heterocycloalkene comprising 1 or 2 heteroatoms independently selected from the group consisting of nitrogen and oxygen atom,
n is 0 or 1,
provided that when n is 0, then X¹, X², X³, and X⁴ are each independently carbon, nitrogen, oxygen, or sulfur atom, wherein a total number of nitrogen, oxygen, and sulfur atoms as X¹, X², X³, or X⁴ is 1, 2, or 3, and a total number of oxygen and sulfur atoms is 0 or 1, and X¹, X², X³, and X⁴ are combined together with the carbon atom that is adjacent to X¹ and X⁴ to form heteroaryl, and
provided that when n is 1, then X¹, X², X³, X⁴, and X⁵ are each independently carbon or nitrogen atom, wherein a total number of nitrogen atoms as X¹, X², X³, X⁴, or X⁵ is 1 or 2, and X¹, X², X³, X⁴, and X⁵ are combined together with the carbon atom that is adjacent to X¹ and X⁵ to form heteroaryl.

### Item 2A

The compound according to Item 1A, or a pharmaceutically acceptable salt thereof, wherein R^{3A} and R^{4A} are hydrogen.

### Item 3A

The compound according to Item 1A or 2A, or a pharmaceutically acceptable salt thereof, wherein the compound is represented by Formula [IIA]: 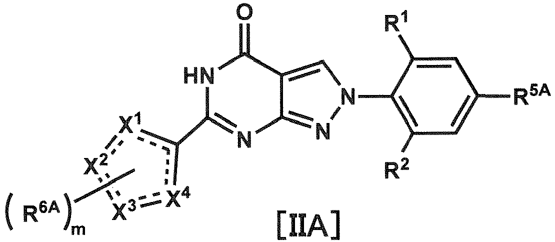 wherein R¹, R², R^{5A}, R^{6A}, X¹, X², X³, X⁴, and m are defined as those defined in Item 1A.

### Item 4A

The compound according to any one of Items 1A to 3A, or a pharmaceutically acceptable salt thereof, wherein X¹, X², X³, and X⁴ are combined together with the carbon atom that is adjacent to X¹ and X⁴ to form pyrazolyl, imidazolyl, oxazolyl, isoxazolyl, thiazolyl, oxadiazolyl, or triazolyl.

### Item 5A

The compound according to any one of Items 1A to 4A, or a pharmaceutically acceptable salt thereof, wherein X¹, X², X³, and X⁴ are combined together with the carbon atom that is adjacent to X¹ and X⁴ to form pyrazolyl, imidazolyl, or thiazolyl.

### Item 6A

The compound according to any one of Items 1A to 5A, or a pharmaceutically acceptable salt thereof, wherein X¹, X², X³, and X⁴ are combined together with the carbon atom that is adjacent to X¹ and X⁴ to form a group of formula (1): 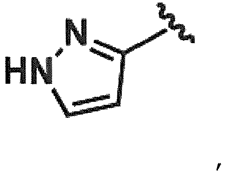 a group of formula (2): 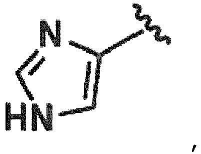 or
a group of formula (3):

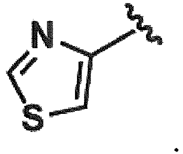

### Item 7A

The compound according to Item 1A or 2A, or a pharmaceutically acceptable salt thereof, wherein the compound is represented by Formula [IIIA]: 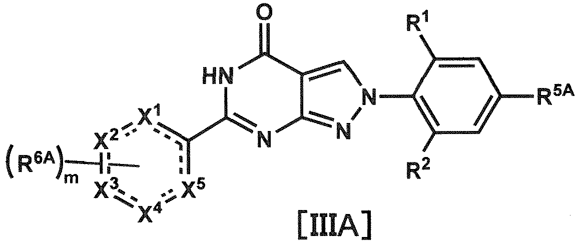 wherein R¹, R², R^{5A}, R^{6A}, X¹, X², X³, X⁴, X⁵, and m are defined as those defined in Item 1A.

### Item 8A

The compound according to any one of Items 1A, 2A, or 7A, or a pharmaceutically acceptable salt thereof, wherein X¹, X², X³, X⁴, and X⁵ are combined together with the carbon atom that is adjacent to X¹ and X⁵ to form pyridyl, pyridazinyl, pyrimidyl, or pyrazinyl.

### Item 9A

The compound according to any one of Item 1A, 2A, 7A, or 8A, or a pharmaceutically acceptable salt thereof, wherein X¹, X², X³, X⁴, and X⁵ are combined together with the carbon atom that is adjacent to X¹ and X⁵ to form pyridazinyl or pyrimidyl.

### Item 10A

The compound according to any one of Item 1A, 2A, 7A, 8A, or 9A, or a pharmaceutically acceptable salt thereof, wherein X¹, X², X³, X⁴, and X⁵ are combined together with the carbon atom that is adjacent to X¹ and X⁵ to form a group of formula (1'): 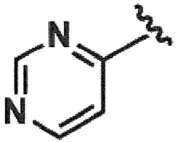 or
a group of formula (2'):

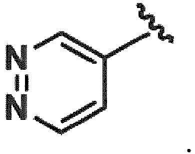

### Item 11A

The compound according to any one of Items 1A to 10A, or a pharmaceutically acceptable salt thereof, wherein m is 0 or 1.

### Item 12A

The compound according to any one of Items 1A to 11A, or a pharmaceutically acceptable salt thereof, wherein at least one of R¹ and R² is:
C₁₋₆ alkyl, wherein the alkyl may be optionally substituted with 1 or 2 substituents independently selected from the group consisting of:
   (a) hydroxy,
   (b) C₁₋₄ alkoxy, and
   (c) C₃₋₆ cycloalkyl, or
halogen.

### Item 13A

A compound selected from the group consisting of the following structural formulae: 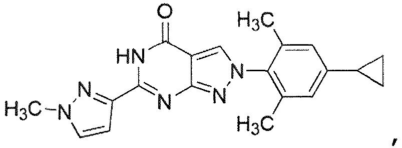 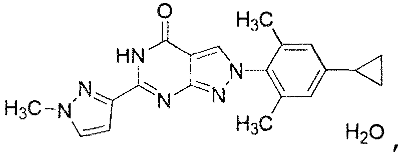 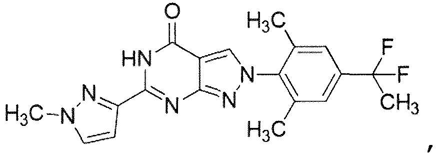 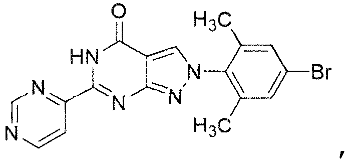 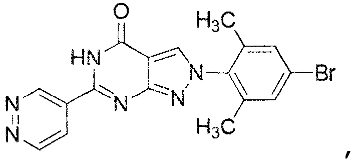 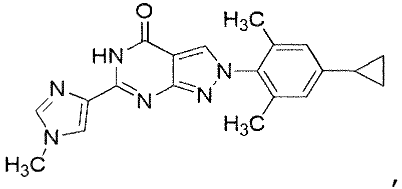 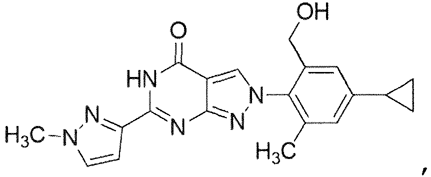 and 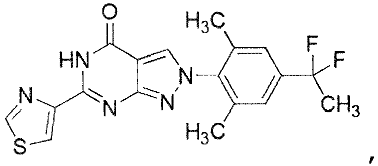 or a pharmaceutically acceptable salt thereof.

### Item 14A

A pharmaceutical composition comprising a compound according to any one of Items 1A to 13A, or a pharmaceutically acceptable salt thereof, and a pharmaceutically acceptable carrier.

### Item 15A

An NLRP3 inflammasome inhibitor comprising a compound according to any one of Items 1A to 13A, or a pharmaceutically acceptable salt thereof.

### Item 16A

A medicament for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome, comprising a compound according to any one of Items 1A to 13A, or a pharmaceutically acceptable salt thereof.

### Item 17A

The medicament according to Item 16A, wherein the inflammatory bowel disease is ulcerative colitis or Crohn's disease.

### Item 18A

The medicament according to Item 16A, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Item 19A

The medicament according to Item 16A, wherein the disease is selected from the group consisting of multiple sclerosis, dry eye, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, frontotemporal dementia, age-related macular degeneration, diabetic macular edema, and hereditary transient corneal endotheliitis.

### Item 20A

A method for inhibiting NLRP3 inflammasome, comprising administering a therapeutically effective amount of a compound according to any one of Items 1A to 13A, or a pharmaceutically acceptable salt thereof, to a mammal.

### Item 21A

A method for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome, comprising administering a therapeutically effective amount of a compound according to any one of Items 1A to 13A, or a pharmaceutically acceptable salt thereof, to a mammal.

### Item 22A

The method according to Item 21A, wherein the inflammatory bowel disease is ulcerative colitis or Crohn's disease.

### Item 23A

The method according to Item 21A, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Item 24A

The method according to Item 21A, wherein the disease is selected from the group consisting of multiple sclerosis, dry eye, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, frontotemporal dementia, age-related macular degeneration, diabetic macular edema, and hereditary transient corneal endotheliitis.

### Item 25A

Use of a compound according to any one of Items 1A to 13A, or a pharmaceutically acceptable salt thereof, in the manufacture of an NLRP3 inflammasome inhibitor.

### Item 26A

Use of a compound according to any one of Items 1A to 13A, or a pharmaceutically acceptable salt thereof, in the manufacture of a medicament for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome.

### Item 27A

The use according to Item 26A, wherein the inflammatory bowel disease is ulcerative colitis or Crohn's disease.

### Item 28A

The use according to Item 26A, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Item 29A

The use according to Item 26A, wherein the disease is selected from the group consisting of multiple sclerosis, dry eye, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, frontotemporal dementia, age-related macular degeneration, diabetic macular edema, and hereditary transient corneal endotheliitis.

### Item 30A

A compound according to any one of Items 1A to 13A, or a pharmaceutically acceptable salt thereof, for use in inhibiting NLRP3 inflammasome.

### Item 31A

A compound according to any one of Items 1A to 13A, or a pharmaceutically acceptable salt thereof, for use in treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome.

### Item 32A

The compound, or a pharmaceutically acceptable salt thereof, for use according to Item 31A, wherein the inflammatory bowel disease is ulcerative colitis or Crohn's disease.

### Item 33A

The compound, or a pharmaceutically acceptable salt thereof, for use according to Item 31A, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

### Item 34A

The compound, or a pharmaceutically acceptable salt thereof, for use according to Item 31A, wherein the disease is selected from the group consisting of multiple sclerosis, dry eye, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, frontotemporal dementia, age-related macular degeneration, diabetic macular edema, and hereditary transient corneal endotheliitis.

### Item 35A

A package for commercial use, comprising the pharmaceutical composition according to Item 14A and a written document associated with the pharmaceutical composition, the document describing that the composition may be used for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome.

### Item 36A

A kit for commercial use, comprising the pharmaceutical composition according to Item 14A and a written document associated with the pharmaceutical composition, the document describing that the composition may be used for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome.

### DESCRIPTION OF EMBODIMENTS

The followings are definitions of terms that may be used herein.

A wavy line as follows: 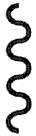 in a chemical formula herein refers to a binding site of the moiety or group represented by the chemical formula.

The term "C₁₋₄ alkyl" refers to a straight- or branched-chain saturated hydrocarbon group having 1 to 4 carbon atoms. "C₁₋₄ alkyl" includes methyl, ethyl, n-propyl, isopropyl, n-butyl, sec-butyl, isobutyl, and tert-butyl. Preferably, methyl and ethyl are included. More preferably, methyl is included.

The term "C₁₋₆ alkyl" refers to a straight- or branched-chain saturated hydrocarbon group having 1 to 6 carbon atoms. "C₁₋₆ alkyl" includes, for example, methyl, ethyl, n-propyl, isopropyl, n-butyl, sec-butyl, isobutyl, tert-butyl, n-pentyl, isopentyl, neopentyl, 2-methylbutyl, 1,1-dimethylpropyl, 1-ethylpropyl, n-hexyl, isohexyl, 1,1-dimethylbutyl, 2,2-dimethylbutyl, 3,3-dimethylbutyl, and 2-ethylbutyl. Preferably, methyl and ethyl are included. More preferably, methyl is included.

The term "C₂₋₆ alkenyl" refers to a straight- or branched-chain unsaturated hydrocarbon group having 2 to 6 carbon atoms and comprising at least one double bond. "C₂₋₆ alkenyl" includes, for example, vinyl, allyl, 1-propenyl, isopropenyl, 2-methyl-1-propenyl, 1-butenyl, 2-butenyl, 1,3-butadienyl, 3-methyl-2-butenyl, 1,1-dimethyl-2-propenyl, 4-methyl-2-pentenyl, 4-methyl-3-pentenyl, and 1-methyl-2-butenyl.

The term "C₂₋₅ alkynyl" refers to a straight- or branched-chain unsaturated hydrocarbon group having 2 to 5 carbon atoms and comprising at least one triple bond. "C₂₋₅ alkynyl" includes, for example, ethynyl, 1-propynyl, 2-propynyl, 1-butynyl, 2-butynyl, 3-butynyl, and 2-pentynyl.

The term "C₁₋₄ alkoxy" refers to a group wherein the above-defined "C₁₋₄ alkyl" binds to an oxygen atom. "C₁₋₄ alkoxy" includes methoxy, ethoxy, n-propoxy, isopropoxy, n-butoxy, sec-butoxy, isobutoxy, and tert-butoxy. Preferably, methoxy is included.

The term "C₁₋₆ alkoxy" refers to a group wherein the above-defined "C₁₋₆ alkyl" binds to an oxygen atom. "C₁₋₆ alkoxy" includes, for example, methoxy, ethoxy, n-propoxy, isopropoxy, n-butoxy, sec-butoxy, isobutoxy, tert-butoxy, pentyloxy, isopentyloxy, neopentyloxy, 2-methylbutoxy, 1,1-dimethylpropoxy, 1-ethylpropoxy, hexyloxy, isohexyloxy, 1,1-dimethylbutoxy, 2,2-dimethylbutoxy, 3,3-dimethylbutoxy, and 2-ethylbutoxy.

The term "halogen" includes, for example, fluorine, chlorine, bromine, and iodine. Preferably, fluorine, chlorine, and bromine are included.

The term "C₁₋₄ haloalkyl" refers to the above-defined "C₁₋₄ alkyl" that is substituted with 1 to 7 halogen atoms independently selected from the group of the above-defined "halogen". "C₁₋₄ haloalkyl" includes, for example, monofluoromethyl, difluoromethyl, trifluoromethyl, chlorofluoromethyl, 2-fluoroethyl, 2-chloroethyl, 2-bromoethyl, 1,1-difluoroethyl, 2,2-difluoroethyl, 1-fluoro-1-methylethyl, 2,2,2-trifluoro-1-methylethyl, 2,2,2-trifluoroethyl, pentafluoroethyl, 3-fluoropropyl, 3-chloropropyl, 1,1-difluoropropyl, 3,3,3-trifluoropropyl, and 4,4,4-trifluorobutyl. Preferably, difluoromethyl and trifluoromethyl are included.

The term "C₁₋₆ haloalkyl" refers to the above-defined "C₁₋₆ alkyl" that is substituted with 1 to 9 halogen atoms independently selected from the group of the above-defined "halogen". "C₁₋₆ haloalkyl" includes, for example, monofluoromethyl, difluoromethyl, trifluoromethyl, chlorofluoromethyl, 2-fluoroethyl, 2-chloroethyl, 2-bromoethyl, 1,1-difluoroethyl, 2,2-difluoroethyl, 1-fluoro-1-methylethyl, 2,2,2-trifluoro-1-methylethyl, 2,2,2-trifluoroethyl, pentafluoroethyl, 3-fluoropropyl, 3-chloropropyl, 1,1-difluoropropyl, 3,3,3-trifluoropropyl, 4,4,4-trifluorobutyl, 5,5,5-trifluoropentyl, and 6,6,6-trifluorohexyl. Preferably, trifluoromethyl and 1,1-difluoroethyl are included.

The term "C₂₋₆ haloalkenyl" refers to the above-defined "C₂₋₆ alkenyl" that is substituted with 1 to 9 halogen atoms independently selected from the group of the above-defined "halogen". "C₂₋₆ haloalkenyl" includes, for example, 2-fluoroethenyl, 3-chloropropenyl, 2-fluoropropenyl, 1-trifluoromethylethenyl, and 4,4,4-trifluoro-2-butenyl.

The term "C₃₋₆ cycloalkyl" refers to a monocyclic saturated hydrocarbon group having 3 to 6 carbon atoms. "C₃₋₆ cycloalkyl" includes, for example, cyclopropyl, cyclobutyl, cyclopentyl, and cyclohexyl. Preferably, cyclopropyl is included.

The term "C₅₋₆ cycloalkene" refers to a monocyclic partially-unsaturated hydrocarbon ring having 5 to 6 carbon atoms and comprising at least one double bond. "C₅₋₆ cycloalkene" includes, for example, cyclopentene, cyclopentadiene, cyclohexene, and cyclohexadiene. Preferably, cyclopentene is included.

The term "C₅₋₆ cycloalkenyl" refers to a monocyclic partially-unsaturated hydrocarbon group having 5 to 6 carbon atoms and comprising at least one double bond. "C₅₋₆ cycloalkenyl" includes, for example, cyclopentenyl, cyclopentadienyl, cyclohexenyl, and cyclohexadienyl.

The term "4- to 6-membered heterocycloalkyl comprising 1 or 2 heteroatoms independently selected from the group consisting of nitrogen and oxygen atom" refers to a 4- to 6-membered monocyclic saturated heterocyclic group comprising one or two heteroatoms independently selected from the group consisting of nitrogen and oxygen atom, besides carbon atoms, as a ring-constituting atom. "4- to 6-membered heterocycloalkyl comprising 1 or 2 heteroatoms independently selected from the group consisting of nitrogen and oxygen atom" includes, for example, azetidinyl, oxetanyl, diazetidinyl, dioxetanyl, pyrrolidinyl, tetrahydrofuranyl, imidazolidinyl, pyrazolidinyl, oxazolidinyl, isoxazolidinyl, dioxolanyl, piperidinyl, tetrahydropyranyl, 1,3-diazacyclohexanyl, piperazinyl, morpholinyl, tetrahydro-1,2-oxazinyl, and dioxanyl. Preferably, oxetanyl is included.

The term "5- to 6-membered heterocycloalkyl comprising 1 or 2 heteroatoms independently selected from the group consisting of nitrogen and oxygen atom" refers to a 5- to 6-membered monocyclic saturated heterocyclic group comprising one or two heteroatoms independently selected from the group consisting of nitrogen and oxygen atom, besides carbon atoms, as a ring-constituting atom. "5- to 6-membered heterocycloalkyl comprising 1 or 2 heteroatoms independently selected from the group consisting of nitrogen and oxygen atom" includes, for example, pyrrolidinyl, tetrahydrofuranyl, imidazolidinyl, pyrazolidinyl, oxazolidinyl, isoxazolidinyl, dioxolanyl, piperidinyl, tetrahydropyranyl, 1,3-diazacyclohexanyl, piperazinyl, morpholinyl, tetrahydro-1,2-oxazinyl, and dioxanyl.

The term "5- to 7-membered heterocycloalkane comprising 1 or 2 heteroatoms independently selected from the group consisting of nitrogen and oxygen atom" refers to a 5- to 7-membered monocyclic saturated heterocycle comprising one or two heteroatoms independently selected from the group consisting of nitrogen and oxygen atom, besides carbon atoms, as a ring-constituting atom. "5- to 7-membered heterocycloalkane comprising 1 or 2 heteroatoms independently selected from the group consisting of nitrogen and oxygen atom" includes, for example, pyrrolidine, tetrahydrofuran, imidazolidine, pyrazolidine, dioxolane, oxazolidine, isoxazolidine, piperidine, tetrahydropyran, 1,2-diazacyclohexane, 1,3-diazacyclohexane, piperazine, dioxane, morpholine, tetrahydro-1,2-oxazine, tetrahydro-1,3-oxazine, azepane, oxepane, diazepane (for example, 1,4-diazepane), dioxepane (for example, 1,4-dioxepane), and oxazepane (for example, 1,4-oxazepane and 1,2-oxazepane). Preferably, pyrrolidine, morpholine, and tetrahydro-1,3-oxazine are included.

The term "5- to 7-membered heterocycloalkene comprising 1 or 2 oxygen atoms" refers to a 5- to 7-membered monocyclic partially-unsaturated heterocycle comprising one or two oxygen atoms, besides carbon atoms, as a ring-constituting atom, and comprising at least one double bond. "5- to 7-membered heterocycloalkene comprising 1 or 2 oxygen atoms" includes, for example, dihydrofuran, dioxole, dihydropyran, dihydrodioxin, pyrane, tetrahydrooxepin, dihydrodioxepin, dihydrooxepin, and dioxepin. Preferably, dihydropyran is included.

The term "5- to 7-membered heterocycloalkene comprising 1 or 2 heteroatoms independently selected from the group consisting of nitrogen and oxygen atom" refers to a 5- to 7-membered monocyclic partially-unsaturated heterocycle comprising one or two heteroatoms independently selected from the group consisting of nitrogen and oxygen atom, besides carbon atoms, as a ring-constituting atom, and comprising at least one double bond. "5- to 7-membered heterocycloalkene comprising 1 or 2 heteroatoms independently selected from the group consisting of nitrogen and oxygen atom" includes, for example, pyrroline, pyrazoline, imidazoline, dihydrofuran, dioxole, oxazoline, isoxazoline, tetrahydropyridine, tetrahydropyrimidine, tetrahydropyridazine, tetrahydropyrazine, dihydropyridine, dihydropyran, dihydrodioxin, pyrane, dihydrooxazine, tetrahydroazepine, tetrahydrodiazepine, dihydroazepine, dihydrodiazepine, tetrahydrooxepin, dihydrodioxepin, dihydrooxepin, dioxepin, tetrahydrooxazepine, and dihydrooxazepine. Preferably, pyrroline and dihydrooxazine are included.

In one embodiment of the present invention, n in Formula [I] and Formula [IA] is 0; X¹, X², X³, and X⁴ are each independently carbon, nitrogen, oxygen, or sulfur atom; X¹, X², X³, and X⁴ are combined together with the carbon atom that is adjacent to X¹ and X⁴ to form heteroaryl, wherein a total number of nitrogen, oxygen, and sulfur atoms as X¹, X², X³, or X⁴ is 1, 2, or 3, and a total number of oxygen and sulfur atoms is 0 or 1. The heteroaryl group refers to a 5-membered monocyclic aromatic heterocyclyl comprising 1 to 3 heteroatoms independently selected from the group consisting of nitrogen, oxygen, and sulfur atom, besides carbon atoms, as a ring-constituting atom, wherein a total number of oxygen and sulfur atoms is 0 or 1. The heteroaryl group includes, for example, pyrrolyl, furyl, thienyl, pyrazolyl, imidazolyl, oxazolyl, isoxazolyl, thiazolyl, isothiazolyl, oxadiazolyl (for example, 1,2,3-oxadiazolyl, 1,2,4-oxadiazolyl, 1,2,5-oxadiazolyl, and 1,3,4-oxadiazolyl), thiadiazolyl (for example, 1,2,3-thiadiazolyl, 1,2,4-thiadiazolyl, 1,2,5-thiadiazolyl, and 1,3,4-thiadiazolyl), and triazolyl (for example, 1,2,3-triazolyl and 1,2,4-triazolyl). Preferably, pyrazolyl, imidazolyl, oxazolyl, isoxazolyl, thiazolyl, 1,3,4-oxadiazolyl, 1,2,3-triazolyl, and 1,2,4-triazolyl are included. More preferably, pyrazolyl, imidazolyl, and thiazolyl are included. Still more preferably, the following groups: 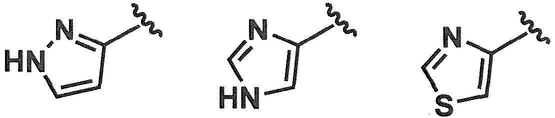 are included.

In another embodiment of the present invention, n in Formula [I] and Formula [IA] is 1; X¹, X², X³, X⁴, and X⁵ are each independently carbon or nitrogen atom; X¹, X², X³, X⁴, and X⁵ are combined together with the carbon atom that is adjacent to X¹ and X⁵ to form heteroaryl, wherein a total number of nitrogen atoms as X¹, X², X³, X⁴, or X⁵ is 1 or 2. The heteroaryl group refers to a 6-membered monocyclic aromatic heterocyclyl comprising 1 or 2 nitrogen atoms, besides carbon atoms, as a ring-constituting atom. The heteroaryl group includes, for example, pyridyl, pyrimidyl, pyridazinyl, and pyrazinyl. Preferably, pyrimidyl and pyridazinyl are included. More preferably, the following groups: 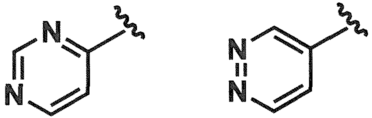 are included.

In certain embodiments of the present invention, a partial structure in Formula [IA]: 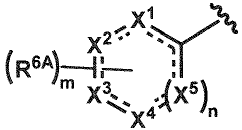 wherein adjacent two R^{6A}s are combined together with adjacent two of X¹, X², X³, X⁴, and X⁵ to which they attach to form a ring structure forms, as a whole, a 8- to 11-membered partially-unsaturated fused ring group comprising 1 to 5 heteroatoms independently selected from the group consisting of nitrogen, oxygen, and sulfur atom. The fused ring group includes, for example, the following groups:

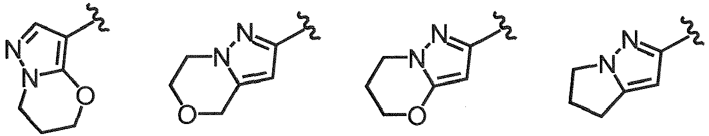

In certain embodiments of the present invention, a partial structure in Formula [IA]: 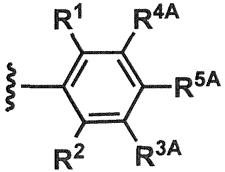 wherein R^{5A} is combined together with R^{3A} or R^{4A} and the carbon atoms to which they attach to form a ring structure forms, as a whole, a 9- to 11-membered partially-unsaturated fused ring group optionally comprising 1 or 2 oxygen atoms, the fused ring group being substituted with R¹ and R² and R^{3A} or R^{4A}. The fused ring group includes, for example, the following groups:

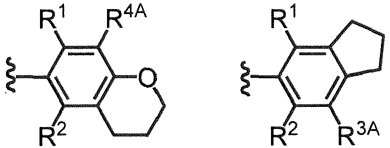

The phrase wherein α may be "optionally substituted with" β means that α is unsubstituted, or any of replaceable hydrogen atoms of α is replaced with β. For example, "C₁₋₆ alkyl optionally substituted with hydroxy" means that C₁₋₆ alkyl is unsubstituted, or any of hydrogen atoms of C₁₋₆ alkyl is replaced with hydroxy.

Embodiments of each substituent of a compound of Formula [I] or [IA] are illustrated as below. Each substituent of a compound of Formula [I] or [IA] is, however, not limited to these embodiments, and a compound of Formula [I] or [IA] also includes any combination of two or more of these embodiments in each substituent.

R¹ and R² are preferably each independently
(1) hydrogen,
(2) C₁₋₆ alkyl, wherein the alkyl may be optionally substituted with 1 or 2 substituents independently selected from the group consisting of:
   (a) hydroxy,
   (b) C₁₋₄ alkoxy, and
   (c) C₃₋₆ cycloalkyl,
(3) C₁₋₆ alkoxy, wherein the alkoxy may be optionally substituted with C₃₋₆ cycloalkyl,
(4) halogen,
(5) C₁₋₄ haloalkyl,
(6) -O-C₁₋₄ haloalkyl, or
(7) C₃₋₆ cycloalkyl.

R¹ and R² are preferably each independently
(1) hydrogen,
(2) C₁₋₆ alkyl, wherein the alkyl may be optionally substituted with 1 or 2 substituents independently selected from the group consisting of:
   (a) hydroxy,
   (b) C₁₋₄ alkoxy, and
   (c) C₃₋₆ cycloalkyl, or
(3) halogen.

R¹ and R² are more preferably each independently C₁₋₆ alkyl, wherein the alkyl may be optionally substituted with hydroxy or C₁₋₄ alkoxy.

R³ and R⁴ are preferably hydrogen.

R⁵ is preferably
(1) hydrogen,
(2) C₁₋₆ alkyl,
(3) C₁₋₄ alkoxy,
(4) halogen,
(5) C₁₋₆ haloalkyl,
(6) -O-C₁₋₄ haloalkyl, or
(7) C₃₋₆ cycloalkyl, wherein the cycloalkyl may be optionally substituted with 1 to 3 halogen or C₁₋₄ haloalkyl.

In the following partial structure: 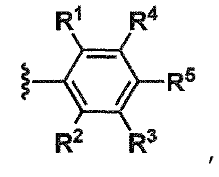 preferably, R¹ and R² are each independently
(1) hydrogen,
(2) C₁₋₆ alkyl, wherein the alkyl may be optionally substituted with 1 or 2 substituents independently selected from the group consisting of:
   (a) hydroxy,
   (b) C₁₋₄ alkoxy, and
   (c) C₃₋₆ cycloalkyl,
(3) C₁₋₆ alkoxy, wherein the alkoxy may be optionally substituted with C₃₋₆ cycloalkyl,
(4) halogen,
(5) C₁₋₄ haloalkyl,
(6) -O-C₁₋₄ haloalkyl, or
(7) C₃₋₆ cycloalkyl;
   R³ and R⁴ are preferably hydrogen; and
   R⁵ is

(1) hydrogen,
(2) C₁₋₆ alkyl,
(3) C₁₋₄ alkoxy,
(4) halogen,
(5) C₁₋₆ haloalkyl,
(6) -O-C₁₋₄ haloalkyl, or
(7) C₃₋₆ cycloalkyl, wherein the cycloalkyl may be optionally substituted with 1 to 3 halogen or C₁₋₄ haloalkyl.

R^{3A} and R^{4A} are preferably each independently hydrogen or C₁₋₄ alkyl.

R^{3A} and R^{4A} are more preferably hydrogen.

R^{5A} is preferably
(1) hydrogen,
(2) C₁₋₆ alkyl,
(3) C₁₋₄ alkoxy,
(4) halogen,
(5) C₁₋₆ haloalkyl,
(6) -O-C₁₋₄ haloalkyl, or
(7) C₃₋₆ cycloalkyl, wherein the cycloalkyl may be optionally substituted with 1 to 3 substituents independently selected from the group consisting of:
   (a) hydroxy,
   (b) halogen, and
   (c) C₁₋₄ haloalkyl, or
   R^{5A} is combined together with R^{3A} or R^{4A} and the carbon atoms that they attach to form

(1) C₅₋₆ cycloalkene, or
(2) 5- to 7-membered heterocycloalkene comprising 1 or 2 oxygen atoms.

R^{5A} is more preferably
(1) halogen,
(2) C₁₋₆ haloalkyl, or
(3) C₃₋₆ cycloalkyl, wherein the cycloalkyl may be optionally substituted with 1 to 3 substituents independently selected from the group consisting of:
   (a) hydroxy,
   (b) halogen, and
   (c) C₁₋₄ haloalkyl.

R^{5A} is still more preferably
(1) halogen, or
(2) C₃₋₆ cycloalkyl, wherein the cycloalkyl may be optionally substituted with 1 to 3 substituents independently selected from the group consisting of:
   (a) hydroxy,
   (b) halogen, and
   (c) C₁₋₄ haloalkyl.

R^{5A} is still more preferably C₃₋₆ cycloalkyl, wherein the cycloalkyl may be optionally substituted with 1 to 3 substituents independently selected from the group consisting of:
(a) hydroxy,
(b) halogen, and
(c) C₁₋₄ haloalkyl.

In the following partial structure: 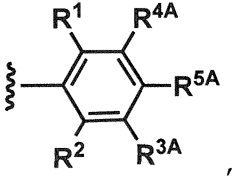 preferably, R¹ and R² are each independently
(1) hydrogen,
(2) C₁₋₆ alkyl, wherein the alkyl may be optionally substituted with 1 or 2 substituents independently selected from the group consisting of:
   (a) hydroxy,
   (b) C₁₋₄ alkoxy, and
   (c) C₃₋₆ cycloalkyl, or
(3) halogen;
   R^{3A} and R^{4A} are each independently hydrogen or C₁₋₄ alkyl;
   R^{5A} is

(1) hydrogen,
(2) C₁₋₆ alkyl,
(3) C₁₋₄ alkoxy,
(4) halogen,
(5) C₁₋6 haloalkyl,
(6) -O-C₁₋₄ haloalkyl, or
(7) C₃₋₆ cycloalkyl, wherein the cycloalkyl may be optionally substituted with 1 to 3 substituents independently selected from the group consisting of:
   (a) hydroxy,
   (b) halogen, and
   (c) C₁₋₄ haloalkyl, or
   R^{5A} is combined together with R^{3A} or R^{4A} and the carbon atoms that they attach to form

(1) C₅₋₆ cycloalkene, or
(2) 5- to 7-membered heterocycloalkene comprising 1 or 2 oxygen atoms.

In the following partial structure: 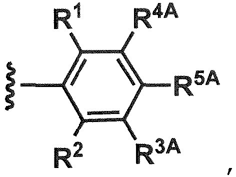 more preferably, R¹ and R² are each independently C₁₋₆ alkyl,
wherein the alkyl may be optionally substituted with hydroxy or C₁₋₄ alkoxy;
R^{3A} and R^{4A} are hydrogen;
R^{5A} is

(1) halogen,
(2) C₁₋₆ haloalkyl, or
(3) C₃₋₆ cycloalkyl, wherein the cycloalkyl may be optionally substituted with 1 to 3 substituents independently selected from the group consisting of:
   (a) hydroxy,
   (b) halogen, and
   (c) C₁₋₄ haloalkyl.

In the following partial structure: 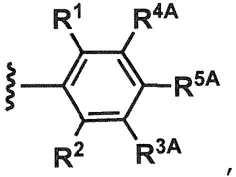 still more preferably, R¹ and R² are each independently C₁₋₆ alkyl, wherein the alkyl may be optionally substituted with hydroxy or C₁₋₄ alkoxy;
R^{3A} and R^{4A} are hydrogen;
R^{5A} is

(1) halogen, or
(2) C₃₋₆ cycloalkyl, wherein the cycloalkyl may be optionally substituted with 1 to 3 substituents independently selected from the group consisting of:
   (a) hydroxy,
   (b) halogen, and
   (c) C₁₋₉ haloalkyl.

In the following partial structure: 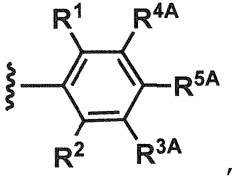 still more preferably, R¹ and R² are each independently C₁₋₆ alkyl, wherein the alkyl may be optionally substituted with hydroxy or C₁₋₄ alkoxy;
R^{3A} and R^{4A} are hydrogen;
R^{5A} is C₃₋₆ cycloalkyl, wherein the cycloalkyl may be optionally substituted with 1 to 3 substituents independently selected from the group consisting of:
   (a) hydroxy,
   (b) halogen, and
   (c) C₁₋₄ haloalkyl.

R⁶ is preferably
(1) C₁₋₉ alkyl, wherein the alkyl may be optionally substituted with C₁₋₉ alkoxy,
(2) C₁₋₄ alkoxy,
(3) halogen, or
(4) C₁₋₄ haloalkyl.

R^{6A} is preferably independently
(1) C₁₋₄ alkyl, wherein the alkyl may be optionally substituted with C₁₋₄ alkoxy,
(2) C₁₋₄ alkoxy,
(3) halogen, or
(4) C₁₋₄ haloalkyl.

R^{6A} is more preferably independently C₁₋₄ alkyl, wherein the alkyl may be optionally substituted with C₁₋₄ alkoxy.

m is preferably 0 or 1.

X¹ is preferably carbon, nitrogen, or oxygen atom.

X¹ is more preferably carbon or nitrogen atom.

X² is preferably carbon or nitrogen atom.

X³ is preferably carbon, nitrogen or sulfur atom.

X⁴ is preferably carbon, nitrogen, or sulfur atom.

X⁴ is more preferably carbon atom.

X⁵ is preferably carbon atom.

n is preferably 0.

In the following partial structure: 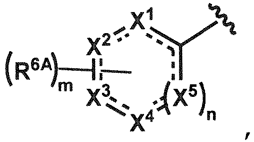 when n is 0, then preferably,
m is 0 or 1;
R^{6A} is
   (1) C₁₋₄ alkyl, wherein the alkyl may be optionally substituted with C₁₋₄ alkoxy,
   (2) C₁₋₄ haloalkyl, or
   (3) 4- to 6-membered heterocycloalkyl comprising 1 or 2 heteroatoms independently selected from the group consisting of nitrogen and oxygen atom;
X¹ is carbon, nitrogen, or oxygen atom;
X² is carbon or nitrogen atom;
X³ is carbon, nitrogen, oxygen, or sulfur atom;
X⁴ is carbon, nitrogen, or sulfur atom, wherein a total number of nitrogen, oxygen, and sulfur atoms as X¹, X², X³, or X⁴ is 1, 2, or 3 and a total number of oxygen and sulfur atoms is 0 or 1; and
X¹, X², X³, and X⁴ are combined together with the carbon atom that is adjacent to X¹ and X⁴ to form heteroaryl.

In the following partial structure: 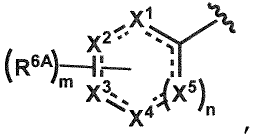 when n is 0, then more preferably,
m is 0 or 1;
R^{6A} is
   (1) C₁₋₄ alkyl, wherein the alkyl may be optionally substituted with C₁₋₄ alkoxy,
   (2) C₁₋₄ haloalkyl, or
   (3) 4- to 6-membered heterocycloalkyl comprising 1 or 2 heteroatoms independently selected from the group consisting of nitrogen and oxygen atom;
X¹ is carbon, nitrogen, or oxygen atom;
X² is carbon or nitrogen atom;
X³ is carbon, nitrogen, oxygen, or sulfur atom;
X⁴ is carbon, nitrogen, or sulfur atom, wherein a total number of nitrogen, oxygen, and sulfur atoms as X¹, X², X³, or X⁴ is 2 or 3 and a total number of oxygen and sulfur atoms is 0 or 1; and
X¹, X², X³, and X⁴ are combined together with the carbon atom that is adjacent to X¹ and X⁴ to form heteroaryl.

In the following partial structure: 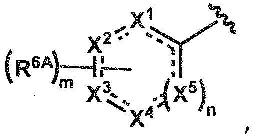 when n is 0, then still more preferably,
m is 0 or 1;
R^{6A} is C₁₋₄ alkyl, wherein the alkyl may be optionally substituted with C₁₋₄ alkoxy;
X¹ is nitrogen atom;
X² is carbon or nitrogen atom;
X³ is carbon, nitrogen, or sulfur atom;
X⁴ is carbon atom, wherein a total number of nitrogen and sulfur atoms as X¹, X², or X³ is 2; and
X¹, X², X³, and X⁴ are combined together with the carbon atom that is adjacent to X¹ and X⁴ to form heteroaryl.

The following partial structure: 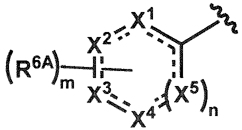 is, when n is 0, still more preferably any one of the following groups: 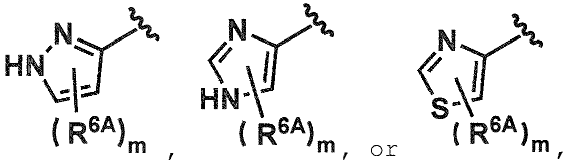 wherein m is 0 or 1; and
R^{6A} is C₁₋₄ alkyl, wherein the alkyl may be optionally substituted with C₁₋₄ alkoxy.

In the following partial structure: 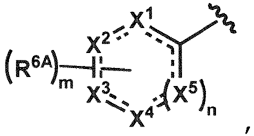 when n is 1, then preferably,
m is 0 or 1;
R^{6A} is
   (1) C₁₋₄ alkoxy,
   (2) halogen, or
   (3) C₁₋₄ haloalkyl;
X¹, X², X³, and X⁴ are each independently carbon or nitrogen atom, wherein a total number of nitrogen atoms as X¹, X², X³, or X⁴ is 1 or 2;
X⁵ is carbon atom; and
X¹, X², X³, X⁴, and X⁵ are combined together with the carbon atom that is adjacent to X¹ and X⁵ to form heteroaryl.

In the following partial structure: 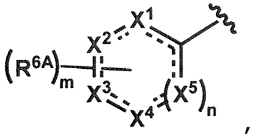 when n is 1, then more preferably,
m is 0;
X¹ and X² are each independently carbon or nitrogen atom;
X³ is nitrogen atom, wherein a total number of nitrogen atoms as X¹, X², or X³ is 2;
X⁴ and X⁵ are carbon atom; and
X¹, X², X³, X⁴, and X⁵ are combined together with the carbon atom that is adjacent to X¹ and X⁵ to form heteroaryl.

The following partial structure: 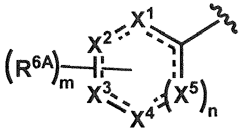 is, when n is 1, still more preferably either of the following groups:

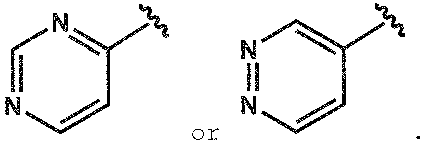

In the following partial structure: 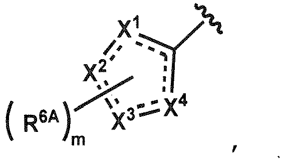 preferably,
m is 0 or 1;
R^{6A} is
   (1) C₁₋₄ alkyl, wherein the alkyl may be optionally substituted with C₁₋₄ alkoxy,
   (2) C₁₋₄ haloalkyl, or
   (3) 4- to 6-membered heterocycloalkyl comprising 1 or 2 heteroatoms independently selected from the group consisting of nitrogen and oxygen atom;
X¹ is carbon, nitrogen, or oxygen atom;
X² is carbon or nitrogen atom;
X³ is carbon, nitrogen, oxygen, or sulfur atom;
X⁴ is carbon, nitrogen, or sulfur atom, wherein a total number of nitrogen, oxygen, and sulfur atoms as X¹, X², X³, or X⁴ is 2 or 3 and a total number of oxygen and sulfur atoms is 0 or 1; and
X¹, X², X³, and X⁴ are combined together with the carbon atom that is adjacent to X¹ and X⁴ to form heteroaryl.

In the following partial structure: 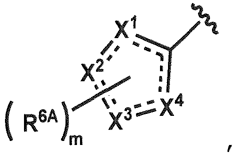 more preferably,
m is 0 or 1;
R^{6A} is C₁₋₄ alkyl, wherein the alkyl may be optionally substituted with C₁₋₄ alkoxy;
X¹ is carbon, nitrogen, or oxygen atom;
X² is carbon or nitrogen atom;
X³ is carbon, nitrogen, oxygen, or sulfur atom;
X⁴ is carbon, nitrogen, or sulfur atom, wherein a total number of nitrogen, oxygen, and sulfur atoms as X¹, X², X³, or X⁴ is 2 or 3 and a total number of oxygen and sulfur atoms is 0 or 1; and
X¹, X², X³, and X⁴ are combined together with the carbon atom that is adjacent to X¹ and X⁴ to form heteroaryl.

In the following partial structure: 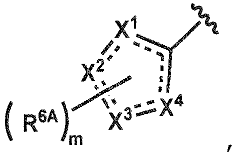 more preferably,
m is 0 or 1;
R^{6A} is C₁₋₄ alkyl, wherein the alkyl may be optionally substituted with C₁₋₄ alkoxy;
X¹ is nitrogen atom;
X² is carbon or nitrogen atom;
X³ is carbon, nitrogen, or sulfur atom;
X⁴ is carbon atom, wherein a total number of nitrogen and sulfur atoms as X¹, X², or X³ is 2; and
X¹, X², X³, and X⁴ are combined together with the carbon atom that is adjacent to X¹ and X⁴ to form heteroaryl.

The following partial structure: 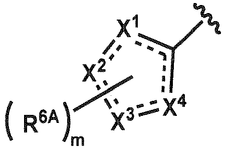 is still more preferably any one of the following groups: 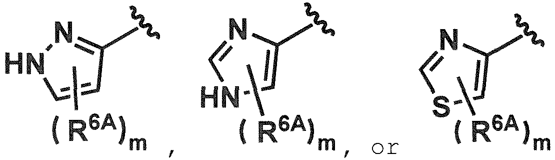 wherein m is 0 or 1; and
R^{6A} is C₁₋₄ alkyl, wherein the alkyl may be optionally substituted with C₁₋₄ alkoxy.

In the following partial structure: 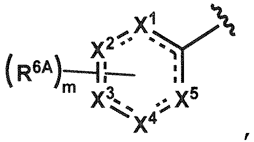 preferably,
m is 0 or 1;
R^{6A} is
   (1) C₁₋₄ alkoxy,
   (2) halogen, or
   (3) C₁₋₄ haloalkyl;
X¹, X², X³, and X⁴ are each independently carbon or nitrogen atom, wherein a total number of nitrogen atoms as X¹, X², X³, or X⁴ is 1 or 2;
X⁵ is carbon atom; and
X¹, X², X³, X⁴, and X⁵ are combined together with the carbon atom that is adjacent to X¹ and X⁵ to form heteroaryl.

In the following partial structure: 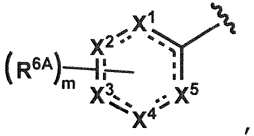 more preferably,
m is 0;
X¹ and X² are each independently carbon or nitrogen atom;
X³ is nitrogen atom, wherein a total number of nitrogen atoms as X¹, X², or X³ is 2;
X⁴ and X⁵ are carbon atom; and
X¹, X², X³, X⁴, and X⁵ are combined together with the carbon atom that is adjacent to X¹ and X⁵ to form heteroaryl.

The following partial structure: 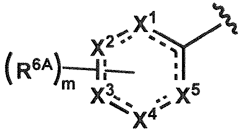 is still more preferably either of the following groups:

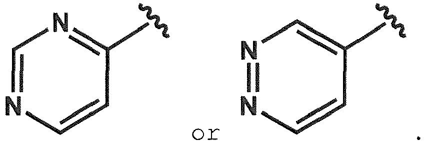

A preferable embodiment of a compound of Formula [I] is a compound of Formula [I] wherein
R¹ and R² are each independently
   (1) hydrogen,
   (2) C₁₋₆ alkyl, wherein the alkyl may be optionally substituted with 1 or 2 substituents independently selected from the group consisting of:
      (a) hydroxy,
      (b) C₁₋₄ alkoxy, and
      (c) C₃₋₆ cycloalkyl,
   (3) C₁₋₆ alkoxy, wherein the alkoxy may be optionally substituted with C₃₋₆ cycloalkyl,
   (4) halogen,
   (5) C₁₋₄ haloalkyl,
   (6) -O-C₁₋₄ haloalkyl, or
   (7) C₃₋₆ cycloalkyl;
R³ and R⁴ are preferably hydrogen;
R⁵ is
   (1) hydrogen,
   (2) C₁₋₆ alkyl,
   (3) C₁₋₄ alkoxy,
   (4) halogen,
   (5) C₁₋₆ haloalkyl,
   (6) -O-C₁₋₄ haloalkyl, or
   (7) C₃₋₆ cycloalkyl, wherein the cycloalkyl may be optionally substituted with 1 to 3 halogen or C₁₋₄ haloalkyl;
R⁶ is
   (1) C₁₋₄ alkyl, wherein the alkyl may be optionally substituted with C₁₋₄ alkoxy,
   (2) C₁₋₄ alkoxy,
   (3) halogen, or
   (4) C₁₋₄ haloalkyl;
m is 0, 1, or 2;
n is 0 or 1;
provided that when n is 0, then X¹, X², X³, and X⁴ are each independently carbon, nitrogen, oxygen, or sulfur atom, wherein a total number of nitrogen, oxygen, and sulfur atoms as X¹, X², X³, or X⁴ is 1, 2, or 3 and a total number of oxygen and sulfur atoms is 0 or 1, and X¹, X², X³, and X⁴ are combined together with the carbon atom that is adjacent to X¹ and X⁴ to form heteroaryl; and
provided that when n is 1, then X¹, X², X³, X⁴, and X⁵ are each independently carbon or nitrogen atom, wherein a total number of nitrogen atoms as X¹, X², X³, X⁴, or X⁵ is 1 or 2, and X¹, X², X³, X⁴, and X⁵ are combined together with the carbon atom that is adjacent to X¹ and X⁵ to form heteroaryl.

A preferable embodiment of a compound of Formula [IA] is a compound of Formula [IA] wherein
R¹ and R² are each independently
   (1) hydrogen,
   (2) C₁₋₆ alkyl, wherein the alkyl may be optionally substituted with 1 or 2 substituents independently selected from the group consisting of:
      (a) hydroxy,
      (b) C₁₋₄ alkoxy, and
      (c) C₃₋₆ cycloalkyl,
   (3) halogen;
R^{3A} and R^{4A} are each independently
   (1) hydrogen, or
   (2) C₁₋₄ alkyl;
R^{5A} is
   (1) hydrogen,
   (2) C₁₋₆ alkyl,
   (3) C₁₋₄ alkoxy,
   (4) halogen,
   (5) C₁₋₆ haloalkyl,
   (6) -O-C₁₋₄ haloalkyl,
   (7) C₃₋₆ cycloalkyl, wherein the cycloalkyl may be optionally substituted with 1 to 3 substituents independently selected from the group consisting of:
      (a) hydroxy,
      (b) halogen, and
      (c) C₁₋₄ haloalkyl, or
R^{5A} is combined together with R^{3A} or R^{4A} and the carbon atoms that they attach to form:
   (1) C₅₋₆ cycloalkene, or
   (2) 5- to 7-membered heterocycloalkene comprising 1 or 2 oxygen atoms;
m is 0 or 1;
R^{6A} is
   (1) C₁₋₄ alkyl, wherein the alkyl may be optionally substituted with C₁₋₄ alkoxy,
   (2) C₁₋₄ alkoxy,
   (3) halogen, or
   (4) C₁₋₄ haloalkyl;
n is 0 or 1;
provided that when n is 0, then
   X¹ is carbon, nitrogen, or oxygen atom;
   X² is carbon or nitrogen atom;
   X³ is carbon, nitrogen, oxygen, or sulfur atom;
   X⁴ is carbon, nitrogen, or sulfur atom, wherein a total number of nitrogen, oxygen, and sulfur atoms as X¹, X², X³, or X⁴ is 2 or 3 and a total number of oxygen and sulfur atoms is 0 or 1; and
   X¹, X², X³, and X⁴ are combined together with the carbon atom that is adjacent to X¹ and X⁴ to form heteroaryl; and provided that when n is 1, then
   X¹, X², X³, and X⁴ are each independently carbon or nitrogen atom, wherein a total number of nitrogen atoms as X¹, X², X³, or X⁴ is 1 or 2;
   X⁵ is carbon atom; and
   X¹, X², X³, X⁴, and X⁵ are combined together with the carbon atom that is adjacent to X¹ and X⁵ to form heteroaryl.

Another preferable embodiment of a compound of Formula [IA] is a compound of Formula [IA] wherein
R¹ and R² are each independently C₁₋₆ alkyl, wherein the alkyl may be optionally substituted with hydroxy or C₁₋₄ alkoxy;
R^{3A} and R^{4A} are hydrogen;
R^{5A} is
   (1) halogen,
   (2) C₁₋₆ haloalkyl, or
   (3) C₃₋₆ cycloalkyl, wherein the cycloalkyl may be optionally substituted with 1 to 3 substituents independently selected from the group consisting of:
      (a) hydroxy,
      (b) halogen, and
      (c) C₁₋₄ haloalkyl;
m is 0 or 1;
R^{6A} is C₁₋₄ alkyl, wherein the alkyl may be optionally substituted with C₁₋₄ alkoxy;
n is 0 or 1;
provided that when n is 0, then
   X¹ is nitrogen atom;
   X² is carbon or nitrogen atom;
   X³ is carbon, nitrogen, or sulfur atom;
   X⁴ is carbon atom, wherein a total number of nitrogen and sulfur atoms as X¹, X², or X³ is 2; and
   X¹, X², X³, and X⁴ are combined together with the carbon atom that is adjacent to X¹ and X⁴ to form heteroaryl; and provided that when n is 1, then
   X¹ and X² are each independently carbon or nitrogen atom;
   X³ is nitrogen atom, wherein a total number of nitrogen atoms as X¹, X², or X³ is 2;
   X⁴ and X⁵ are carbon atom; and
   X¹, X², X³, X⁴, and X⁵ are combined together with the carbon atom that is adjacent to X¹ and X⁵ to form heteroaryl.

Still another preferable embodiment of a compound of Formula [IA] is a compound of Formula [IIA]: 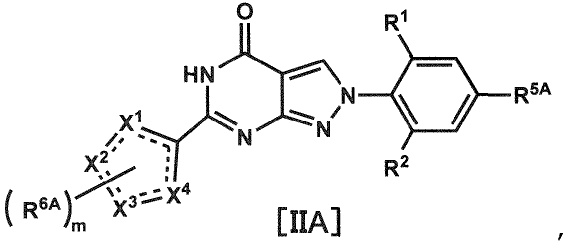 wherein R¹ and R² are each independently C₁₋₆ alkyl, wherein the alkyl may be optionally substituted with hydroxy or C₁₋₄ alkoxy;
R^{5A} is
   (1) halogen,
   (2) C₁₋₆ haloalkyl, or
   (3) C₃₋₆ cycloalkyl, wherein the cycloalkyl may be optionally substituted with 1 to 3 substituents independently selected from the group consisting of:
      (a) hydroxy,
      (b) halogen, and
      (c) C₁₋₄ haloalkyl;
m is 0 or 1;
R^{6A} is
   (1) C₁₋₄ alkyl, wherein the alkyl may be optionally substituted with C₁₋₄ alkoxy,
   (2) C₁₋₄ haloalkyl, or
   (3) 4- to 6-membered heterocycloalkyl comprising 1 or 2 heteroatoms independently selected from the group consisting of nitrogen and oxygen atom;
X¹ is carbon, nitrogen, or oxygen atom;
X² is carbon or nitrogen atom;
X³ is carbon, nitrogen, oxygen, or sulfur atom;
X⁴ is carbon, nitrogen, or sulfur atom, wherein a total number of nitrogen, oxygen, and sulfur atoms as X¹, X², X³, or X⁴ is 2 or 3 and a total number of oxygen and sulfur atoms is 0 or 1; and
X¹, X², X³, and X⁴ are combined together with the carbon atom that is adjacent to X¹ and X⁴ to form heteroaryl.

Still another preferable embodiment of a compound of Formula [IA] is a compound of Formula [IIIA]: 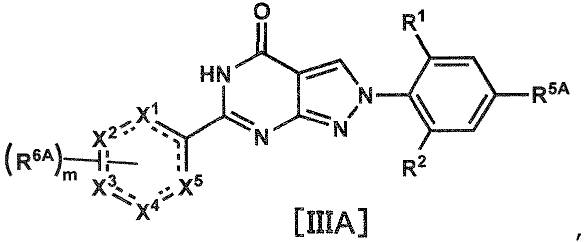 wherein R¹ and R² are each independently C₁₋₆ alkyl, wherein the alkyl may be optionally substituted with hydroxy or C₁₋₄ alkoxy;
R^{5A} is
   (1) halogen,
   (2) C₁₋₆ haloalkyl, or
   (3) C₃₋₆ cycloalkyl, wherein the cycloalkyl may be optionally substituted with 1 to 3 substituents independently selected from the group consisting of:
      (a) hydroxy,
      (b) halogen, and
      (c) C₁₋₄ haloalkyl;
m is 0 or 1;
R^{6A} is
   (1) C₁₋₄ alkoxy,
   (2) halogen, or
   (3) C₁₋₄ haloalkyl;
X¹, X², X³, and X⁴ are each independently carbon or nitrogen atom, wherein a total number of nitrogen atoms as X¹, X², X³, or X⁴ is 1 or 2;
X⁵ is carbon atom; and
X¹, X², X³, X⁴, and X⁵ are combined together with the carbon atom that is adjacent to X¹ and X⁵ to form heteroaryl.

In one preferable embodiment of a compound of Formula [IIA], R¹ and R² are each independently C₁₋₆ alkyl, wherein the alkyl may be optionally substituted with hydroxy or C₁₋₄ alkoxy;
R^{5A} is
   (1) halogen,
   (2) C₁₋₆ haloalkyl, or
   (3) C₃₋₆ cycloalkyl, wherein the cycloalkyl may be optionally substituted with 1 to 3 substituents independently selected from the group consisting of:
      (a) hydroxy,
      (b) halogen, and
      (c) C₁₋₄ haloalkyl;
m is 0 or 1;
R^{6A} is
   (1) C₁₋₄ alkyl, wherein the alkyl may be optionally substituted with C₁₋₄ alkoxy,
   (2) C₁₋₄ haloalkyl, or
   (3) 4- to 6-membered heterocycloalkyl comprising 1 or 2 heteroatoms independently selected from the group consisting of nitrogen and oxygen atom; and
X¹, X², X³, and X⁴ are combined together with the carbon atom that is adjacent to X¹ and X⁴ to form pyrazolyl, imidazolyl, or thiazolyl.

Another preferable embodiment of a compound of Formula [IIA] is a compound of Formula [IIA-I]: 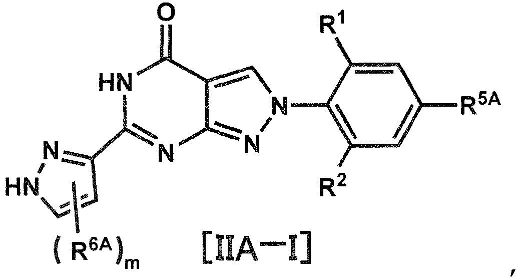 wherein R¹ and R² are each independently C₁₋₆ alkyl, wherein the alkyl may be optionally substituted with hydroxy or C₁₋₄ alkoxy;
R^{5A} is
   (1) halogen,
   (2) C₁₋₆ haloalkyl, or
   (3) C₃₋₆ cycloalkyl, wherein the cycloalkyl may be optionally substituted with 1 to 3 substituents independently selected from the group consisting of:
      (a) hydroxy,
      (b) halogen, and
      (c) C₁₋₄ haloalkyl;
m is 0 or 1;
R^{6A} is C₁₋₄ alkyl, wherein the alkyl may be optionally substituted with C₁₋₄ alkoxy.

Still another preferable embodiment of a compound of Formula [IIA] is a compound of Formula [IIA-II]: 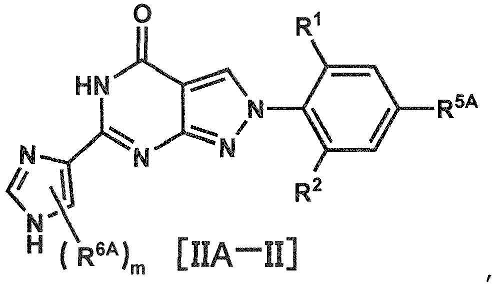 wherein R¹ and R² are each independently C₁₋₆ alkyl, wherein the alkyl may be optionally substituted with hydroxy or C₁₋₄ alkoxy;
R^{5A} is
   (1) halogen,
   (2) C₁₋₆ haloalkyl, or
   (3) C₃₋₆ cycloalkyl, wherein the cycloalkyl may be optionally substituted with 1 to 3 substituents independently selected from the group consisting of:
      (a) hydroxy,
      (b) halogen, and
      (c) C₁₋₄ haloalkyl;
m is 0 or 1;
R^{6A} is C₁₋₄ alkyl, wherein the alkyl may be optionally substituted with C₁₋₄ alkoxy.

Still another preferable embodiment of a compound of Formula [IIA] is a compound of Formula [IIA-III]: 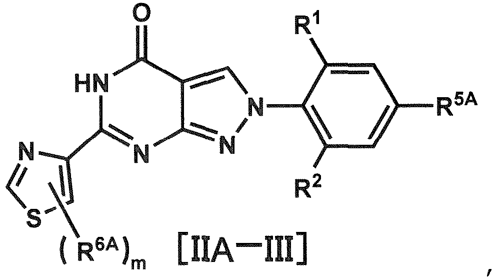 wherein R¹ and R² are each independently C₁₋₆ alkyl, wherein the alkyl may be optionally substituted with hydroxy or C₁₋₄ alkoxy;
R^{5A} is
   (1) halogen,
   (2) C₁₋₆ haloalkyl, or
   (3) C₃₋₆ cycloalkyl, wherein the cycloalkyl may be optionally substituted with 1 to 3 substituents independently selected from the group consisting of:
      (a) hydroxy,
      (b) halogen, and
      (c) C₁₋₄ haloalkyl;
m is 0 or 1;
R^{6A} is C₁₋₄ alkyl, wherein the alkyl may be optionally substituted with C₁₋₄ alkoxy.

In one preferable embodiment of a compound of Formula [IIIA], R¹ and R² are each independently C₁₋₆ alkyl, wherein the alkyl may be optionally substituted with hydroxy or C₁₋₄ alkoxy;
R^{5A} is
   (1) halogen,
   (2) C₁₋₆ haloalkyl, or
   (3) C₃₋₆ cycloalkyl, wherein the cycloalkyl may be optionally substituted with 1 to 3 substituents independently selected from the group consisting of:
      (a) hydroxy,
      (b) halogen, and
      (c) C₁₋₄ haloalkyl;
m is 0; and
X¹, X², X³, X⁴, and X⁵ are combined together with the carbon atom that is adjacent to X¹ and X⁵ to form pyridazinyl or pyrimidyl.

Another preferable embodiment of a compound of Formula [IIIA] is a compound of Formula [IIIA-I]: 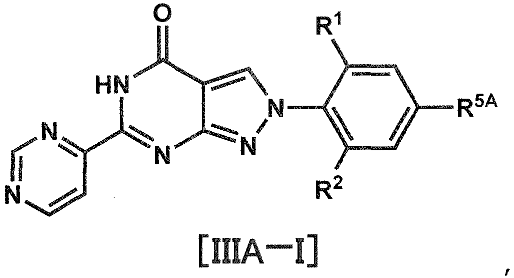 wherein R¹ and R² are each independently C₁₋₆ alkyl, wherein the alkyl may be optionally substituted with hydroxy or C₁₋₄ alkoxy;
R^{5A} is
(1) halogen,
(2) C₁₋₆ haloalkyl, or
(3) C₃₋₆ cycloalkyl, wherein the cycloalkyl may be optionally substituted with 1 to 3 substituents independently selected from the group consisting of:
   (a) hydroxy,
   (b) halogen, and
   (c) C₁₋₄ haloalkyl.

Still another preferable embodiment of a compound of Formula [IIIA] is a compound of Formula [IIIA-II]: 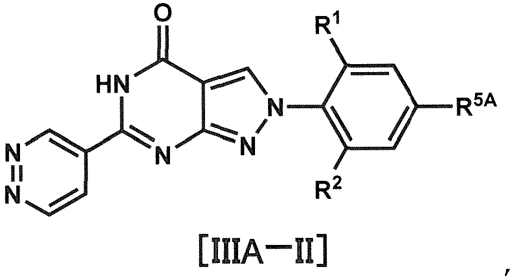 wherein R¹ and R² are each independently C₁₋₆ alkyl, wherein the alkyl may be optionally substituted with hydroxy or C₁₋₄ alkoxy;
R^{5A} is
(1) halogen,
(2) C₁₋₆ haloalkyl, or
(3) C₃₋₆ cycloalkyl, wherein the cycloalkyl may be optionally substituted with 1 to 3 substituents independently selected from the group consisting of:
   (a) hydroxy,
   (b) halogen, and
   (c) C₁₋₄ haloalkyl.

Another preferable embodiment of a compound of Formula [IA] is a compound of Formula [IV], [V], [VI], [VII], [VIII], [IX], [X], or [XI] : 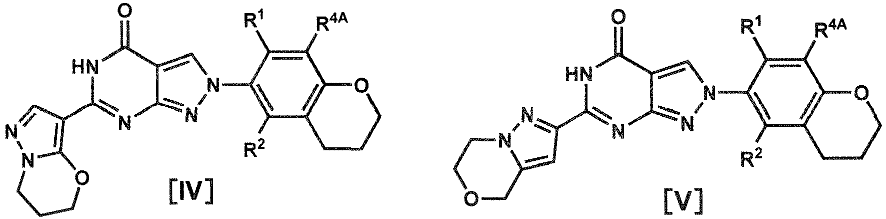 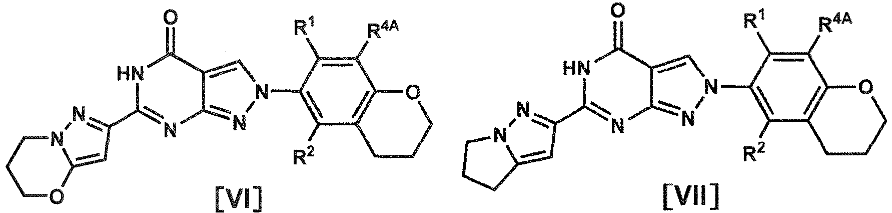 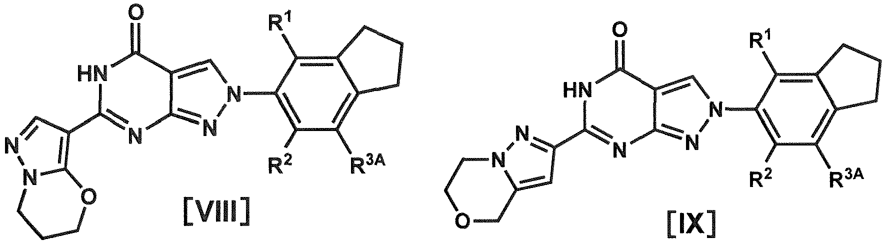 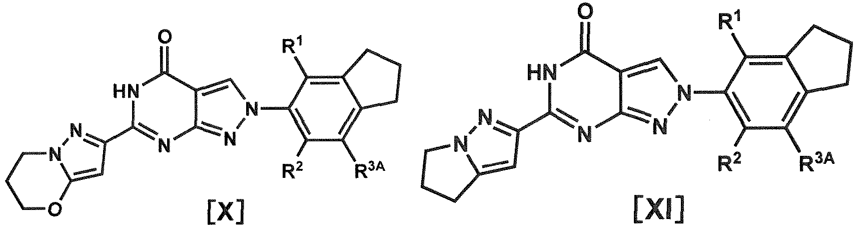 wherein R¹, R², R^{3A}, and R^{4A} are defined as those defined in Item 1A.

The term "pharmaceutically acceptable salt" used herein may be any salts known in the art that are not associated with excessive toxicity. Such a pharmaceutically acceptable salt includes, specifically, salts with inorganic acids, salts with organic acids, salts with inorganic bases, and salts with organic bases. Various forms of pharmaceutically acceptable salts are well known in the art, and are described in, for example, the following references:
(a) Berge et al., J. Pharm. Sci., 66, p1-19 (1977),
(b) Stahl et al., "Handbook of Pharmaceutical Salt: Properties, Selection, and Use" (Wiley-VCH, Weinheim, Germany, 2002),
(c) Paulekuhn et al., J. Med. Chem., 50, p6665-6672 (2007).

A compound of Formula [I] or Formula [IA] may be reacted with an inorganic acid, organic acid, inorganic base, or organic base according to methods known per se to give a corresponding pharmaceutically acceptable salt thereof.

Such a salt with inorganic acid includes salts with hydrofluoric acid, hydrochloric acid, hydrobromic acid, hydroiodic acid, nitric acid, phosphoric acid, and sulfuric acid. Such a salt preferably includes salts with hydrochloric acid, nitric acid, sulfuric acid, phosphoric acid, and hydrobromic acid.

Such a salt with organic acid includes salts with acetic acid, adipic acid, alginic acid, 4-aminosalicylic acid, anhydromethylenecitric acid, benzoic acid, benzenesulfonic acid, calcium edetate, camphor acid, camphor-10-sulfonic acid, carbonic acid, citric acid, edetic acid, ethane-1,2-disulfonic acid, dodecylsulfuric acid, ethanesulfonic acid, fumaric acid, glucoheptonic acid, gluconic acid, glucuronic acid, glucoheptonic acid, glycollylarsanilic acid, hexylresorcinol acid, hydroxynaphthoic acid, 2-hydroxy-1-ethanesulfonic acid, lactic acid, lactobionic acid, malic acid, maleic acid, mandelic acid, methanesulfonic acid, methylsulfuric acid, methylnitric acid, methylenebis(salicylic acid), galactaric acid, naphthalene-2-sulfonic acid, 2-naphthoic acid, 1,5-naphthalenedisulfonic acid, oleic acid, oxalic acid, pamoic acid, pantothenic acid, pectic acid, picric acid, propionic acid, polygalacturonic acid, salicylic acid, stearic acid, succinic acid, tannic acid, tartaric acid, teoclic acid, thiocyanic acid, trifluoroacetic acid, p-toluenesulfonic acid, undecanoic acid, aspartic acid, and glutamic acid. Such a salt preferably includes salts with oxalic acid, maleic acid, citric acid, fumaric acid, lactic acid, malic acid, succinic acid, tartaric acid, acetic acid, trifluoroacetic acid, benzoic acid, glucuronic acid, oleic acid, pamoic acid, methanesulfonic acid, benzenesulfonic acid, p-toluenesulfonic acid, and 2-hydroxy-1-ethanesulfonic acid.

Such a salt with inorganic base includes salts with lithium, sodium, potassium, magnesium, calcium, barium, aluminum, zinc, bismuth, and ammonium. Such a salt preferably includes salts with sodium, potassium, calcium, magnesium, and zinc.

Such a salt with organic base includes salts with arecoline, betaine, choline, clemizole, ethylenediamine, N-methylglucamine, N-benzylphenethylamine, tris(hydroxymethyl)methylamine, arginine, and lysine. Such a salt preferably includes salts with tris(hydroxymethyl)methylamine, N-methylglucamine, and lysine.

Compound [I] or Compound [IA] may exist in its solvate form. The term "solvate" means a compound where a solvent molecule is coordinated with, for example, Compound [I] or Compound [IA]. The solvate may be any pharmaceutically acceptable solvates; and includes, for example, a hydrate, an acetic acid solvate, an acetone solvate, an ethanolate, and a dimethyl sulfoxide solvate of Compound [I] or Compound [IA]. Such a solvate specifically includes a hemihydrate, monohydrate, dihydrate, acetic acid monosolvate, acetone monosolvate, and monoethanolate of a compound of Formula [I] or Formula [IA]; a monohydrate and acetone monosolvate of a sodium salt of a compound of Formula [I] or Formula [IA]; and a 2/3 ethanolate of a dihydrochloride salt of a compound of Formula [I] or Formula [IA]. These solvates may be obtained according to any of known methods.

Compound [I] or Compound [IA] may exist as a tautomer. In that case, Compound [I] or Compound [IA] may exist as an individual tautomer or a mixture of tautomers. For example, a structure represented by the following formula: 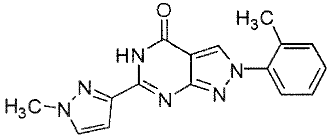 means, unless otherwise specified, that a compound represented by the structure may exist and/or be represented as:
(1) 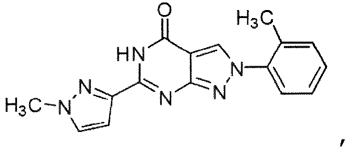
(2) 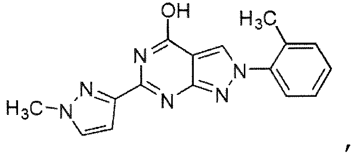
(3) 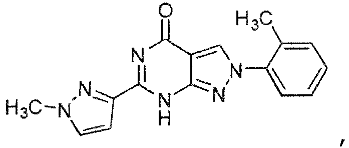
(4) 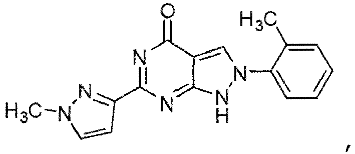 or
(5) a mixture of these structures.

Compound [I] or Compound [IA] may have a carbon-carbon double bond. In that case, Compound [I] or Compound [IA] may exist as an E-isomer, a Z-isomer, or a mixture of E- and Z-isomers.

Compound [I] or Compound [IA] may exist as a stereoisomer which should be recognized as a cis/trans isomer. In that case, Compound [I] or Compound [IA] may exist as a cis-isomer, a trans-isomer, or a mixture of cis- and trans-isomers.

Compound [I] or Compound [IA] may have one or more asymmetric carbon atoms. In that case, Compound [I] or Compound [IA] may exist as a single enantiomer, a single diastereomer, a mixture of enantiomers, or a mixture of diastereomers.

Compound [I] or Compound [IA] may exist as an atropisomer. In that case, Compound [I] or Compound [IA] may exist as an individual atropisomer or a mixture of atropisomers.

Compound [I] or Compound [IA] may simultaneously have multiple structural features that can provide the above isomers. Compound [I] or Compound [IA] may also contain the above isomers in any ratios.

Formulae, chemical structures, or chemical names without specifying a stereochemistry herein include all the above isomers which may exist, unless otherwise specified.

Diastereomer mixtures may be isolated into each diastereomer by a conventional method such as chromatography and crystallization. Each diastereomer may be also prepared by using a starting material which is a single isomer in terms of stereochemistry or by a synthetic method using a stereoselective reaction.

A mixture of enantiomers may be isolated into each single enantiomer by a well-known method in the art. For example, a mixture of enantiomers may be reacted with a substantially pure enantiomer which is known as a chiral auxiliary to form a mixture of diastereomers, which may be then isolated into a diastereomer with an enhanced isomeric ratio or a substantially pure single diastereomer by a common method such as fractionated crystallization or chromatography. The isolated diastereomer may be converted into a desirable enantiomer by removing the added chiral auxiliary under a cleavage reaction. A mixture of enantiomers may also be directly separated by chromatography well known in the art using a chiral stationary phase. Alternatively, either of enantiomers may also be obtained by using a substantially pure and optically active starting material or a stereoselective synthesis (i.e., asymmetric induction) from a prochiral intermediate with a chiral auxiliary or asymmetric catalyst.

An absolute configuration may be determined by X-ray crystallographic analysis of a crystalline product or intermediate. In that case, the crystalline product or intermediate may be, if needed, induced by an agent having an asymmetric center with a known configuration.

Compound [I] or Compound [IA] may be labeled with an isotope atom such as ²H (D), ³H, ¹¹C, ¹³C, ¹⁴C, ¹³N, ¹⁵N, ¹⁵O, ¹⁸O, ¹⁸F, ³⁵S, and ¹²³I. For example, in the case where a compound of Formula [I] or Formula [IA] has a methyl group, the methyl group may be replaced with a -CD₃ group. The compound thus obtained is also encompassed in the present invention. A compound of Formula [I] or Formula [IA] that is labeled with an isotope atom may be useful for medicines, pharmacokinetic studies, *in vitro* and/or *in vivo* assays, and/or diagnostics such as positron emission tomography (PET) and single photon emission computed tomography (SPECT). A compound of Formula [I] or Formula [IA] that is labeled with an isotope atom may be prepared using an isotope-labeled compound, instead of a corresponding non-labeled compound, according to known methods or the methods described herein.

Compound [I] or Compound [IA] is preferably a substantially purified Compound [I] or Compound [IA]. A more preferable one is Compound [I] or Compound [IA] purified in an 80% or more purity.

A pharmaceutical composition in the present invention may be prepared by optionally mixing Compound [I] or Compound [IA] with at least one or more pharmaceutically acceptable carrier(s) in a certain amount according to known methods in the art of pharmaceutical formulations. The content of Compound [I] or Compound [IA] in the pharmaceutical composition varies depending on dosage forms and doses, and is for example 0.1 to 100% by weight of the composition.

A dosage form of Compound [I] or Compound [IA] includes oral preparations such as tablets, capsules, granules, powders, lozenges, syrups, emulsions, and suspensions; and parenteral preparations such as external preparations, suppositories, injections, eye drops, nasal preparations, and pulmonary preparations.

The term "pharmaceutically acceptable carrier" used herein includes various organic or inorganic carrier substances which are conventionally used for a component of a formulation. Such substances include, for example, excipients, disintegrants, binders, fluidizers, and lubricants for solid preparations; solvents, solubilization agents, suspending agents, tonicity agents, buffering agents, and soothing agents for liquid preparations; and bases, emulsifying agents, wetting agents, stabilizers, stabilizing agents, dispersing agents, plasticizing agents, pH adjusters, absorption promoters, gelators, antiseptic agents, bulking agents, solubilizers, solubilization agents, and suspending agents for semisolid preparations. Additives such as preserving agents, antioxidant agents, coloring agents, and sweetening agents may be further used, if needed.

Such excipients include, for example, lactose, white soft sugar, D-mannitol, D-sorbitol, corn starch, dextrin, microcrystalline cellulose, crystalline cellulose, carmellose, carmellose calcium, sodium carboxymethylstarch, low-substituted hydroxypropylcellulose, and gum arabic.

Such disintegrants include, for example, carmellose, carmellose calcium, carmellose sodium, sodium carboxymethylstarch, croscarmellose sodium, crospovidone, low-substituted hydroxypropylcellulose, hydroxypropylmethyl cellulose, and crystalline cellulose.

Such binders include, for example, hydroxypropylcellulose, hydroxypropylmethyl cellulose, povidone, crystalline cellulose, white soft sugar, dextrin, starch, gelatin, carmellose sodium, and gum arabic.

Such fluidizers include, for example, light anhydrous silicic acid and magnesium stearate.

Such lubricants include, for example, magnesium stearate, calcium stearate, and talc.

Such solvents include, for example, purified water, ethanol, propylene glycol, macrogol, sesame oil, corn oil, and olive oil.

Such solubilization agents include, for example, propylene glycol, D-mannitol, benzyl benzoate, ethanol, triethanolamine, sodium carbonate, and sodium citrate.

Such suspending agents include, for example, benzalkonium chloride, carmellose, hydroxypropylcellulose, propylene glycol, povidone, methylcellulose, and glyceryl monostearate.

Such tonicity agents include, for example, glucose, D-sorbitol, sodium chloride, and D-mannitol.

Such buffering agents include, for example, sodium hydrogen phosphate, sodium acetate, sodium carbonate, and sodium citrate.

Such soothing agents include, for example, benzyl alcohol.

Such bases include, for example, water, oils from animals or vegetables such as olive oil, corn oil, arachis oil, sesame oil, and castor oil, lower alcohols such as ethanol, propanol, propylene glycol, 1,3-butylene glycol, and phenol, higher fatty acids and esters thereof, waxes, higher alcohol, polyhydric alcohol, hydrocarbons such as white petrolatum, liquid paraffin, and paraffin, hydrophilic petrolatum, purified lanolin, absorption ointment, hydrous lanolin, hydrophilic ointment, starch, pullulan, gum arabic, tragacanth gum, gelatin, dextran, cellulose derivatives such as methylcellulose, carboxymethyl cellulose, hydroxyethyl cellulose, and hydroxypropyl cellulose, synthetic polymers such as carboxyvinyl polymer, sodium polyacrylate, polyvinylalcohol, and polyvinylpyrrolidone, propylene glycol, macrogol such as Macrogol 200 to 600, and a combination of two or more of them.

Such preserving agents include, for example, ethyl parahydroxybenzoate, chlorobutanol, benzyl alcohol, sodium dehydroacetate, and sorbic acid.

Such anti-oxidant agents include, for example, sodium sulfite and ascorbic acid.

Such coloring agents include, for example, food colors (e.g., Food Red No. 2 or No. 3, Food Yellow No. 4 or No. 5) and β-carotene.

Such sweetening agents include, for example, saccharin sodium, dipotassium glycyrrhizinate, and aspartame.

A pharmaceutical composition in the present invention may be administered to human as well as mammals other than human, such as mice, rats, hamsters, guinea pigs, rabbits, cats, dogs, pigs, cattle, horses, sheep, and monkeys, orally or parenterally such as locally, rectally, intravenously, intramuscularly, and subcutaneously. While a dose, which may be referred to as "a therapeutically effective amount" herein, may vary depending on subjects, diseases, symptoms, dosage forms, routes of administration, and the like, the dose of a compound of Formula [I], or a pharmaceutically acceptable salt thereof, or a compound of Formula [IA], or a pharmaceutically acceptable salt thereof, as the active ingredient ranges generally from about 0.01 mg to 1 g per day, for example when administered orally to an adult patient. The dose may be administered once to several times in a divided amount.

Compound [I] or Compound [IA] has an inhibitory activity of NLRP3 inflammasome and is useful for treating and/or preventing various diseases or conditions that may be expected to be improved by adjusting the NLRP3 inflammasome activity. The various diseases or conditions that may be expected to be improved by adjusting the NLRP3 inflammasome activity include, for example, a disease selected from the group consisting of multiple sclerosis, chronic kidney disease, inflammatory bowel disease (for example, ulcerative colitis and Crohn's disease), arteriosclerosis, Cryopyrinassociated periodic syndrome (for example, familial cold autoinflammatory syndrome, Muckle-Wells syndrome, chronic infantile neurologic cutaneous and articular syndrome, and neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome.

The expression "inhibiting NLRP3 inflammasome" means inhibiting the function of NLRP3 inflammasome so as to disappear or reduce its activity; and, for example, inhibiting the function of NLRP3 inflammasome on the basis of the condition of Test example 1 as described below. Inhibiting the function of the NLRP3 inflammasome suppresses the production amounts of IL-1β and/or IL-18, and preferably, the production amounts of IL-1β and IL-18. Preferably, "inhibiting NLRP3 inflammasome" means "inhibiting human NLRP3 inflammasome."

Compound [I] or Compound [IA] has an NLRP3 inflammasome inhibitory activity, and Compound [I] or Compound [IA], or a pharmaceutically acceptable salt thereof, may be used as it is or may be formulated, if needed, for use as an NLRP3 inflammasome inhibitor.

The term "treating" used herein includes improving symptoms, preventing aggravation, maintaining remission, preventing exacerbation, and preventing relapse.

The term "preventing" used herein includes suppressing and delaying the onset of symptoms.

As long as an embodiment disclosed herein is compatible with other embodiments disclosed in other portions of the description, any two or more combinations of these embodiments are also intended to be included in the present invention.

### GENERAL METHOD OF PREPARATION

General methods for preparing a compound of Formula [I], or a pharmaceutically acceptable salt thereof, or a compound of Formula [IA], or a pharmaceutically acceptable salt thereof, are illustrated as below. A method for preparing a compound of Formula [I], or a pharmaceutically acceptable salt thereof, or a compound of Formula [IA], or a pharmaceutically acceptable salt thereof, is however not limited thereto.

Each compound obtained in each step may be isolated and/or purified, if necessary, according to any of known methods such as distillation, recrystallization, and column chromatography, or optionally, a subsequent step can proceed without isolation and/or purification.

Herein, the term "room temperature" refers to a temperature that has not been controlled and includes 1°C to 40°C as one embodiment.

Abbreviations used herein are as follows.
HATU: O-(7-azabenzotriazol-1-yl)-N,N,N',N'-tetramethyluronium hexafluorophosphate
WSC: 1-ethyl-3-(3-dimethylaminopropyl)carbodiimide, hydrochloride

### Preparation method A1: Preparation method of Compound [I] or a salt thereof

Compound [I] or a salt thereof may be prepared according to, for example, Preparation method A1 as follows.

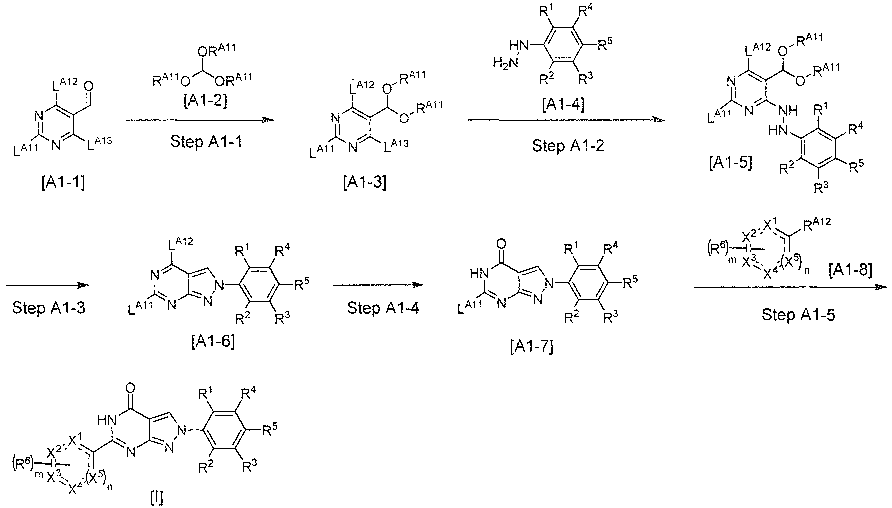

In the scheme, R¹, R², R³, R⁴, R⁵, R⁶, X¹, X², X³, X⁴, X⁵, m, and n are as defined above,
R^{A11} is each independently C₁₋₄ alkyl,
R^{A12} is boronic acid, boronic acid ester (for example, boronic acid pinacol ester), trifluoroborate, or tributyltin, and
L^{A11}, L^{A12}, and L^{A13} are each independently a leaving group (for example, halogen, methanesulfonyloxy, and p-toluenesulfonyloxy).

### (Step A1-1)

Compound [A1-3] or a salt thereof may be prepared by reacting Compound [A1-1] or a salt thereof with Compound [A1-2] in a solvent in the presence of an acid.

The acid includes, for example, sulfuric acid, hydrochloric acid, formic acid, perchloric acid, methanesulfonic acid, and p-toluenesulfonic acid. The acid is preferably sulfuric acid or p-toluenesulfonic acid.

The solvent includes, for example, toluene, methanol, ethanol, isopropanol, tetrahydrofuran, 1,4-dioxane, and a mixed solvent thereof. The solvent is preferably toluene.

The reaction temperature ranges, for example, from 0°C to 150°C, preferably from 5°C to 40°C.

Compound [A1-1] or a salt thereof is commercially available, or may be prepared from commercially available products according to known methods.

Compound [A1-2] is commercially available, or may be prepared from commercially available products according to known methods.

### (Step A1-2)

Compound [A1-5] or a salt thereof may be prepared by reacting Compound [A1-3] or a salt thereof with Compound [A1-4] or a salt thereof in a solvent in the presence of a base.

The base includes, for example, triethylamine, 1,8-diazabicyclo[5,4,0]-7-undecene, and N,N-diisopropylethylamine. The base is preferably triethylamine or N,N-diisopropylethylamine.

The solvent includes, for example, toluene, methanol, ethanol, tetrahydrofuran, and a mixed solvent thereof. The solvent is preferably toluene or methanol.

The reaction temperature ranges, for example, from - 78°C to 100°C, preferably from 0°C to 40°C.

Compound [A1-4] or a salt thereof is commercially available, or may be prepared from commercially available products according to known methods.

### (Step A1-3)

Compound [A1-6] or a salt thereof may be prapared by reacting Compound [A1-5] or a salt thereof in a solvent in the presence of an acid.

The acid includes, for example, trifluoroacetic acid, sulfuric acid, hydrochloric acid, and triethylsilyl trifluoromethanesulfonate. The acid is preferably trifluoroacetic acid.

The solvent includes, for example, toluene, tetrahydrofuran, ethyl acetate, cyclopentyl methyl ether, dichloromethane, and a mixed solvent thereof. The solvent is preferably toluene.

The reaction temperature ranges, for example, from - 78°C to 60°C, preferably from 0°C to 40°C.

### (Step A1-4)

Compound [A1-7] or a salt thereof may be prepared by reacting Compound [A1-6] or a salt thereof in a solvent in the presence of a base.

The base includes, for example, sodium hydroxide and potassium hydroxide. The base is preferably sodium hydroxide.

The solvent includes, for example, tetrahydrofuran, 1,2-dimethoxyethane, 1,4-dioxane, chloroform, and a mixed solvent thereof. The solvent is preferably tetrahydrofuran.

The reaction temperature ranges, for example, from 0°C to 150°C, preferably from 50°C to 100°C.

### (Step A1-5)

Compound [I] or a salt thereof may be prepared by reacting Compound [A1-7] or a salt thereof with Compound [A1-8] or a salt thereof in a solvent in the presence of a catalyst and a base.

The catalyst includes, for example, [1,1'-bis(di-phenylphosphino)ferrocene]palladium (II) dichloride dichloromethane adduct, tetrakis(triphenylphosphine)palladium (0), [1,1'-bis(di-tert-butylphosphino)ferrocene]palladium (II) dichloride, and bis(di-tert-butyl(4-dimethylaminophenyl)phosphine)dichloropalladium (II). The catalyst is preferably [1,1'-bis(di-phenylphosphino)ferrocene]palladium (II) dichloride dichloromethane adduct.

The base includes, for example, tripotassium phosphate, cesium carbonate, potassium carbonate, and lithium chloride. The base is preferably tripotassium phosphate.

When R^{A12} is, for example, boronic acid, boronic acid ester (such as boronic acid pinacol ester), or trifluoroborate, the solvent includes, for example, water, toluene, 1,2-dimethoxyethane, 1,4-dioxane, N,N-dimethylacetamide, and a mixed solvent thereof. The solvent is preferably a mixed solvent of toluene and water.

When R^{A12} is, for example, tributyltin, the solvent includes, for example, toluene, N,N-dimethylacetamide, N,N-dimethylformamide, and dimethylsulfoxide. The solvent is preferably N,N-dimethylacetamide.

The reaction temperature ranges, for example, from 10°C to 200°C, preferably from 50°C to 150°C.

Compound [A1-8] or a salt thereof is commercially available, or may be prepared from commercially available products according to known methods.

The Preparation method may be carried out, instead of Compound [A1-4] or a salt thereof, with a compound, or a salt thereof, having on a benzene ring a functional group or a protected substituent that may be converted into various substituents by known reactions to give a compound corresponding to Compound [I], or a salt thereof, followed by conversion of the functional group or the protected substituent into the various substituents to give Compound [I] or a salt thereof. For example, the Preparation method may be carried out, instead of Compound [A1-4] or a salt thereof, with a hydrazine compound, or a salt thereof, that is substituted with a phenyl group having L^{A41}, as mentioned below, to give a compound corresponding to Compound [I], i.e., Compound [I-A], or a salt thereof, followed by conversion of L^{A41} into Ring Cy^{A41} according to Preparation method A4 to give Compound [I-B] or a salt thereof.

### Preparation method A1A: Preparation method of Compound [IA] or a salt thereof

Compound [IA] or a salt thereof may be prepared in accordance with Preparation method A1 using Compound [A1A-4] or a salt thereof instead of Compound [A1-4] or a salt thereof, and Compound [A1A-8] or a salt thereof instead of Compound [A1-8] or a salt thereof.

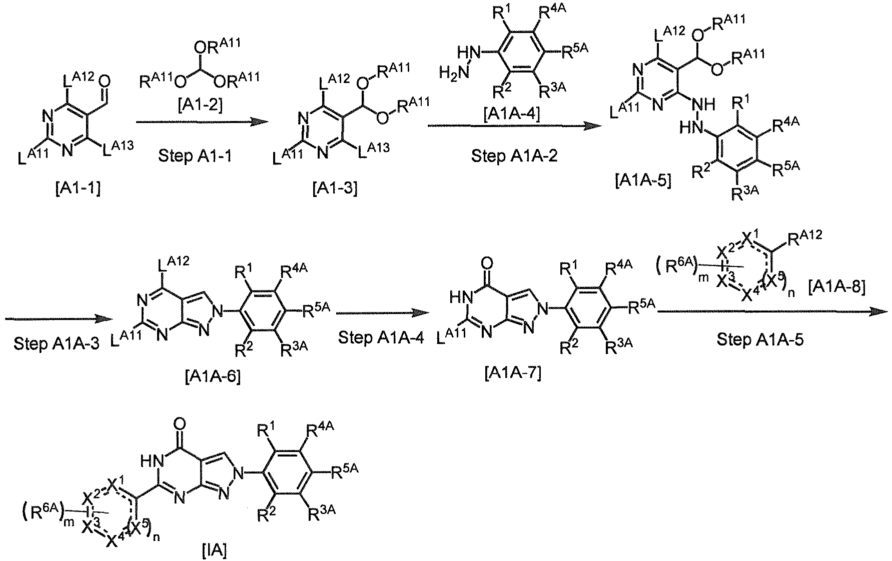

In the scheme, each symbol is as defined above.

Compound [A1A-4] or a salt thereof is commercially available, or may be prepared from commercially available products according to known methods.

Compound [A1A-8] or a salt thereof is commercially available, or may be prepared from commercially available products according to known methods.

The Preparation method may be carried out, instead of Compound [A1A-4] or a salt thereof, with a compound, or a salt thereof, having on a benzene ring a functional group or a protected substituent that may be converted into various substituents by known reactions to give a compound corresponding to Compound [IA], or a salt thereof, followed by conversion of the functional group or the protected substituent into the various substituents to give Compound [IA] or a salt thereof. For example, the Preparation method may be carried out, instead of Compound [A1A-4] or a salt thereof, with a hydrazine compound, or a salt thereof, that is substituted with a phenyl group having L^{A41}, as mentioned below, to give a compound corresponding to Compound [IA], i.e., Compound [IA-A], or a salt thereof, followed by conversion of L^{A41} into Ring Cy^{A41A} according to Preparation method A4A to give Compound [IA-B] or a salt thereof.

### Preparation method A2: Preparation method of Compound [I] or a salt thereof

Compound [I] or a salt thereof may also be prepared by, for example, Preparation method A2 as follows.

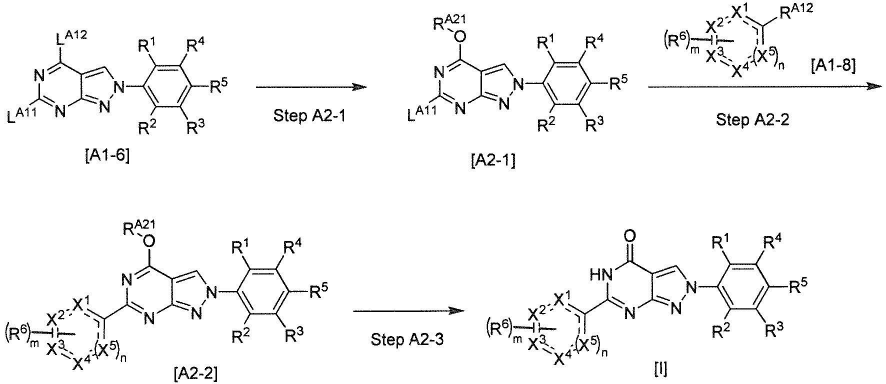

In the scheme, R¹, R², R³, R⁴, R⁵, R⁶, X¹, X², X³, X⁴, X⁵, m, n, L^{A11}, L^{A12}, and R^{A12} are as defined above,
R^{A21} is a protecting group of hydroxy group (for example, benzyl, 4-methoxybenzyl, and 2-methoxybenzyl), and R^{A21} is preferably benzyl.

### (Step A2-1)

Compound [A2-1] or a salt thereof may be prepared by reacting Compound [A1-6] or a salt thereof in a solvent in the presence of an alcohol and a base.

The alcohol includes, for example, benzyl alcohol, 4-methoxybenzyl alcohol, and 2-methoxybenzyl alcohol. The alcohol is preferably benzyl alcohol.

The base includes, for example, sodium hydride, potassium tert-butoxide, and sodium tert-butoxide. The base is preferably sodium hydride.

The solvent includes, for example, tetrahydrofuran, N,N-dimethylformamide, and a mixed solvent thereof. The solvent is preferably tetrahydrofuran.

The reaction temperature ranges, for example, from - 20°C to 100°C, preferably from 0°C to 50°C.

### (Step A2-2)

Compound [A2-2] or a salt thereof may be prepared by reacting Compound [A2-1] or a salt thereof with Compound [A1-8] or a salt thereof in a solvent in the presence of a catalyst and a base.

The catalyst includes, for example, [1,1'-bis(di-phenylphosphino)ferrocene]palladium (II) dichloride dichloromethane adduct, tetrakis(triphenylphosphine)palladium (0), [1,1'-bis(di-tert-butylphosphino)ferrocene]palladium (II) dichloride, and bis(di-tert-butyl(4-dimethylaminophenyl)phosphine)dichloropalladium (II). The catalyst is preferably [1,1'-bis(di-tert-butylphosphino)ferrocene]palladium (II) dichloride or bis(di-tert-butyl(4-dimethylaminophenyl)phosphine)dichloropalladium (II).

The base includes, for example, tripotassium phosphate, cesium carbonate, potassium carbonate, and lithium chloride. The base is preferably tripotassium phosphate or lithium chloride.

When R^{A12} is, for example, boronic acid, boronic acid ester (such as boronic acid pinacol ester), or trifluoroborate, the solvent includes, for example, water, toluene, 1,2-dimethoxyethane, 1,4-dioxane, N,N-dimethylacetamide, and a mixed solvent thereof. The solvent is preferably a mixed solvent of toluene and water.

When R^{A12} is, for example, tributyltin, the solvent includes, for example, toluene, N,N-dimethylacetamide, N,N-dimethylformamide, and dimethylsulfoxide. The solvent is preferably N,N-dimethylacetamide.

The reaction temperature ranges, for example, from 10°C to 200°C, preferably from 50°C to 150°C.

### (Step A2-3)

Compound [I] or a salt thereof may be prepared by reacting Compound [A2-2] or a salt thereof in the presence of an acid.

The acid includes, for example, formic acid, trifluoroacetic acid, and hydrochloric acid. The acid is preferably formic acid.

The reaction temperature ranges, for example, from 0°C to 120°C, preferably from 10°C to 100°C.

### Preparation method A2A: Preparation method of Compound [IA] or a salt thereof

Compound [IA] or a salt thereof may also be prepared by, for example, Preparation method A2A as follows.

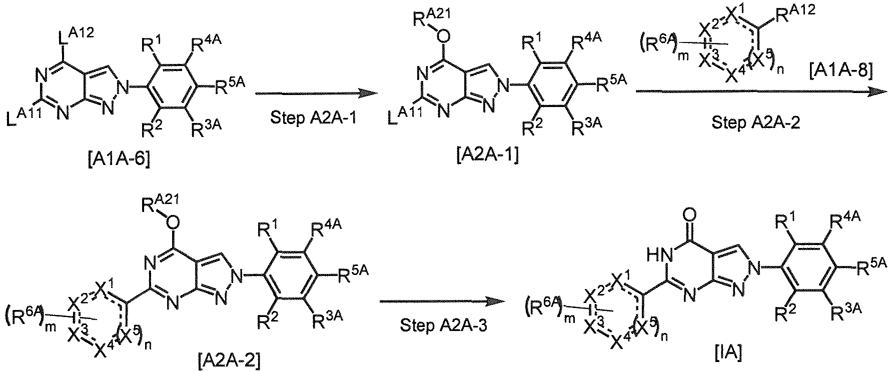

In the scheme, each symbol is as defined above.

### (Step A2A-1)

Compound [A2A-1] or a salt thereof may be prepared in accordance with Step A2-1 using Compound [A1A-6] or a salt thereof instead of Compound [A1-6] or a salt thereof.

### (Step A2A-2)

Compound [A2A-2] or a salt thereof may be prepared in accordance with Step A2-2 using Compound [A1A-8] or a salt thereof instead of Compound [A1-8] or a salt thereof.

### (Step A2A-3)

Compound [IA] or a salt thereof may be prepared by removing R^{A21} from Compound [A2A-2] or a salt thereof under a deprotection reaction. The deprotection reaction may be carried out under a suitable condition depending on R^{A21}.

For example, when R^{A21} is benzyl, Compound [IA] or a salt thereof may be prepared by reacting Compound [A2A-2] or a salt thereof in the presence of an acid. A solvent may be optionally added in the reaction.

The acid includes, for example, formic acid, trifluoroacetic acid, and hydrochloric acid. The acid is preferably formic acid.

The solvent includes, for example, toluene, tetrahydrofuran, and 1,4-dioxane.

The reaction temperature ranges, for example, from 0°C to 120°C, preferably from 10°C to 100°C.

### Preparation method A3: Preparation method of Compound [I] or a salt thereof

Compound [I] or a salt thereof may also be prepared by, for example, Preparation method A3 as follows.

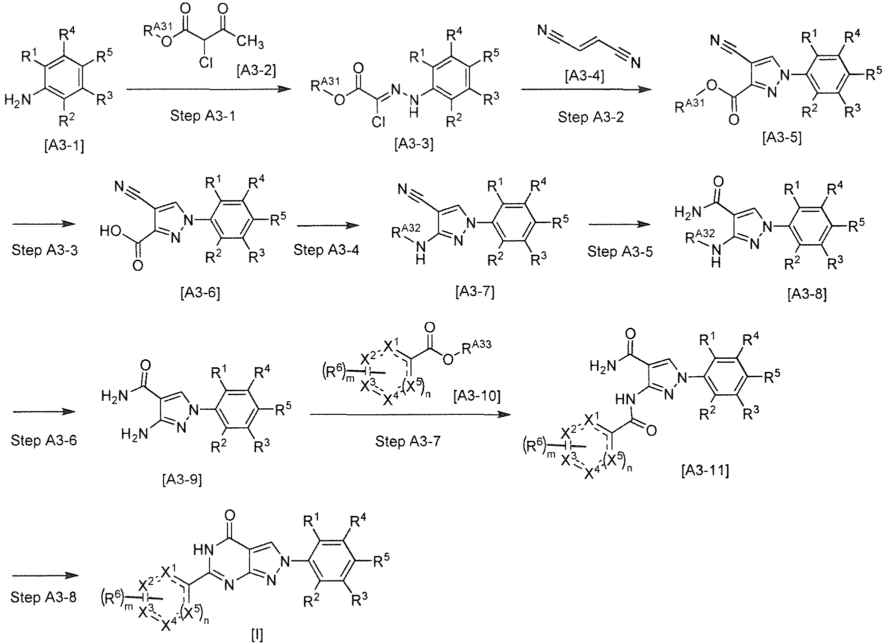

In the scheme, R¹, R², R³, R⁴, R⁵, R⁶, X¹, X², X³, X⁴, X⁵, m, and n are as defined above,
R^{A31} is C₁₋₄ alkyl,
R^{A32} is a protecting group of amino group (for example, tert-butoxycarbonyl), and
R^{A33} is hydrogen or C₁₋₄ alkyl.

### (Step A3-1)

Compound [A3-3] or a salt thereof may be prepared by reacting Compound [A3-1] or a salt thereof with Compound [A3-2] or a salt thereof in a solvent in the presence of an oxidizing agent, acid, and additive.

The oxidizing agent includes, for example, sodium nitrite, butyl nitrite, and isoamyl nitrite. The oxidizing agent is preferably sodium nitrite.

The acid includes, for example, concentrated hydrochloric acid, concentrated sulfuric acid, and nitric acid. The acid is preferably concentrated hydrochloric acid.

The additive includes, for example, sodium acetate, and potassium acetate. The additive is preferably sodium acetate.

The solvent includes, for example, ethanol, methanol, butanol, water, and a mixed solvent thereof. The solvent is preferably a mixed solvent of ethanol and water.

The reaction temperature ranges, for example, from - 40°C to 50°C, preferably from -10°C to 40°C.

Compound [A3-1] or a salt thereof is commercially available, or may be prepared from commercially available products according to known methods.

Compound [A3-2] or a salt thereof is commercially available, or may be prepared from commercially available products according to known methods.

### (Step A3-2)

Compound [A3-5] or a salt thereof may be prepared by reacting Compound [A3-3] or a salt thereof with Compound [A3-4] in a solvent in the presence of a base.

The base includes, for example, triethylamine, N,N-diisopropylethylamine, and 1,8-diazabicyclo[5,4,0]-7-undecene. The base is preferably triethylamine.

The solvent includes, for example, chloroform, 1,2-dichloroethane, dichloromethane, and a mixed solvent thereof. The solvent is preferably chloroform.

The reaction temperature ranges, for example, from 20°C to 120°C, preferably from 50°C to 100°C.

Compound [A3-4] is commercially available.

### (Step A3-3)

Compound [A3-6] or a salt thereof may be prepared by reacting Compound [A3-5] or a salt thereof in a solvent in the presence of a base.

The base includes, for example, sodium hydroxide, potassium hydroxide, lithium hydroxide, and calcium hydroxide. The base is preferably sodium hydroxide.

The solvent includes, for example, ethanol, methanol, butanol, tetrahydrofuran, water, and a mixed solvent thereof. The solvent is preferably a mixed solvent of ethanol and water.

The reaction temperature ranges, for example, from 0°C to 100°C, preferably from 5°C to 50°C.

### (Step A3-4)

Compound [A3-7] or a salt thereof may be prepared by reacting Compound [A3-6] or a salt thereof in a solvent in the presence of a reactant and a base.

The reactant includes, for example, sodium azide and diphenylphosphoryl azide. The reactant is preferably diphenylphosphoryl azide.

The base includes, for example, triethylamine, N,N-diisopropylethylamine, and 1,8-diazabicyclo[5,4,0]-7-undecene. The base is preferably triethylamine.

The solvent includes, for example, tert-butanol, benzyl alcohol, tetrahydrofuran, and a mixed solvent thereof. The solvent is preferably tert-butanol.

The reaction temperature ranges, for example, from 0°C to 150°C, preferably from 50°C to 120°C.

### (Step A3-5)

Compound [A3-8] or a salt thereof may be prepared by reacting Compound [A3-7] or a salt thereof in a solvent in the presence of an oxidizing agent and a base.

The oxidizing agent includes, for example, hydrogen peroxide solution, triiron tetraoxide, and manganese dioxide. The oxidizing agent is preferably hydrogen peroxide solution.

The base includes, for example, sodium hydroxide, potassium hydroxide, and barium hydroxide. The base is preferably sodium hydroxide.

The solvent includes, for example, ethanol, dimethylsulfoxide, water, and a mixed solvent thereof. The solvent is preferably a mixed solvent of ethanol, dimethylsulfoxide, and water.

The reaction temperature ranges, for example, from - 20°C to 50°C, preferably from 10°C to 40°C.

### (Step A3-6)

Compound [A3-9] or a salt thereof may be prepared by removing R^{A32} from Compound [A3-8] or a salt thereof under a deprotection reaction. The deprotection reaction may be carried out under a suitable condition depending on R^{A32}.

For example, when R^{A32} is tert-butoxycarbonyl, Compound [A3-9] or a salt thereof may be prepared by reacting Compound [A3-8] or a salt thereof in a solvent in the presence of an acid.

The acid includes, for example, hydrogen chloride, trifluoroacetic acid, and sulfuric acid. The acid is preferably hydrogen chloride.

The solvent includes, for example, ethyl acetate, cyclopentyl methyl ether, and a mixed solvent thereof. The solvent is preferably ethyl acetate.

The reaction temperature ranges, for example, from 0°C to 80°C, preferably from 10°C to 50°C.

Compound [A3-9] or a salt thereof may also be prepared by reversing the order of Steps A3-5 and A3-6.

### (Step A3-7)

Compound [A3-11] or a salt thereof may be prepared by reacting Compound [A3-9] or a salt thereof with Compound [A3-10] or a salt thereof in a solvent in the presence of a condensing agent and a base.

The condensing agent includes, for example, HATU, WSC, and propylphosphonic acid anhydride. The condensing agent is preferably HATU.

The base includes, for example, sodium methoxide, triethylamine, N,N-diisopropylethylamine, potassium tert-butoxide, and 1,8-diazabicyclo[5,4,0]-7-undecene. The base is preferably N,N-diisopropylethylamine.

The solvent includes, for example, methanol, N-methylpyrrolidone, N,N-dimethylacetamide, and a mixed solvent thereof. The solvent is preferably N-methylpyrrolidone.

The reaction temperature ranges, for example, from 0°C to 120°C, preferably from 50°C to 100°C.

Compound [A3-10] or a salt thereof is commercially available, or may be prepared from commercially available products according to known methods.

### (Step A3-8)

Compound [I] or a salt thereof may be prepared by reacting Compound [A3-11] or a salt thereof in a solvent in the presence of a base.

The base includes, for example, sodium methoxide, sodium tert-butoxide, and 1,8-diazabicyclo[5,4,0]-7-undecene. The base is preferably 1,8-diazabicyclo[5,4,0]-7-undecene.

The solvent includes, for example, ethanol, methanol, butanol, water, and a mixed solvent thereof. The solvent is preferably a mixed solvent of ethanol and water.

The reaction temperature ranges, for example, from 0°C to 180°C, preferably from 50°C to 150°C.

The Preparation method may be carried out, instead of Compound [A3-1] or a salt thereof, with a compound, or a salt thereof, having on a benzene ring a functional group or a protected substituent that may be converted into various substituents by known reactions to give a compound corresponding to Compound [I], or a salt thereof, followed by conversion of the functional group or the protected substituent into the various substituents to give Compound [I] or a salt thereof. For example, the Preparation method may be carried out, instead of Compound [A3-1] or a salt thereof, with a compound, or a salt thereof, having an amino group and L^{A41}, as mentioned below, on the benzene ring to give a compound corresponding to Compound [I], i.e., Compound [I-A], or a salt thereof, followed by conversion of L^{A41} into Ring Cy^{A41} according to Preparation method A4 to give Compound [I-B] or a salt thereof.

The Preparation method may be carried out, instead of Compound [A3-1] or a salt thereof, with a compound, or a salt thereof, having on a benzene ring a functional group or a protected substituent that may be converted into various substituents by known reactions to give a compound corresponding to Compound [A3-9], or a salt thereof, followed by conversion of the functional group or the protected substituent into the various substituents to give Compound [A3-9] or a salt thereof. For example, the Preparation method may be carried out, instead of Compound [A3-1] or a salt thereof, with a compound, or a salt thereof, having an amino group and L^{A41}, as mentioned below, on the benzene ring to give a compound corresponding to Compound [A3-9], i.e., Compound [A3-9-A], or a salt thereof, followed by conversion of L^{A41} into Ring Cy^{A41} according to Preparation method A5 to give Compound [A3-9-B] or a salt thereof.

### Preparation method A3A: Preparation method of Compound [IA] or a salt thereof

Compound [IA] or a salt thereof may also be prepared by, for example, Preparation method A3A as follows.

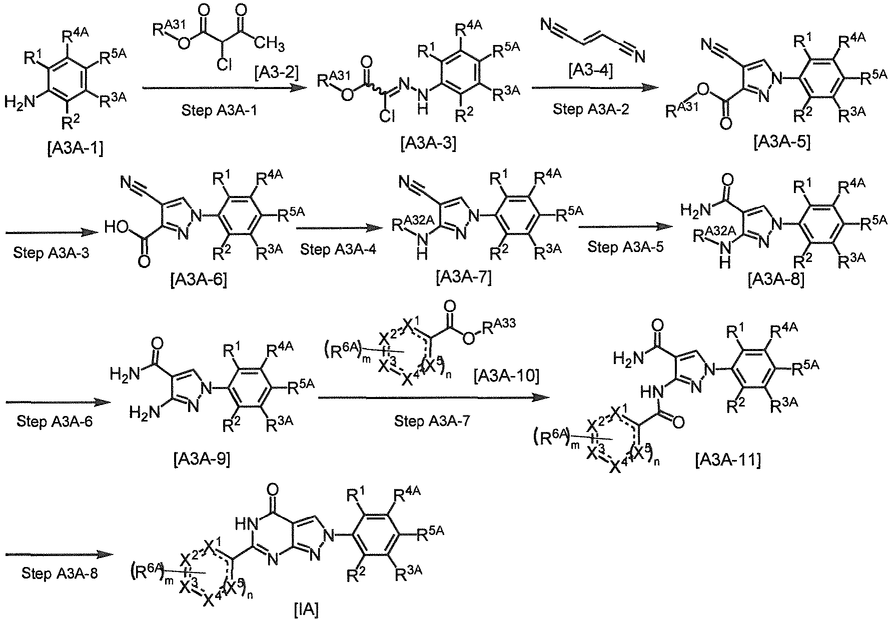

In the scheme, R¹, R², R^{3A}, R^{4A}, R^{5A}, R^{6A}, R^{A31}, R^{A33}, X¹, X², X³, X⁴, X⁵, m, and n are as defined above, and
R^{A32A} is a protecting group of hydrogen or amino group (for example, tert-butoxycarbonyl).

### (Step A3A-1)

Compound [A3A-3] or a salt thereof may be prepared in accordance with Step A3-1 using Compound [A3A-1] or a salt thereof instead of Compound [A3-1] or a salt thereof.

Compound [A3A-1] or a salt thereof is commercially available, or may be prepared from commercially available products according to known methods.

### (Step A3A-2)

Compound [A3A-5] or a salt thereof may be prepared in accordance with Step A3-2 using Compound [A3A-3] or a salt thereof instead of Compound [A3-3] or a salt thereof.

### (Step A3A-3)

Compound [A3A-6] or a salt thereof may be prepared in accordance with Step A3-3 using Compound [A3A-5] or a salt thereof instead of Compound [A3-5] or a salt thereof.

### (Step A3A-4)

Compound [A3A-7] or a salt thereof may be prepared in accordance with Step A3-4 using Compound [A3A-6] or a salt thereof instead of Compound [A3-6] or a salt thereof.

### (Step A3A-5)

Compound [A3A-8] or a salt thereof may be prepared in accordance with Step A3-5 using Compound [A3A-7] or a salt thereof instead of Compound [A3-7] or a salt thereof.

### (Step A3A-6)

Compound [A3A-9] or a salt thereof may be prepared by removing R^{A32A} from Compound [A3A-8] or a salt thereof under a deprotection reaction. The deprotection reaction may be carried out under a suitable condition depending on R^{A32A}.

For example, when R^{A32A} is tert-butoxycarbonyl, Compound [A3A-9] or a salt thereof may be prepared by reacting Compound [A3A-8] or a salt thereof in a solvent in the presence of an acid.

The acid includes, for example, hydrogen chloride, trifluoroacetic acid, and sulfuric acid. The acid is preferably hydrogen chloride.

The solvent includes, for example, ethyl acetate, cyclopentyl methyl ether, and a mixed solvent thereof. The solvent is preferably ethyl acetate.

The reaction temperature ranges, for example, from 0°C to 80°C, preferably from 10°C to 50°C.

Compound [A3A-9] or a salt thereof may also be prepared by performing Step A3A-6 for Compound [A3A-7] or a salt thereof, followed by the reaction of Step A3A-5.

### (Step A3A-7)

Compound [A3A-11] or a salt thereof may be prepared in accordance with Step A3-7 using Compound [A3A-9] or a salt thereof instead of Compound [A3-9] or a salt thereof, and Compound [A3A-10] or a salt thereof instead of Compound [A3-10] or a salt thereof.

Compound [A3A-10] or a salt thereof is commercially available, or may be prepared from commercially available products according to known methods.

### (Step A3A-8)

Compound [IA] or a salt thereof may be prepared in accordance with Step A3-8 using Compound [A3A-11] or a salt thereof instead of Compound [A3-11] or a salt thereof.

The Preparation method may be carried out, instead of Compound [A3A-1] or a salt thereof, with a compound, or a salt thereof, having on a benzene ring a functional group or a protected substituent that may be converted into various substituents by known reactions to give a compound corresponding to Compound [IA], or a salt thereof, followed by conversion of the functional group or the protected substituent into the various substituents to give Compound [IA] or a salt thereof. For example, the Preparation method may be carried out, instead of Compound [A3A-1] or a salt thereof, with a compound, or a salt thereof, having an amino group and L^{A41}, as mentioned below, on the benzene ring to give a compound corresponding to Compound [IA], i.e., Compound [IA-A], or a salt thereof, followed by conversion of L^{A41} into Ring Cy^{A41A} according to Preparation method A4A to give Compound [IA-B] or a salt thereof.

The Preparation method may be carried out, instead of Compound [A3A-1] or a salt thereof, with a compound, or a salt thereof, having on a benzene ring a functional group or a protected substituent that may be converted into various substituents by known reactions to give a compound corresponding to Compound [A3A-8], or a salt thereof, followed by conversion of the functional group or the protected substituent into the various substituents to give Compound [A3A-8] or a salt thereof. For example, the Preparation method may be carried out, instead of Compound [A3A-1] or a salt thereof, with a compound, or a salt thereof, having an amino group and L^{A41}, as mentioned below, on the benzene ring to give a compound corresponding to Compound [A3A-8], i.e., Compound [A3A-8-A], or a salt thereof, followed by conversion of L^{A41} into Ring Cy^{A41A} according to Preparation method A5A to give Compound [A3A-8-B] or a salt thereof.

### Preparation method A4: Preparation method of Compound [I-B] or a salt thereof

Compound [I-B] or a salt thereof may be prepared by, for example, Preparation method A4 as follows.

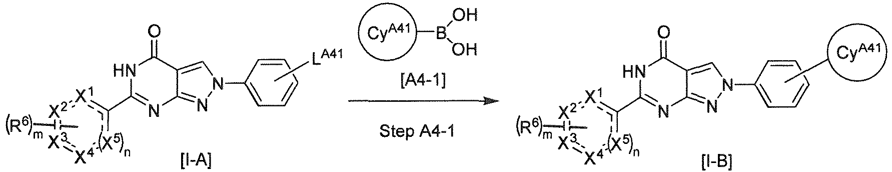

In the scheme, R⁶, X¹, X², X³, X⁴, X⁵, m, and n are as defined above,
Cy^{A41} is C₃₋₆ cycloalkyl, wherein the cycloalkyl may be optionally substituted with 1 to 3 halogen or C₁₋₄ haloalkyl, and
L^{A41} is a leaving group (for example, halogen, methanesulfonyloxy, and trifluoromethanesulfonyloxy), provided that the leaving group is attached to ortho- or para-position of the benzene ring.

### (Step A4-1)

Compound [I-B] or a salt thereof may be prepared by reacting Compound [I-A] or a salt thereof with Compound [A4-1] or a derivative thereof (for example, cyclopropylboronic acid pinacol ester and potassium cyclopropyltrifluoroborate) in a solvent in the presence of a catalyst and a base.

The catalyst includes, for example, [1,1'-bis(diphenylphosphino)ferrocene]palladium (II) dichloride dichloromethane adduct, tetrakis(triphenylphosphine)palladium (0), and [1,1'-bis(di-tert-butylphosphino)ferrocene]palladium (II) dichloride. The catalyst is preferably [1,1'-bis(di-tert-butylphosphino)ferrocene]palladium (II) dichloride.

The base includes, for example, tripotassium phosphate, cesium carbonate, and potassium carbonate. The base is preferably tripotassium phosphate.

The solvent includes, for example, water, toluene, 1,2-dimethoxyethane, 1,4-dioxane, and a mixed solvent thereof. The solvent is preferably a mixed solvent of toluene and water.

The reaction temperature ranges, for example, from 10°C to 200°C, preferably from 50°C to 150°C.

Compound [I-A] or a salt thereof may be prepared from commercially available products according to known methods. Compound [I-A] or a salt thereof may be prepared by, for example, the above-mentioned Preparation methods.

Compound [A4-1] or a derivative thereof is commercially available, or may be prepared from commercially available products according to known methods.

### Preparation method A4A: Preparation method of Compound [IA-B] or a salt thereof

Compound [IA-B] or a salt thereof may be prepared in accordance with Preparation method A4 using Compound [IA-A] or a salt thereof instead of Compound [I-A] or a salt thereof, and Compound [A4A-1] or a salt thereof instead of Compound [A4-1] or a salt thereof.

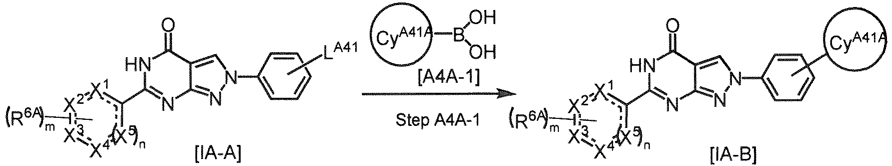

In the scheme, R^{6A}, L^{A41}, X¹, X², X³, X⁴, X⁵, m, and n are as defined above, and

Cy^{A41A} is C₃₋₆ cycloalkyl, wherein the cycloalkyl may be optionally substituted with 1 to 3 substituents independently selected from the group consisting of:
(a) hydroxy,
(b) halogen, and
(c) C₁₋₄ haloalkyl.

Compound [IA-A] or a salt thereof may be prepared from commercially available products according to known methods. Compound [IA-A] or a salt thereof may be prepared by, for example, the above-mentioned Preparation methods.

Compound [A4A-1] or a derivative thereof is commercially available, or may be prepared from commercially available products according to known methods.

### Preparation method A5: Preparation method of Compound [A3-9-B] or a salt thereof

Compound [A3-9-B] or a salt thereof may be prepared by, for example, Preparation method A5 as follows.

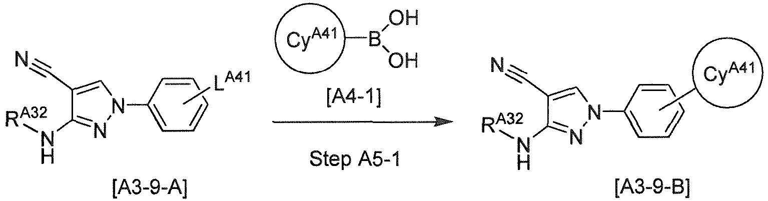

In the scheme, each symbol is as defined above.

### (Step A5-1)

Compound [A3-9-B] or a salt thereof may be prepared by reacting Compound [A3-9-A] or a salt thereof with Compound [A4-1] or a derivative thereof (for example, cyclopropylboronic acid pinacol ester and potassium cyclopropyltrifluoroborate) in a solvent in the presence of a catalyst and a base.

The catalyst includes, for example, [1,1'-bis(di-phenylphosphino)ferrocene]palladium (II) dichloride dichloromethane adduct, tetrakis(triphenylphosphine)palladium (0), and [1,1'-bis(di-tert-butylphosphino)ferrocene]palladium (II) dichloride. The catalyst is preferably [1,1'-bis(di-tert-butylphosphino)ferrocene]palladium (II) dichloride.

The base includes, for example, tripotassium phosphate, cesium carbonate, and potassium carbonate. The base is preferably tripotassium phosphate.

The solvent includes, for example, water, toluene, 1,2-dimethoxyethane, 1,4-dioxane, and a mixed solvent thereof. The solvent is preferably a mixed solvent of toluene and water.

The reaction temperature ranges, for example, from 10°C to 200°C, preferably from 50°C to 150°C.

Compound [A3-9-A] or a salt thereof may be prepared from commercially available products according to known methods. Compound [A3-9-A] or a salt thereof may be prepared by, for example, the above-mentioned Preparation methods.

### Preparation method A5A: Preparation method of Compound [A3A-8-B] or a salt thereof

Compound [A3A-8-B] or a salt thereof may be prepared by, for example, Preparation method A5A as follows.

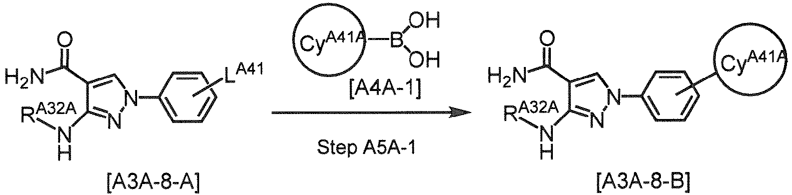

In the scheme, each symbol is as defined above.

### (Step A5A-1)

Compound [A3A-8-B] or a salt thereof may be prepared by reacting Compound [A3A-8-A] or a salt thereof with Compound [A4A-1] or a derivative thereof (for example, cyclopropylboronic acid pinacol ester and potassium cyclopropyltrifluoroborate) in a solvent in the presence of a catalyst and a base.

The catalyst includes, for example, [1,1'-bis(di-phenylphosphino)ferrocene]palladium (II) dichloride dichloromethane adduct, tetrakis(triphenylphosphine)palladium (0), and [1,1'-bis(di-tert-butylphosphino)ferrocene]palladium (II) dichloride. The catalyst is preferably [1,1'-bis(di-tert-butylphosphino)ferrocene]palladium (II) dichloride.

The base includes, for example, tripotassium phosphate, cesium carbonate, and potassium carbonate. The base is preferably tripotassium phosphate.

The solvent includes, for example, water, toluene, 1,2-dimethoxyethane, 1,4-dioxane, and a mixed solvent thereof. The solvent is preferably a mixed solvent of toluene and water.

The reaction temperature ranges, for example, from 10°C to 200°C, preferably from 50°C to 150°C.

Compound [A3A-8-A] or a salt thereof may be prepared from commercially available products according to known methods. Compound [A3A-8-A] or a salt thereof may be prepared by, for example, the above-mentioned Preparation methods.

### Preparation method A6: Preparation method of Compound [IA] or a salt thereof

Compound [IA] or a salt thereof may be prepared by, for example, Preparation method A6 as follows.

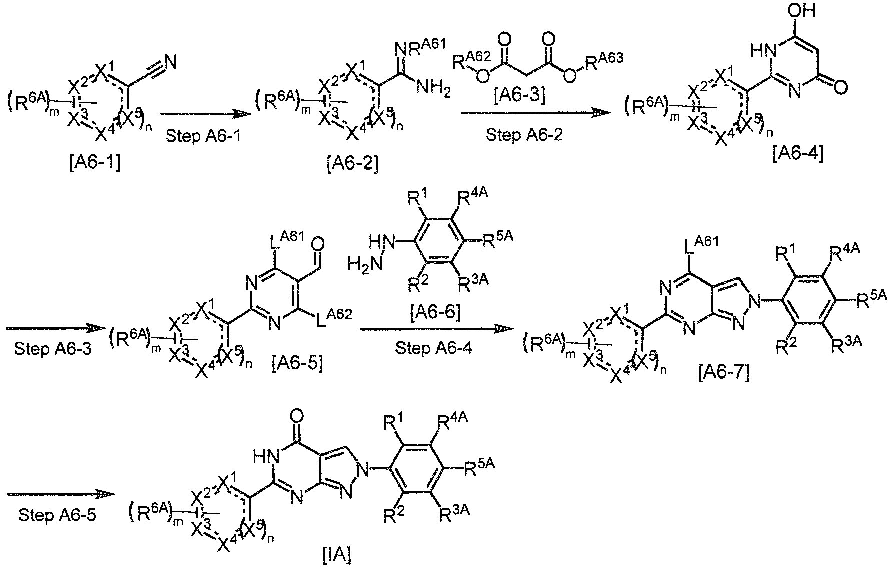

In the scheme, R¹, R², R^{3A}, R^{4A}, R^{5A}, R^{6A}, X¹, X², X³, X⁴, X⁵, m, and n are as defined above,
R^{A61} is hydrogen, or a protecting group of amino group (for example, trimethylsilyl),
R^{A62} and R^{A63} are each independently C₁₋₄ alkyl, and
L^{A61} and L^{A62} are each independently a leaving group (for example, halogen, methanesulfonyloxy, and p-toluenesulfonyloxy).

### (Step A6-1)

Compound [A6-2] or a salt thereof may be prepared by reacting Compound [A6-1] or a salt thereof in a solvent in the presence of a reactant. An acid may be optionally added in the reaction.

The reactant includes lithium bis(trimethylsilyl)amide, ammonia, and ammonium chloride. The reactant is preferably lithium bis(trimethylsilyl)amide.

The acid includes, for example, hydrogen chloride, sulfuric acid.

The solvent includes, for example, methanol, tetrahydrofuran, 1,4-dioxane, and a mixed solvent thereof. The solvent is preferably tetrahydrofuran.

The reaction temperature ranges, for example, from - 78°C to 40°C, preferably -10°C to 10°C.

Compound [A6-1] or a salt thereof is commercially available, or may be prepared from commercially available products according to known methods.

### (Step A6-2)

Compound [A6-4] or a salt thereof may be prepared by reacting Compound [A6-2] or a salt thereof with Compound [A6-3] or a salt thereof in a solvent.

The solvent includes, for example, methanol, ethanol, isopropanol, tetrahydrofuran, and a mixed solvent thereof. The solvent is preferably a mixed solvent of tetrahydrofuran and methanol.

The reaction temperature ranges, for example, from room temperature to 120°C, preferably from 60°C to 100°C.

Compound [A6-3] or a salt thereof is commercially available, or may be prepared from commercially available products according to known methods.

### (Step A6-3)

Compound [A6-5] or a salt thereof may be prepared by reacting Compound [A6-4] or a salt thereof in a solvent in the presence of the Vilsmeier reagent, followed by addition of phosphorus oxychloride and heating; the reaction temperature is, for example, 80°C.

The solvent includes, for example, N,N-dimethylformamide, toluene, benzene, and a mixed solvent thereof. The solvent is preferably a mixed solvent of N,N-dimethylformamide and toluene.

The reaction temperature ranges, for example, from - 20°C to 100°C, preferably from -10°C to 90°C.

The Vilsmeier reagent is prepared from, for example, phosphorus oxychloride and N,N-dimethylformamide.

### (Step A6-4)

Compound [A6-7] or a salt thereof may be prepared by reacting Compound [A6-5] or a salt thereof with Compound [A6-6] or a salt thereof in a solvent in the presence of a base.

The base includes, for example, triethylamine, N,N-diisopropylethylamine, and 1,8-diazabicyclo[5,4,0]-7-undecene. The base is preferably triethylamine.

The solvent includes, for example, methanol, ethanol, tetrahydrofuran, toluene, cyclopentyl methyl ether, acetonitrile, water, and a mixed solvent thereof. The solvent is preferably a mixed solvent of tetrahydrofuran and water.

The reaction temperature ranges, for example, from - 20°C to 80°C, preferably from 0°C to room temperature.

Compound [A6-6] or a salt thereof is commercially available, or may be prepared from commercially available products according to known methods.

### (Step A6-5)

Compound [IA] or a salt thereof may be prepared by reacting Compound [A6-7] or a salt thereof in a solvent in the presence of an acid.

The acid includes, for example, formic acid, trifluoroacetic acid, and hydrochloric acid. The acid is preferably trifluoroacetic acid.

The solvent includes, for example, water, acetonitrile, tetrahydrofuran, and a mixed solvent thereof. The solvent is preferably a mixed solvent of acetonitrile and water.

The reaction temperature ranges, for example, from 0°C to 100°C, preferably 50°C to 70°C.

The Preparation method may be carried out, instead of Compound [A6-6] or a salt thereof, with a compound, or a salt thereof, having on a benzene ring a functional group or a protected substituent that may be converted into various substituents by known reactions to give a compound corresponding to Compound [IA], or a salt thereof, followed by conversion of the functional group or the protected substituent into the various substituents to give Compound [IA] or a salt thereof. For example, the Preparation method may be carried out, instead of Compound [A6-6] or a salt thereof, with a hydrazine compound, or a salt thereof, that is substituted with a phenyl group having L^{A41} to give a compound corresponding to Compound [IA], i.e., Compound [IA-A], or a salt thereof, followed by conversion of L^{A41} into Ring Cy^{A41A} according to Preparation method A4A to give Compound [IA-B] or a salt thereof.

### EXAMPLES

Preparation methods of a compound of formula [I], or a pharmaceutically acceptable salt thereof, or a compound of formula [IA], or a pharmaceutically acceptable salt thereof, are described specifically in the following Preparation examples. However, preparation methods of a compound of formula [I], or a pharmaceutically acceptable salt thereof, or a compound of formula [IA], or a pharmaceutically acceptable salt thereof, are not intended to be limited thereto.

% refers to weight %, unless otherwise specified. The ratio recited in a mixed solvent refers to a volume ratio, unless otherwise specified.

NMRs were determined at 400 MHz.

### [Preparation example 1]: Synthesis of 2-(4-cyclopropyl-2,6-dimethylphenyl)-6-(1-methyl-1H-pyrazol-3-yl)-2,5-dihydro-4H-pyrazolo[3,4-d]pyrimidin-4-one (Example 4)

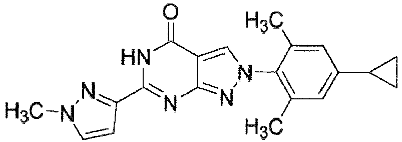

### Step 1-1: Benzyl 2-(4-bromo-2,6-dimethylphenyl)hydrazine-1-carboxylate

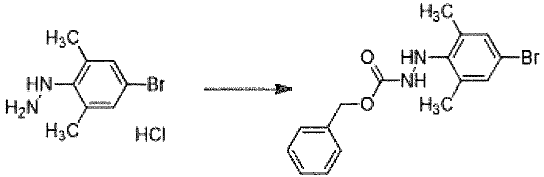

Under a nitrogen atmosphere, to a mixture of (4-bromo-2,6-dimethylphenyl)hydrazine hydrochloride (147 g) and tetrahydrofuran (1000 mL) were added N,N-diisopropylethylamine (224 mL) and benzyl chloroformate (83 mL) at 0°C, and the mixture was stirred at room temperature for 40 minutes. Toluene (441 mL) and water (441 mL) were added to the reaction mixture, which was then stirred at room temperature for 20 minutes. After separation, the resulted organic layer was dried over anhydrous sodium sulfate, and about 1 L of the solvent was removed under reduced pressure. To the residue was added hexane (294 mL), and the mixture was stirred for 1 hour. Then, thereto was added hexane (294 mL), and the mixture was stirred for 15 minutes. The resulted solid was collected by filtration, and then washed with a mixed solution of hexane/toluene (v/v = 2/1) to give a titled compound (179 g).
¹H-NMR (CDCl₃) δ: 7.32 (5H, br s), 7.09 (2H, s), 6.52 (1H, br s), 5.67 (1H, br s), 5.06 (2H, s), 2.33 (6H, br s).

### Step 1-2: Benzyl 2-(4-cyclopropyl-2,6-dimethylphenyl)hydrazine-1-carboxylate

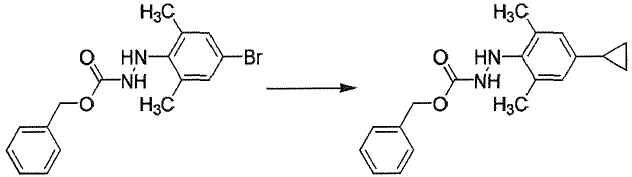

Under an argon atmosphere, tripotassium phosphate (190 g) was dissolved in water (357 mL), and then thereto were added toluene (715 mL), benzyl 2-(4-bromo-2,6-dimethylphenyl)hydrazine-1-carboxylate (89.3 g), cyclopropylboronic acid (61.7 g), and [1,1'-bis(di-phenylphosphino)ferrocene]palladium (II) dichloride dichloromethane adduct (4.18 g), and the mixture was stirred at 105°C for 4 hours. The reaction mixture was let cool to room temperature, followed by separation after addition of 6 M hydrochloric acid (298 mL). The resulted organic layer was dried over anhydrous magnesium sulfate, and then the solvent was removed under reduced pressure. To the residue was added hexane (450 mL), and the mixture was stirred. Then, the resulted solid was collected by filtration to give the titled compound (64.2 g).
¹H-NMR (CDCl₃) δ: 7.31 (5H, br s), 6.68 (2H, s), 6.48 (1H, br s), 5.65 (1H, br s), 5.06 (2H, s), 2.32 (6H, br s), 1.80-1.73 (1H, m), 0.87-0.84 (2H, m), 0.61-0.59 (2H, m).

### Step 1-3: (4-Cyclopropyl-2,6-dimethylphenyl)hydrazine hydrochloride

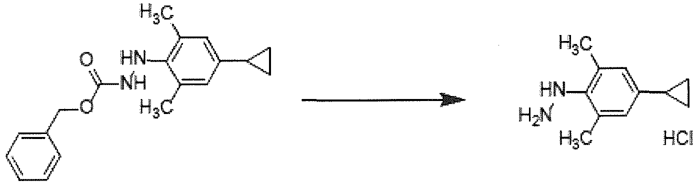

Under a nitrogen atmosphere, to a mixture of benzyl 2-(4-cyclopropyl-2,6-dimethylphenyl)hydrazine-1-carboxylate (133 g) that was synthesized in accordance with Step 1-2 and ethanol (798 mL) was added 4 M aqueous sodium hydroxide solution (536 mL), and the mixture was stirred at 80°C for 3 hours. The reaction mixture was ice-cooled, and thereto was added. Then, the mixture was extracted with toluene (1 L) after addition of acetic acid (73.6 mL). The resulted organic layer was dried over anhydrous magnesium sulfate, and then was concentrated until the volume of solution became about 1/4. Toluene (500 mL) was added to the resulted mixture, and a 4 M solution of hydrogen chloride in dioxane (102 mL) was added slowly to the mixture with stirring under ice cooling. Hexane (50 mL) was added to the mixture, and the mixture was stirred at room temperature for 10 minutes. Then, the resulted solid was collected by filtration and washed with hexane to give the titled compound (83 g).
¹H-NMR (DMSO-d6) δ: 9.50 (3H, br s), 6.79 (2H, s), 6.66 (1H, br s), 2.33 (6H, s), 1.83-1.81 (1H, m), 0.97-0.84 (2H, m), 0.65-0.62 (2H, m).

### Step 1-4: 2,4,6-Trichloro-5-(dimethoxymethyl)pyrimidine

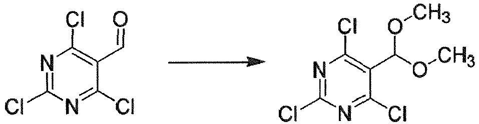

Under a nitrogen atmosphere, to a mixture of 2,4,6-trichloropyrimidine-5-carbaldehyde (250 g) and toluene (1.5 L) were added trimethyl orthoformate (750 mL) and sulfuric acid (1.6 mL), and the mixture was stirred at room temperature for 1.5 hours. To the reaction mixture was added basic silica gel (Fuji Silysia, 500 g), and the mixture was stirred for 2 hours. Then, the added silica gel was removed by filtration. The silica gel was washed with ethyl acetate (1.0 L), and then the solvent was removed under reduced pressure to give the titled compound (268 g).
¹H-NMR (CDCl₃) δ: 5.70 (1H, s), 3.50 (6H, s).

### Step 1-5: 4,6-Dichloro-2-(4-cyclopropyl-2,6-dimethylphenyl)-2H-pyrazolo[3,4-d]pyrimidine

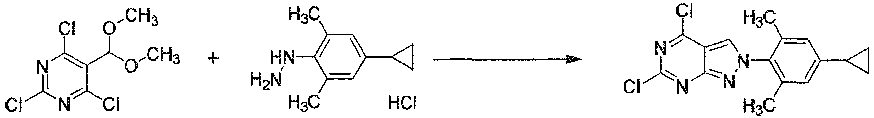

Under a nitrogen atmosphere, to a mixture of 2,4,6-trichloro-5-(dimethoxymethyl)pyrimidine (94 g) and toluene (1160 mL) were added (4-cyclopropyl-2,6-dimethylphenyl)hydrazine hydrochloride (83 g) and triethylamine (102 mL) under ice cooling, and the mixture was stirred at the same temperature for 40 minutes. To the reaction mixture was added dropwise and slowly trifluoroacetic acid (98 mL), and the mixture was stirred at room temperature for 2 hours. Under an ice bath, the reaction mixture was added dropwise and slowly to an aqueous solution (776 mL) of tripotassium phosphate (310 g), and then the mixture was stirred at room temperature for 10 minutes. After separation, the resulted aqueous layer was extracted with toluene. All organic layers were collected and dried over anhydrous magnesium sulfate, and then the solvent was removed under reduced pressure to give a crude product (162 g) of the titled compound.
¹H-NMR (CDCl₃) δ: 8.17 (1H, s), 6.89 (2H, s), 1.98 (6H, s), 1.94-1.87 (1H, m), 1.07-1.01 (2H, m), 0.77-0.72 (2H, m).

### Step 1-6: 6-Chloro-2-(4-cyclopropyl-2,6-dimethylphenyl)-2,5-dihydro-4H-pyrazolo[3,4-d]pyrimidin-4-one

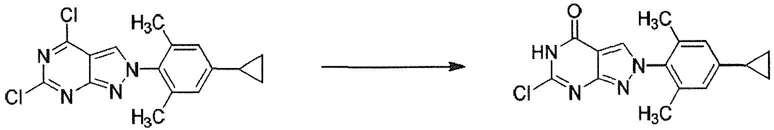

Under a nitrogen atmosphere, to a mixture of a crude product (162 g) of 4,6-dichloro-2-(4-cyclopropyl-2,6-dimethylphenyl)-2H-pyrazolo[3,4-d]pyrimidine and tetrahydrofuran (970 mL) was added 2M aqueous sodium hydroxide solution (913 mL), and the mixture was stirred at 75°C for 3 hours. The reaction mixture was ice-cooled, and then thereto was added 6 M hydrochloric acid (243 mL). To the reaction mixture was added ethyl acetate, and after separation, the resulted aqueous layere was extracted with ethyl acetate. All organic layers were collected and dried over anhydrous magnesium sulfate, and then the solvent was removed under reduced pressure. To the resulted solid was added ethyl acetate, and the mixture was stirred at room temperature for 10 minutes. Then, the resulted solid was collected by filtration and washed with ethyl acetate. The resulted solid was dried under reduced pressure to give the titled compound (74 g).
¹H-NMR (DMSO-D₆) δ: 12.89 (1H, br s), 8.80 (1H, s), 7.00 (2H, s), 2.03-1.95 (7H, m), 1.03-1.01 (2H, m), 0.78-0.76 (2H, m).

### Step 1-7: 2-(4-Cyclopropyl-2,6-dimethylphenyl)-6-(1-methyl-1H-pyrazol-3-yl)-2,5-dihydro-4H-pyrazolo[3,4-d]pyrimidin-4-one

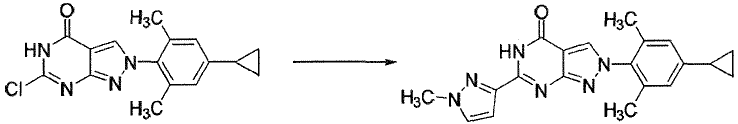

Under an argon atmosphere, tripotassium phosphate (148 g) was dissolved in water (510 mL), and then thereto were added toluene (880 mL), 6-chloro-2-(4-cyclopropyl-2,6-dimethylphenyl)-2,5-dihydro-4H-pyrazolo[3,4-d]pyrimidin-4-one (73 g), 1-methyl-3-(4,4,5,5-tetramethyl-1,3,2-dioxaborolan-2-yl)-1H-pyrazole (57.9 g), and [1,1'-bis(di-phenylphosphino)ferrocene]palladium (II) dichloride dichloromethane adduct (4.73 g). The reaction mixture was stirred at 110°C for 3 hours, and then let cool to 60°C. Thereto was added tetrahydrofuran (290 mL), and the mixture was cooled to 15°C with an ice bath. Then, thereto was added dropwise and slowly 6 M hydrochloric acid (193 mL), and the mixture was stirred for 20 minutes. To the reaction mixture was added tetrahydrofuran (150 mL), and then the mixture was extracted with ethyl acetate. The resulted organic layer was dried over anhydrous magnesium sulfate, and then the solvent was removed under reduced pressure. To the residue were added toluene (500 mL) and ethyl acetate (500 mL), and the mixture was stirred at room temperature for 30 minutes. Then, the resulted solid was collected by filtration and washed with toluene. The resulted solid was dissolved in tetrahydrofuran (800 mL), and then thereto was added ISOLUTE Si-TMT (metal scavenging-silica gel, manufactured by Biotage, 0.47 mmol TMT/g, 49 g). The mixture was stirred at room temperature for 2 hours. The added ISOLUTE Si-TMT was removed by filtration and washed with ethyl acetate (700 mL). To the resulted solution were added silica gel (70 g) and basic silica gel (Fuji Silysia, 70 g), and the mixture was stirred at room temperature for 3 hours. The added silica gel was removed by filtration and washed sequentially with a mixed solution (v/v = 1/1, 300 mL) of ethyl acetate/tetrahydrofuran and ethyl acetate (700 mL), and then the solvent was removed under reduced pressure.

To the resulted solid was added acetone (180 mL), and then the mixture was stirred under ice cooling for 30 minutes. The resulted solid was collected by filtration, washed with cooled acetone (180 mL), and then dried under reduced pressure to give a crystal of the titled compound (Form α, 49.7 g).
¹H-NMR (DMSO-D₆) δ: 11.21 (1H, s), 8.70 (1H, s), 7.90 (1H, d, *J* = 2.3 Hz), 6.97 (2H, s), 6.96 (1H, d, *J* = 2.3 Hz), 3.98 (3H, s), 1.96-1.94 (7H, m), 1.01-0.97 (2H, m), 0.75-0.73 (2H, m) .
LC-MS(MH+): 361.

### Step 1-8: 2-(4-Cyclopropyl-2,6-dimethylphenyl)-6-(1-methyl-1H-pyrazol-3-yl)-2,5-dihydro-4H-pyrazolo[3,4-d]pyrimidin-4-one monohydrate

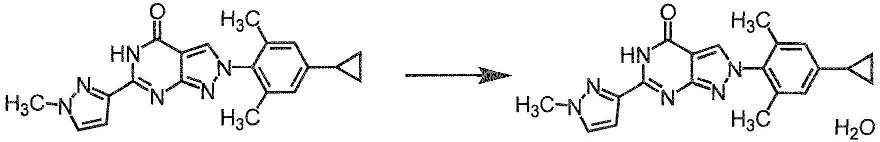

A crystal of 2-(4-cyclopropyl-2,6-dimethylphenyl)-6-(1-methyl-1H-pyrazol-3-yl)-2,5-dihydro-4H-pyrazolo[3,4-d]pyrimidin-4-one (Form α, 16.0 g) was dissolved in methanol (100 mL) at 80°C under heating. At the same temperature, water (100 mL) was added thereto, and then the mixture was stirred for 1 hour. Then, water (200 mL) was further added thereto. The mixture was let cool to room temperature and stirred for additional 2 hours. The resulted solid was collected by filtration, and then dried under reduced pressure to give a crystal of the titled compound (Form β, 16.2 g).
¹H-NMR (DMSO-d6) δ: 11.19 (1H, s), 8.69 (1H, s), 7.90 (1H, d, *J* = 2.3 Hz), 6.97 (2H, s), 6.95 (1H, d, *J* = 2.3 Hz), 3.97 (3H, s), 1.98-1.91 (7H, m), 1.00-0.98 (2H, m), 0.76-0.72 (2H, m).
LC-MS(MH+): 361

### [Preparation example 2]: Synthesis of 2-{4-(1,1-difluoroethyl)-2,6-dimethylphenyl}-6-(1-methyl-1H-pyrazol-3-yl)-2,5-dihydro-4H-pyrazolo[3,4-d]pyrimidin-4-one

### (Example 8)

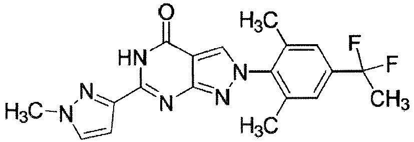

### Step 2-1: 1-(4-Bromo-3,5-dimethylphenyl)ethan-1-one

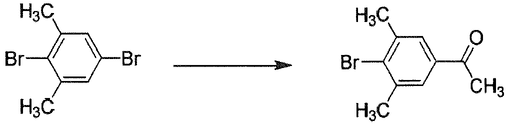

Under an argon atmosphere, to a mixture of 2,5-dibromo-1,3-dimethylbenzene (11.6 g) and tetrahydrofuran (170 mL) was added dropwise and slowly a solution of n-butyllithium in n-hexane (1.59 M, 25.2 mL) at -78°C, and then the mixture was stirred at the same temperature for 20 minutes. To the reaction mixture was added N-methoxy-N-methylacetamide (5.61 mL), and then the mixure was stirred at the same temperature for 1 hour. To the reaction mixture was added aqueous saturated ammonium chloride solution, and then the mixture was extracted with ethyl acetate. The resulted organic layer was washed with brine and dried over anhydrous magnesium sulfate, and then the solvent was removed under reduced pressure. The residue was purified by column chromatography (Developing solvent: hexane/ethyl acetate) to give the titled compound (5.79 g).
¹H-NMR (CDCl₃) δ: 7.64 (2H, s), 2.57 (3H, s), 2.47 (6H, s).

### Step 2-2: 2-Bromo-5-(1,1-difluoroethyl)-1,3-dimethylbenzene

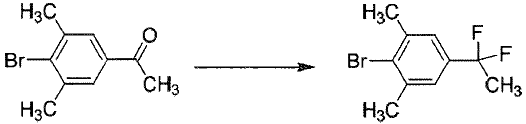

Under an argon atmosphere, to 1-(4-bromo-3,5-dimethylphenyl)ethan-1-one (5.0 g) were added a 50% solution of bis(2-methoxyethyl)aminosulfur trifluoride in tetrahydrofuran (48.7 mL) and methanol (0.089 mL) at 0°C, and the mixture was stirred at 80°C for 7 hours. The reaction mixture was added dropwise to aqueous saturated sodium hydrogen carbonate solution and then extracted with ethyl acetate. The resulted organic layer was washed with brine and dried over anhydrous magnesium sulfate, and then the solvent was removed under reduced pressure. The residue was purified by column chromatography (Developing solvent: hexane/ethyl acetate) to give the titled compound (3.58 g). ¹H-NMR (CDCl₃) δ: 7.20 (2H, s), 2.44 (6H, s), 1.88 (3H, t, J = 18.1 Hz).

### Step 2-3: Di-tert-butyl 1-{4-(1,1-difluoroethyl)-2,6-dimethylphenyl}hydrazine-1,2-dicarboxylate

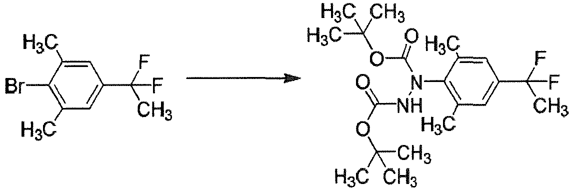

Under an argon atmosphere, to a mixture of 2-bromo-5-(1,1-difluoroethyl)-1,3-dimethylbenzene (3.58 g) and tetrahydrofuran (72 mL) was added a solution of n-butyllithium in n-hexane (1.56 M, 11 mL) at -78°C, and the mixture was stirred at the same temperature for 30 minutes. To the reaction mixture was added di-tert-butyl azodicarboxylate (4.96 g), and then the mixture was stirred for additional 30 minutes. To the reaction mixture was added aqueous saturated ammonium chloride solution, and then the mixture was extracted with ethyl acetate. The resulted organic layer was washed with brine and dried over anhydrous magnesium sulfate, and then the solvent was removed under reduced pressure. The residue was purified by column chromatography (Developing solvent: hexane/ethyl acetate) to give the titled compound (3.38 g).
¹H-NMR (CDCl₃) δ: 7.19-7.18 (2H, m), 6.60 (1H, br s), 2.38 (6H, s), 1.94-1.82 (3H, m), 1.55-1.37 (18H, m).

### Step 2-4: {4-(1,1-Difluoroethyl)-2, 6-dimethylphenyl}hydrazine hydrochloride

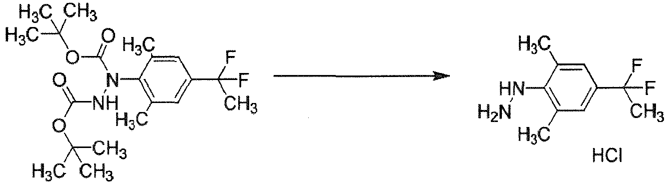

To di-tert-butyl 1-{4-(1,1-difluoroethyl)-2,6-dimethylphenyl}hydrazine-1,2-dicarboxylate (3.38 g) was added a 4 M solution of hydrogen chloride in ethyl acetate (34 mL), and the mixture was stirred at room temperature for 1.5 hours. The solvent was removed under reduced pressure to give the titled compound (2.0 g).
¹H-NMR (DMSO-D₆) δ: 9.61 (3H, br s), 7.29 (2H, s), 6.85 (1H, br s), 2.42 (6H, s), 1.93 (3H, t, *J* = 18.8 Hz).

### Step 2-5: 2,4-Dichloro-6-[2-{4-(1,1-difluoroethyl)-2,6-dimethylphenyl}hydrazinyl]-5-(dimethoxymethyl)pyrimidine

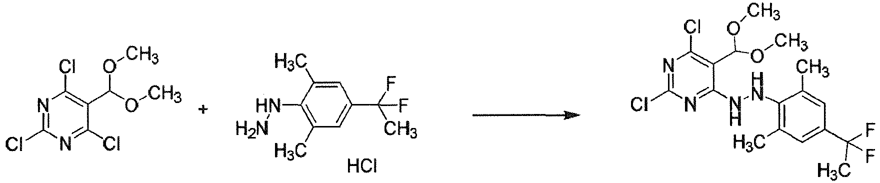

Under an argon atmosphere, to a mixture of {4- (1, 1-difluoroethyl)-2,6-dimethylphenyl}hydrazine hydrochloride (2.0 g), triethylamine (3.53 mL), and methanol (40 mL) was added at 0°C 2,4,6-trichloro-5-(dimethoxymethyl)pyrimidine (2.18 g) which was synthesized in accordance with Preparation example 1 Step 1-4. The mixture was stirred at room temperature for 1 hour, and then the solvent was removed under reduced pressure. Ethyl acetate was added thereto. The resulted solid was removed by filtration, and then the solvent was removed under reduced pressure to give a crude product of the titled compound (3.56 g).
LC-MS (MH+): 421.

### Step 2-6: 4,6-Dichloro-2-{4-(1,1-difluoroethyl)-2,6-dimethylphenyl}-2H-pyrazolo[3,4-d]pyrimidine

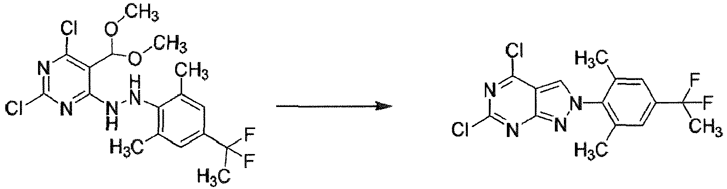

Under an argon atmosphere, to a mixture of a crude product of 2,4-dichloro-6-[2-{4-(1,1-difluoroethyl)-2,6-dimethylphenyl}hydrazinyl]-5-(dimethoxymethyl)pyrimidine (3.56 g) and toluene (36 mL) was added dropwise and slowly trifluoroacetic acid (1.62 mL), and then the mixture was stirred at room temperature for 1 hour. The reaction mixture was added dropwise and slowly to 2 M aqueous tripotassium phosphate solution and then extracted with ethyl acetate. The resulted organic layer was washed with brine and dried over anhydrous magnesium sulfate, and then the solvent was removed under reduced pressure. The residue was purified by column chromatography (Developing solvent: hexane/ethyl acetate) to give the titled compound (1.89 g).
LC-MS (MH+): 357.

### Step 2-7: 6-Chloro-2-{4-(1,1-difluoroethyl)-2,6-dimethylphenyl}-2,5-dihydro-4H-pyrazolo[3,4-d]pyrimidin-4-one

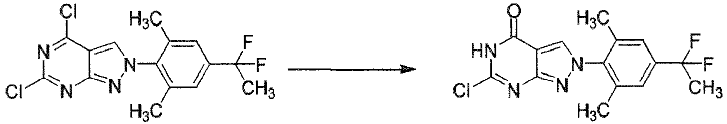

To a mixture of 4,6-dichloro-2-{4-(1,1-difluoroethyl)-2,6-dimethylphenyl}-2H-pyrazolo[3,4-d]pyrimidine (1.89 g) and tetrahydrofuran (19 mL) was added 2M aqueous sodium hydroxide solution (5.29 mL), and the mixture was stirred at room temperature for 2 hours. The reaction mixture was neutralized with 2 M hydrochloric acid and then extracted with ethyl acetate. The resulted organic layer was washed with brine and dried over anhydrous magnesium sulfate, and then the solvent was removed under reduced pressure. The residue was purified by column chromatography (Developing solvent: hexane/ethyl acetate), and then the resulted solid was stirred in a mixed solution of ethyl acetate/diisopropyl ether. The resulted solid was collected by filtration, and then dried under reduced pressure to give the titled compound (1.03 g).
¹H-NMR (DMSO-D₆) δ: 12.90 (1H, br s), 8.85 (1H, s), 7.49 (2H, s), 2.03-1.98 (9H, m).

### Step 2-8: 2-{4-(1,1-Difluoroethyl)-2,6-dimethylphenyl}-6-(1-methyl-1H-pyrazol-3-yl)-2,5-dihydro-4H-pyrazolo[3,4-d]pyrimidin-4-one

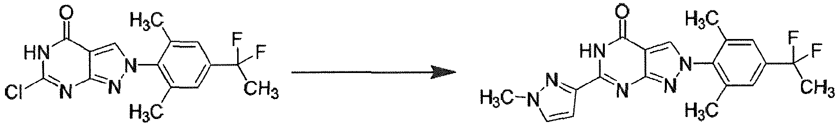

Under an argon atmosphere, to a mixture of 6-chloro-2-{4-(1,1-difluoroethyl)-2,6-dimethylphenyl}-2,5-dihydro-4H-pyrazolo[3,4-d]pyrimidin-4-one (1.03 g) and toluene/water (v/v = 5/1, 12.9 mL) were added 1-methyl-3-(4,4,5,5-tetramethyl-1,3,2-dioxaborolan-2-yl)-1H-pyrazole (0.949 g), [1,1'-bis(di-phenylphosphino)ferrocene]palladium (II) dichloride dichloromethane adduct (0.198 g), and tripotassium phosphate (1.94 g), and the mixture was stirred at 105°C for 2 hours. The reaction mixture was let cool to room temperature, and 2M hydrochloric acid was added dropwise and slowly thereto. Then, the reaction mixture was extracted with ethyl acetate. The resulted organic layer was washed with brine and dried over anhydrous magnesium sulfate, and then the solvent was removed under reduced pressure.

The residue was dissolved in a mixed solution of tetrahydrofuran/ethyl acetate, and then thereto was added ISOLUTE Si-TMT (metal scavenging-silica gel, manufactured by Biotage, 0.47 mmol TMT/g, 3 g). Then, the mixture was stirred at room temperature for 1 hour. The added ISOLUTE Si-TMT was removed by filtration and washed with ethyl acetate, and then the solvent was removed under reduced pressure. The residue was purified by column chromatography (Developing solvent: hexane/ethyl acetate). The resulted solid was stirred in a mixed solution of ethyl acetate/diisopropyl ether, and then the resulted solid was collected by filtration to give the titled compound (1.02 g).
¹H-NMR (DMSO-D₆) δ: 11.28 (1H, s), 8.80 (1H, s), 7.90 (1H, d, J = 2.2 Hz), 7.50 (2H, s), 6.96 (1H, d, *J* = 2.2 Hz), 3.98 (3H, s), 2.05-1.98 (9H, m).
LC-MS (MH+) : 385.

### [Preparation example 3]: Synthesis of 2-(4-cyclopropyl-2,6-dimethylphenyl)-6-(1-methyl-1H-imidazol-4-yl)-2,5-dihydro-4H-pyrazolo[3,4-d]pyrimidin-4-one (Example 39)

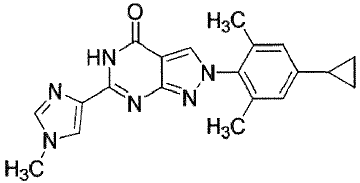

### Step 3-1: Benzyl 2-(4-bromo-2,6-dimethylphenyl)hydrazine-1-carboxylate

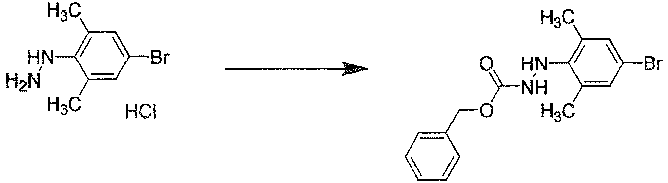

Under a nitrogen atmosphere, to a mixture of (4-bromo-2,6-dimethylphenyl)hydrazine hydrochloride (150 g) and tetrahydrofuran (1.0 L) were added N,N-diisopropylethylamine (220 mL) and benzyl chloroformate (82 mL) at 0°C, and the mixture was stirred at room temperature for 1 hour. To the reaction mixture were added toluene (500 mL) and water (500 mL), and the mixture was stirred at room temperature for 20 minutes. After separation, the resulted organic layer was dried over anhydrous sodium sulfate and anhydrous magnesium sulfate, and the solvent was removed under reduced pressure. To the residue were added toluene (200 mL) and hexane (600 mL), and the mixture was stirred. Then, the resulted solid was collected by filtration to give the titled compound (180 g).
¹H-NMR (CDCl₃) δ: 7.32 (5H, br s), 7.09 (2H, s), 6.52 (1H, br s), 5.67 (1H, br s), 5.06 (2H, s), 2.33 (6H, br s).

### Step 3-2: Benzyl 2-(4-cyclopropyl-2,6-dimethylphenyl)hydrazine-1-carboxylate

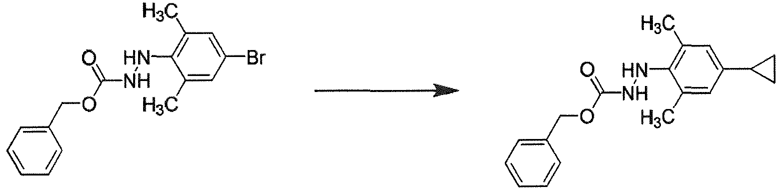

Under a nitrogen atmosphere, tripotassium phosphate (380 g) was dissolved in water (710 mL), and then thereto were added toluene (1400 mL), benzyl 2-(4-bromo-2,6-dimethylphenyl)hydrazine-1-carboxylate (180 g), cyclopropylboronic acid (110 g), and [1,1'-bis(diphenylphosphino)ferrocene]palladium (II) dichloride dichloromethane adduct (8.3 g). The mixture was stirred at 110°C for 5 hours. The reaction mixture was let cool to room temperature, and then thereto was added 6 M hydrochloric acid (600 mL). Then, the mixture was stirred for 10 minutes. The aqueous layer was removed by separation, and the resulted organic layer was dried over anhydrous magnesium sulfate. Then, the solvent was removed under reduced pressure. To the residue was added a mixed solution of toluene/hexane (v/v = 1/1, 360 mL), and the mixture was stirred for 2 hours. Then, thereto was added hexane (360 mL), and the mixture was stirred for additional 1 hour. The resulted solid was collected by filtration and washed sequentially with a mixed solution of toluene/hexane (v/v = 1/10, 550 mL) and hexane (300 mL) to give the titled compound (100 g).
¹H-NMR (CDCl₃) δ: 7.31 (5H, br s), 6.68 (2H, s), 6.48 (1H, br s), 5.65 (1H, br s), 5.06 (2H, s), 2.32 (6H, br s), 1.80-1.73 (1H, m), 0.87-0.84 (2H, m), 0.61-0.59 (2H, m).

### Step 3-3: (4-Cyclopropyl-2,6-dimethylphenyl)hydrazine hydrochloride

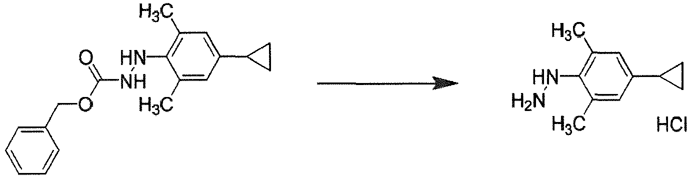

Under a nitrogen atmosphere, to a mixture of benzyl 2-(4-cyclopropyl-2,6-dimethylphenyl)hydrazine-1-carboxylate (100 g) and ethanol (600 mL) was added 4 M aqueous sodium hydroxide solution (400 mL), and the mixture was stirred at 80°C for 3 hours. The reaction mixture was let cool, and then thereto was added acetic acid (55 mL). Then, the mixture was extracted with toluene. The resulted organic layer was dried over anhydrous magnesium sulfate and concentrated until the volume of solution became about 1/4. To the resulted mixture was added toluene (400 mL), and a 4 M solution of hydrogen chloride in 1,4-dioxane (77 mL) was added slowly to the mixture with stirring under ice cooling. The mixture was stirred at room temperature for 30 minutes, and then thereto was added hexane (40 mL). The mixture was stirred for additional 5 minutes. The resulted solid was collected by filtration and then washed sequentially with a mixed solution of toluene/hexane (v/v = 1/1) and hexane to give the titled compound (54 g).
¹H-NMR (DMSO-d₆) δ: 9.50 (3H, br s), 6.79 (2H, s), 6.66 (1H, br s), 2.33 (6H, s), 1.83-1.81 (1H, m), 0.97-0.84 (2H, m), 0.65-0.62 (2H, m).

### Step 3-4: 4,6-Dichloro-2-(4-cyclopropyl-2,6-dimethylphenyl)-2H-pyrazolo[3,4-d]pyrimidine

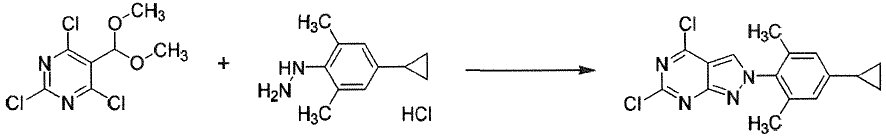

Under a nitrogen atmosphere, to a mixture of 2,4,6-trichloro-5-(dimethoxymethyl)pyrimidine (63 g) which was synthesized in accordance with Preparation example 1 Step 1 -4 and toluene (780 mL) were added (4-cyclopropyl-2,6-dimethylphenyl)hydrazine hydrochloride (54 g) and triethylamine (68 mL) under ice cooling, and the mixture was stirred at the same temperature for 40 minutes. To the reaction mixture was added dropwise and slowly trifluoroacetic acid (66 mL), and then the mixture was stirred at room temperature for 2 hours. The reaction mixture was added dropwise and slowly to a solution of tripotassium phosphate (210 g) in water (470 mL) under ice cooling, and then the mixture was stirred at room temperature for 10 minutes. The organic layer was separated, and then the aqueous layer was extracted with toluene. All organic layers were collected and dried over anhydrous magnesium sulfate, and then the solvent was removed under reduced pressure to give a crude product of the titled compound (102 g). A part of the resulted crude product was purified by column chromatography (Developing solvent: hexane/ethyl acetate) to give the titled compound (1.6 g).
LC-MS (MH+): 333.

### Step 3-5: 4-(Benzyloxy)-6-chloro-2-(4-cyclopropyl-2,6-dimethylphenyl)-2H-pyrazolo[3,4-d]pyrimidine

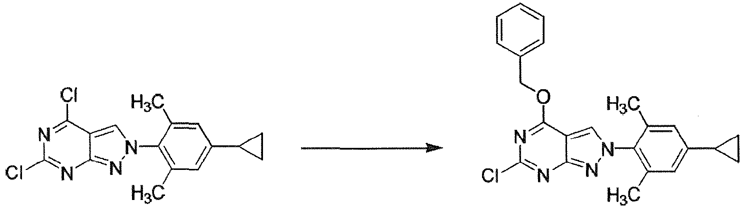

Under an argon atmosphere, to a mixture of sodium hydride (60% in oil, 200 mg) and tetrahydrofuran (24 mL) was added benzyl alcohol (630 mg), and the mixture was stirred at 50°C for 30 minutes. The reaction mixture was cooled under ice cooling, and then thereto was added 4,6-dichloro-2-(4-cyclopropyl-2,6-dimethylphenyl)-2H-pyrazolo[3,4-d]pyrimidine (1.6 g). The mixture was stirred at the same temperature for 30 minutes. To the reaction mixture was added 2 M hydrochloric acid (0.73 mL), and then the mixture was extracted with ethyl acetate. The resulted organic layer was dried over anhydrous magnesium sulfate, and then the solvent was removed under reduced pressure. The residue was added to a mixed solution of hexane/ethyl acetate (v/v = 4/1), and the mixture was stirred. Then, the resulted solid was collected by filtration to give the titled compound (1.8 g).
¹H-NMR (CDCl₃) δ: 8.00 (1H, s), 7.55-7.52 (2H, m), 7.43-7.41 (3H, m), 6.85 (2H, s), 5.64 (2H, s), 1.95 (6H, s), 1.91-1.86 (1H, m), 1.02-0.99 (2H, m), 0.73-0.71 (2H, m).
LC-MS (MH+): 405.

### Step 3-6: A mixture of 4-(benzyloxy)-2-(4-cyclopropyl-2,6-dimethylphenyl)-6-(1-methyl-1H-imidazol-4-yl)-2H-pyrazolo[3,4-d]pyrimidine and 2-(4-cyclopropyl-2,6-dimethylphenyl)-6-(1-methyl-1H-imidazol-4-yl)-2,5-dihydro-4H-pyrazolo[3,4-d]pyrimidin-4-one

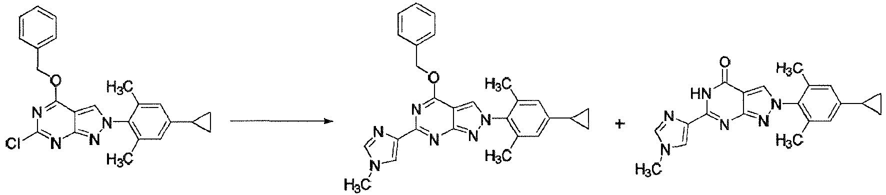

Under an argon atmosphere, to a mixture of 4-(benzyloxy)-6-chloro-2-(4-cyclopropyl-2,6-dimethylphenyl)-2H-pyrazolo[3,4-d]pyrimidine (70 mg) and N,N-dimethylacetamide (1.0 mL) were added bis(di-tert-butyl(4-dimethylaminophenyl)phosphine)dichloropalladium (II) (12 mg), lithium chloride (7.3 mg), and 1-methyl-4-(tributylstannyl)-1H-imidazole (0.11 mL), and the mixture was stirred at 120°C for 3 hours. The reaction mixture was purified by column chromatography (Developing solvent: methanol/ethyl acetate) to give a mixture (110 mg) of 4-(benzyloxy)-2-(4-cyclopropyl-2,6-dimethylphenyl)-6-(1-methyl-1H-imidazol-4-yl)-2H-pyrazolo[3,4-d]pyrimidine and 2-(4-cyclopropyl-2,6-dimethylphenyl)-6-(1-methyl-1H-imidazol-4-yl)-2,5-dihydro-4H-pyrazolo[3,4-d]pyrimidin-4-one.
4-(Benzyloxy)-2-(4-cyclopropyl-2,6-dimethylphenyl)-6-(1-methyl-1H-imidazol-4-yl)-2H-pyrazolo[3,4-d]pyrimidine LC-MS (MH+): 451.
2-(4-Cyclopropyl-2,6-dimethylphenyl)-6-(1-methyl-1H-imidazol-4-yl)-2,5-dihydro-4H-pyrazolo[3,4-d]pyrimidin-4-one
LC-MS (MH+): 361.

### Step 3-7: 2-(4-Cyclopropyl-2,6-dimethylphenyl)-6-(1-methyl-1H-imidazol-4-yl)-2,5-dihydro-4H-pyrazolo[3,4-d]pyrimidin-4-one

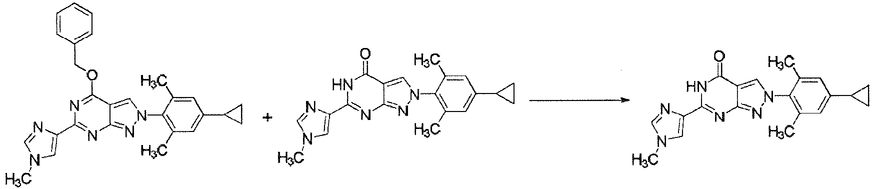

Under an argon atmosphere, to a mixture (110 mg) of 4-(benzyloxy)-2-(4-cyclopropyl-2,6-dimethylphenyl)-6-(1-methyl-1H-imidazol-4-yl)-2H-pyrazolo[3,4-d]pyrimidine and 2-(4-cyclopropyl-2,6-dimethylphenyl)-6-(1-methyl-1H-imidazol-4-yl)-2,5-dihydro-4H-pyrazolo[3,4-d]pyrimidin-4-one was added formic acid (1.0 mL), and the mixture was stirred at 90°C for 1 hour. Then, the solvent was removed under reduced pressure. The residue was purified by reversed column chromatography (Developing solvent: acetonitrile/water), and then treated with hexane/ethyl acetate to give a solid, the titled compound (23 mg).
¹H-NMR (DMSO-D₆) δ: 10.77 (1H, br s), 8.70 (1H, s), 8.06 (1H, d, *J* = 1.3 Hz), 7.87 (1H, d, *J* = 1.3 Hz), 7.00 (2H, s), 3.80 (3H, s), 2.02-1.94 (7H, m), 1.04-1.01 (2H, m), 0.79-0.76 (2H, m) .
LC-MS (MH+): 361.

### [Preparation example 4]: Synthesis of 2-{4-(1,1-difluoroethyl)-2,6-dimethylphenyl}-6-(1-methyl-1H-imidazol-4-yl)-2,5-dihydro-4H-pyrazolo[3,4-d]pyrimidin-4-one

### (Example 41)

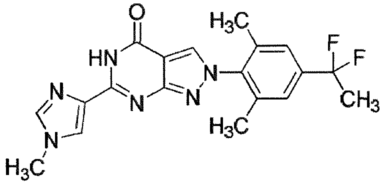

### Step 4-1: tert-Butyl(4-iodo-2,6-dimethylphenyl)carbamate

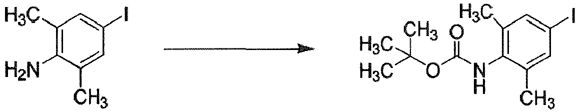

To a mixture of 4-iodo-2,6-dimethylaniline (24 g) and ethanol (73 mL) was added di-tert-butyl dicarbonate (40 mL), and the mixture was stirred at room temperature overnight. To the reaction mixture was added water, and then the resulted solid was collected by filtration and washed with water and hexane. The resulted solid was dissolved in ethyl acetate (150 mL), and then thereto was added silica gel (24 g). The mixture was stirred for 15 minutes, and then the added silica gel was removed by filtration. Silica gel was washed with a mixed solution of hexane/ethyl acetate (v/v = 1/1), and then the solvent was removed under reduced pressure. The resulted solid was stirred and washed with hexane, and then collected by filtration to give the titled compound (29 g).
¹H-NMR (DMSO-D₆) δ: 8.43 (1H, br s), 7.43 (2H, s), 2.11 (6H, s), 1.44 (9H, s).

### Step 4-2: Ethyl 2-[4-{(tert-butoxycarbonyl)amino)}-3,5-dimethylphenyl]-2,2-difluoroacetate

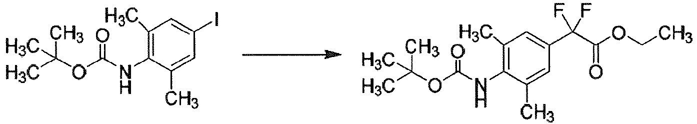

Under a nitrogen atmosphere, to a mixture of tert-butyl(4-iodo-2,6-dimethylphenyl)carbamate (29 g) and dimethylsulfoxide (230 mL) were added 2-bromo-2,2-difluoroethyl acetate (21 mL) and copper (16 g), and the mixture was stirred at 70°C for 4 hours. A solid was removed by filtration, and then aqueous saturated ammonium chloride solution was added to the resultant. Then, the mixture was extracted with ethyl acetate. The resulted organic layer was washed sequentially with aqueous saturated ammonium chloride solution, water, and brine, and then thereto were added anhydrous sodium sulfate and silica gel. The mixture was stirred. The resulted solid was removed by filtration and washed with ethyl acetate, and then the solvent was removed under reduced pressure to give the titled compound (18 g).
¹H-NMR (DMSO-D₆) δ: 8.63 (1H, br s), 7.27 (2H, s), 4.31 (2H, q, *J* = 7.0 Hz), 2.21 (6H, s), 1.45 (9H, s), 1.23 (3H, t, *J* = 7.0 Hz).

### Step 4-3: tert-Butyl {4-(1,1-difluoro-2-hydroxyethyl)-2,6-dimethylphenyl}carbamate

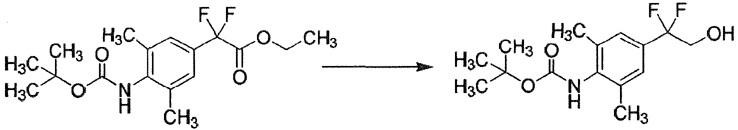

Under a nitrogen atmosphere, to a mixture of ethyl 2-[4-{(tert-butoxycarbonyl)amino)}-3,5-dimethylphenyl]-2,2-difluoroacetate (30 g) which was synthesized in accordance with Step 4-2 and ethanol (240 mL) was added slowly sodium borohydride (2.7 g) over 16 minutes. The reaction mixture was stirred at room temperature for 30 minutes, and then cooled with an ice bath. 1M hydrochloric acid was added slowly thereto at the same temperature, and then the mixture was diluted with ethyl acetate, hexane, and water. The resulted mixture was separated, and then the aqueous layer was extracted with a mixed solution of ethyl acetate/hexane (v/v = 1/1). All organic layers were collected, and then were washed sequentially with water, aqueous saturated sodium hydrogen carbonate solution, and brine and dried over anhydrous sodium sulfate. Then, the solvent was removed under reduced pressure. The residue was stirred in a mixed solution of hexane/ethyl acetate (180 mL/9 mL). The resulted solid was collected by filtration, and then washed with hexane to give the titled compound (25 g).
¹H-NMR (DMSO-D₆) δ: 8.53 (1H, br s), 7.19 (2H, s), 5.58 (1H, t, *J* = 6.2 Hz), 3.81 (2H, td, *J* = 14.1, 6.2 Hz), 2.19 (6H, s), 1.45 (9H, s).

### Step 4-4: 2-[4-{(tert-Butoxycarbonyl)amino}-3,5-dimethylphenyl]-2,2-difluoroethyl trifluoromethanesulfonate

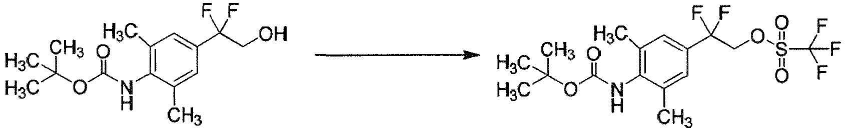

Under a nitrogen atmosphere, to a mixture of tert-butyl {4-(1,1-difluoro-2-hydroxyethyl)-2,6-dimethylphenyl}carbamate (16 g), pyridine (5.6 mL), and toluene (130 mL) was added dropwise and slowly trifluoromethanesulfonic acid anhydride (9.4 mL) under ice cooling, and then the mixture was stirred at the same temperature for 1 hour. Water was added to the reaction mixture, and then the mixture was extracted with ethyl acetate. The resulted organic layer was washed sequentially with 1 M hydrochloric acid, water, and aqueous saturated sodium hydrogen carbonate solution, and then thereto were added anhydrous sodium sulfate and silica gel. Then, the mixture was stirred at room temperature for 15 minutes. The resulted solid was removed by filtration, and then the resultant was washed with a mixed solution of ethyl acetate/toluene (v/v = 1/2). Then, the solvent was removed under reduced pressure. To the residue was added a mixed solution of hexane/ethyl acetate (v/v = 30/1, 50 mL), and then the mixture was stirred at room temperature for 1.5 hours. Then, the mixture was stirred under ice cooling for 15 minutes. The resulted solid was collected by filtration, and then washed with hexane to give the titled compound (21 g).
¹H-NMR (DMSO-D₆) δ: 8.63 (1H, br s), 7.33 (2H, s), 5.33 (2H, t, J = 13.9 Hz), 2.22 (6H, s), 1.45 (9H, s).

### Step 4-5: tert-Butyl{4-(1,1-difluoroethyl)-2,6-dimethylphenyl}carbamate

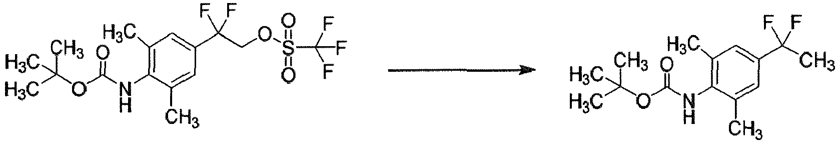

Under a nitrogen atmosphere, to a mixture of 2-[4-{ (tert-butoxycarbonyl)amino}-3,5-dimethylphenyl]-2,2-difluoroethyl trifluoromethanesulfonate (21 g) and dimethylsulfoxide (150 mL) was added slowly sodium borohydride (2.7 g). The reaction mixture was stirred at room temperature overnight, and then thereto were added 1 M hydrochloric acid (60 mL) and water (140 mL) under a water bath. The resulted mixture was extracted with ethyl acetate/hexane (v/v = 6/5), and then washed sequentially with water and aqueous saturated sodium hydrogen carbonate solution and dried over anhydrous sodium sulfate. Then, the solvent was removed under reduced pressure to give a crude product of the titled compound (14 g).

### Step 4-6: 4-(1,1-Difluoroethyl)-2,6-dimethylaniline hydrochloride

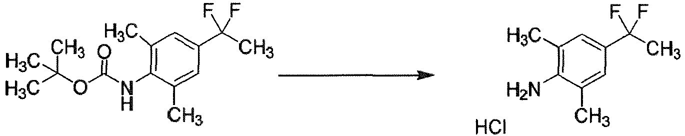

To a mixture of a crude product of tert-butyl{4-(1,1-difluoroethyl)-2,6-dimethylphenyl}carbamate (14 g) and ethyl acetate (68 mL) was added a 4 M solution of hydrogen chloride in ethyl acetate (140 mL), and the mixture was stirred at room temperature for 2 hours. The resulted solid was collected by filtration, and then washed with ethyl acetate to give the titled compound (11 g).
¹H-NMR (DMSO-D₆) δ: 7.15 (2H, s), 7.10 (3H, br s), 2.24 (6H, s), 1.90 (3H, t, *J* = 19.1 Hz).

### Step 4-7: {4-(1,1-Difluoroethyl)-2,6-dimethylphenyl}hydrazine hydrochloride

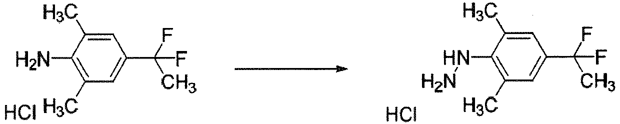

To 4-(1,1-difluoroethyl)-2,6-dimethylaniline hydrochloride (2.0 g) were added concentrated hydrochloric acid (9.0 mL) and 6 M hydrochloric acid (15 mL), and then the mixture was cooled to -20°C. To the reaction mixture was added dropwise and slowly a solution of sodium nitrite (690 mg) in water (3.0 mL), and then the mixture was stirred at 0°C for 40 minutes. Thereto was added dropwise and slowly a solution of tin (II) chloride dihydrate (6.1 g) in concentrated sulfuric acid (9.0 mL) at -20°C over 5 minutes, and then the mixture was stirred at 0°C for 5 hours. The resulted solid was collected by filtration, and then washed sequentially with ice-cooled 2 M hydrochloric acid and a mixed solution of hexane/ethyl acetate (v/v = 4/1) to give the titled compound (1.8 g).
¹H-NMR (DMSO-D₆) δ: 9.68 (3H, br s), 7.29 (2H, s), 6.84 (1H, s), 2.42 (6H, s), 1.93 (3H, t, *J* = 18.9 Hz).

### Step 4-8: 2,4-Dichloro-6-[2-{4-(1,1-difluoroethyl)-2,6-dimethylphenyl}hydrazinyl]-5-(dimethoxymethyl)pyrimidine

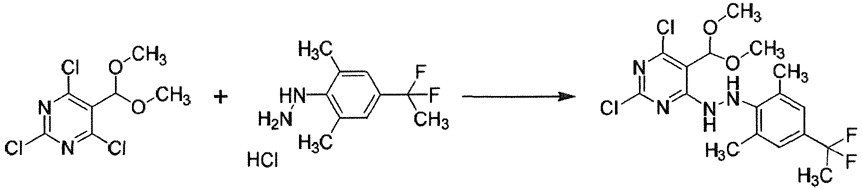

Under an argon atmosphere, to a mixture of {4-(1,1-difluoroethyl)-2,6-dimethylphenyl}hydrazine hydrochloride (500 mg), triethylamine (0.88 mL), and methanol (10 mL) was added 2,4,6-trichloro-5-(dimethoxymethyl)pyrimidine (540 mg) which was synthesized in accordance with Preparation example 1 Step 1-4 at 0°C, and the mixture was stirred at room temperature for 1.5 hours. The solvent was removed under reduced pressure, and then thereto was added ethyl acetate. The resulted solid was removed by filtration, and the solvent was removed under reduced pressure to give a crude product of the titled compound (890 mg).
LC-MS (MH+): 421.

### Step 4-9: 4,6-Dichloro-2-{4-(1,1-difluoroethyl)-2,6-dimethylphenyl}-2H-pyrazolo[3,4-d]pyrimidine

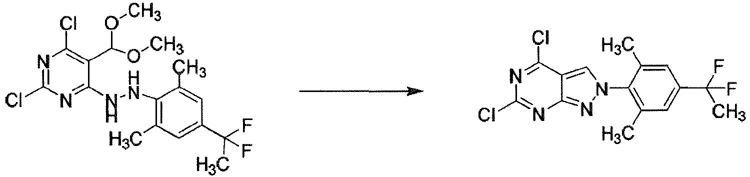

Under an argon atmosphere, to a mixture of a crude product of 2,4-dichloro-6-[2-{4-(1,1-difluoroethyl)-2,6-dimethylphenyl}hydrazinyl]-5-(dimethoxymethyl)pyrimidine (890 mg) and toluene (8.9 mL) was added trifluoroacetic acid (0.40 mL), and the mixture was stirred at room temperature for 1 hour. Then, the mixture was added dropwise to a 2M aqueous solution of tripotassium phosphate. The resulted mixture was extracted with ethyl acetate, and the organic layer was washed with brine and dried over anhydrous magnesium sulfate. Then, the solvent was removed under reduced pressure. The residue was purified by column chromatography (Developing solvent: hexane/ethyl acetate) to give the titled compound (520 mg).
¹H-NMR (DMSO-D₆) δ: 9.36 (1H, s), 7.55 (2H, s), 2.05-2.00 (9H, m).
LC-MS (MH+): 357.

### Step 4-10: 4-(Benzyloxy)-6-chloro-2-{4-(1,1-difluoroethyl)-2,6-dimethylphenyl}-2H-pyrazolo[3,4-d]pyrimidine

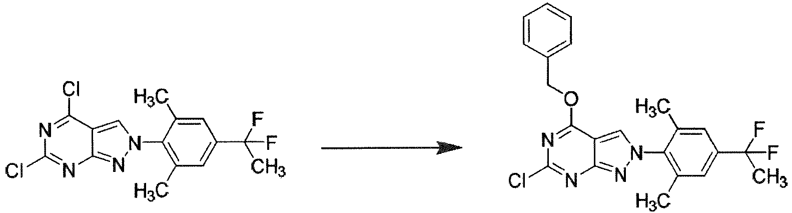

Under an argon atmosphere, to a mixture of benzyl alcohol (0.18 mL) and tetrahydrofuran (10 mL) was added sodium hydride (60% in oil, 61 mg), and the mixture was stirred at 50°C for 30 minutes. The reaction mixture was cooled to 0°C, and then thereto was added 4,6-dichloro-2-{4-(1,1-difluoroethyl)-2,6-dimethylphenyl}-2H-pyrazolo[3,4-d]pyrimidine (520 mg). Then, the mixture was stirred at the same temperature for 1 hour. The reaction mixture was diluted with ethyl acetate, and then thereto was added 2 M hydrochloric acid (1.0 mL). Then, the mixture was extracted with ethyl acetate. The resulted organic layer was washed sequentially with water and brine and dried over anhydrous magnesium sulfate, and then the solvent was removed under reduced pressure. The residue was stirred with a mixed solution of hexane/diisopropyl ether (v/v = 1/1), and then the resulted solid was collected by filtration to give the titled compound (430 mg).
¹H-NMR (DMSO-D₆) δ: 9.05 (1H, s), 7.59-7.57 (2H, m), 7.51 (2H, s), 7.45-7.41 (3H, m), 5.65 (2H, s), 2.04-1.98 (9H, m).

### Step 4-11: 2-{4-(1,1-Difluoroethyl)-2,6-dimethylphenyl}-6-(1-methyl-1H-imidazol-4-yl)-2,5-dihydro-4H-pyrazolo[3,4-d]pyrimidin-4-one

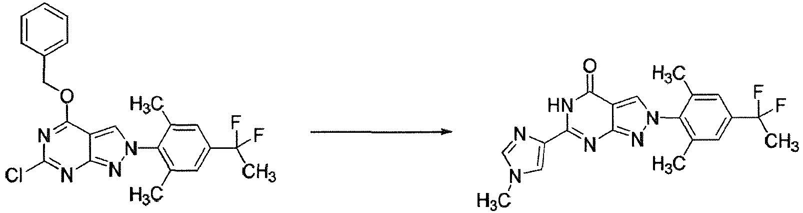

Under an argon atmosphere, to a mixture of 4-(benzyloxy)-6-chloro-2-{4-(1,1-difluoroethyl)-2,6-dimethylphenyl}-2H-pyrazolo[3,4-d]pyrimidine (80 mg) and N,N-dimethylacetamide (1.0 mL) were added bis(di-tert-butyl(4-dimethylaminophenyl)phosphine)dichloropalladium (II) (13 mg), lithium chloride (7.9 mg), and 1-methyl-4-(tributylstannyl)-1H-imidazole (0.12 mL), and the mixture was stirred at 120°C for 3 hours. The reaction mixture was purified by column chromatography (Developing solvent: methanol/ethyl acetate) and reversed-phase column chromatography (Developing solvent: acetonitrile/water) to give the titled compound (8.2 mg).
¹H-NMR (DMSO-D₆) δ: 10.78 (1H, s), 8.77 (1H, s), 8.04 (1H, d, *J* = 1.3 Hz), 7.84 (1H, d, *J* = 1.3 Hz), 7.49 (2H, s), 3.77 (3H, s), 2.05-1.98 (9H, m).
LC-MS (MH+): 385.

### [Preparation example 5]: Synthesis of 2-(4-bromo-2,6-dimethylphenyl)-6-(pyrimidine-4-yl)-2,5-dihydro-4H-pyrazolo[3,4-d]pyrimidin-4-one (Example 12)

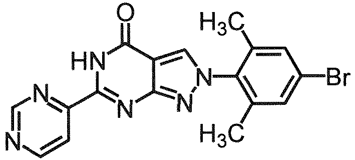

### Step 5-1: Methyl 2-{2-(4-bromo-2,6-dimethylphenyl)hydrazinylidene}-2-chloroacetate

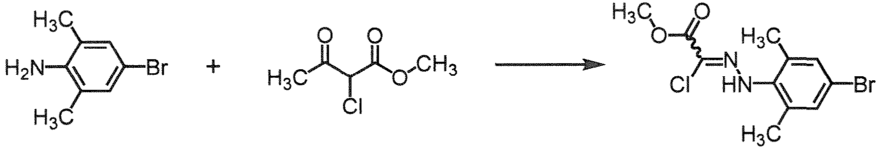

A mixture of 4-bromo-2,6-dimethylaniline (45 g), ethanol (72 mL), water (25 mL), and concentrated hydrochloric acid (47 mL) was cooled to -10°C, and then thereto was added dropwise and slowly an aqueous solution (54 mL) of sodium nitrite (17 g) over 30 minutes. The mixture was stirred at the same temperature for 30 minutes, and then thereto were added sequentially methyl 2-chloro-3-oxobutanoate (27 mL) and sodium acetate (55 g). Then, the mixture was stirred at room temperature for 2 hours. The reaction mixture was extracted with ethyl acetate. The resulted organic layer was dried over anhydrous magnesium sulfate, and then the solvent was removed under reduced pressure to give a crude product of the titled compound (81 g).
¹H-NMR (CDCl₃) δ: 7.28 (2H, t, *J* = 0.6 Hz), 7.19 (1H, br s), 3.90 (3H, s), 2.34 (6H, s).

### Step 5-2: Methyl 1-(4-bromo-2,6-dimethylphenyl)-4-cyano-1H-pyrazole-3-carboxylate

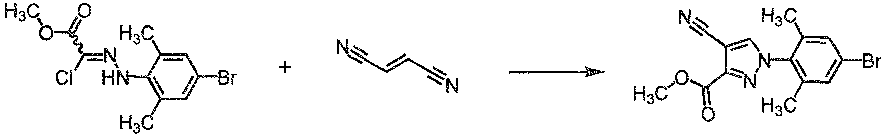

Under an argon atmosphere, to a mixture of a crude product of methyl 2-{2-(4-bromo-2,6-dimethylphenyl)hydrazinylidene}-2-chloroacetate (81 g) and chloroform (580 mL) were added triethylamine (36 mL) and fumaronitrile (20 g), and then the mixture was stirred at 80°C for 40 minutes. The reaction mixture was let cool to room temperature, and then thereto was added silica gel (250 g). Then, the mixture was stirred at room temperature for 30 minutes. Silica gel was removed by filtration with Celite, and then washed with ethyl acetate. Solvent was removed from all the resulted organic layers under reduced pressure, and then the resulted solid was washed with ethyl acetate to give the titled compound (37 g).
¹H-NMR (DMSO-d₆) δ: 9.01 (1H, s), 7.57 (2H, s), 3.90 (3H, s), 1.98 (6H, s).

### Step 5-3: 1-(4-Bromo-2,6-dimethylphenyl)-4-cyano-1H-pyrazole-3-carboxylic acid

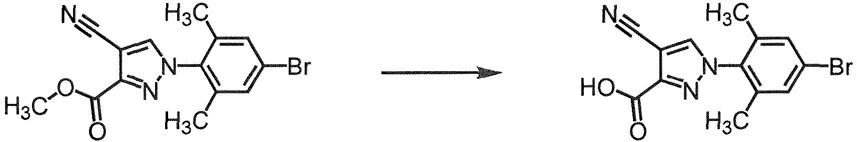

To a mixture of methyl 1-(4-bromo-2,6-dimethylphenyl)-4-cyano-1H-pyrazole-3-carboxylate (36 g) and methanol (360 mL) was added 2 M aqueous sodium hydroxide solution (110 mL), and the mixture was stirred at room temperature for 2.5 hours. The reaction mixture was adjusted to pH = 1 by addition of 6 M hydrochloric acid, and then extracted with ethyl acetate. The resulted organic layer was dried over anhydrous magnesium sulfate, and then the solvent was removed under reduced pressure. To the resulted solid was added a mixed solution of ethyl acetate/diisopropyl ether (v/v = 1/1, 200 mL), and the mixture was stirred for 5 minutes. Then, hexane was added thereto until a solid stopped precipitating. The resulted solid was collected by filtration to give the titled compound (30 g).
¹H-NMR (DMSO-d₆) δ: 13.86 (1H, br s), 8.96 (1H, s), 7.56 (2H, s), 1.97 (6H, s).

### Step 5-4: 3-Amino-1-(4-bromo-2,6-dimethylphenyl)-1H-pyrazole-4-carbonitrile

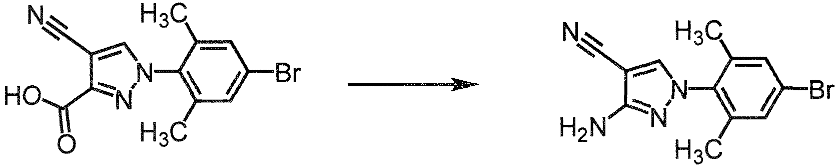

Under an argon atmosphere, to a mixture of 1-(4-bromo-2,6-dimethylphenyl)-4-cyano-1H-pyrazole-3-carboxylic acid (30 g) and t-butanol (450 mL) were added triethylamine (26 mL) and diphenylphosphoryl azide (30 mL) at room temperature, and then the mixture was stirred at 90°C for 3 hours. The solvent was removed under reduced pressure, and then to the residue were added chloroform (180 mL) and trifluoroacetic acid (22 mL). The mixture was stirred at 80°C for 1 hour. The reaction mixture was cooled to 0°C, and then neutralized with aqueous saturated sodium hydrogen carbonate solution. The reaction mixture was extracted with ethyl acetate, and the resulted organic layer was dried over anhydrous sodium sulfate, and then the solvent was removed under reduced pressure. The residue was purified by column chromatography (Developing solvent: 15 vol% to 60 vol% of ethyl acetate/hexane) and then, the resulted solid was washed with a mixed solution of diisopropyl ether/ethyl acetate (v/v = 1/1) to give the titled compound (11 g).
¹H-NMR (DMSO-d₆) δ: 8.31 (1H, s), 7.46 (2H, s), 5.76 (2H, s), 2.01 (6H, s).
LC-MS (MH+): 291.

### Step 5-5: 3-Amino-1-(4-bromo-2,6-dimethylphenyl)-1H-pyrazole-4-carboxamide

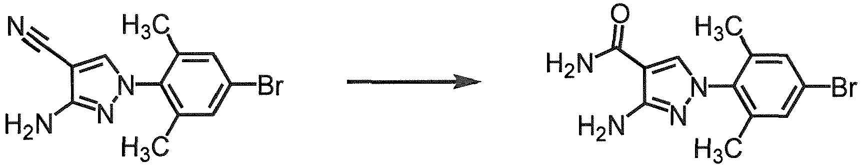

Under an argon atmosphere, to a mixture of 3-amino-1-(4-bromo-2,6-dimethylphenyl)-1H-pyrazole-4-carbonitrile (11 g), dimethylsulfoxide (17 mL), and ethanol (66 mL) were added sodium hydroxide (4.5 g) and 30% hydrogen peroxide solution (15 mL) at 0°C, and the mixture was stirred at room temperature for 40 minutes. The reaction mixture was cooled to 0°C, and then thereto was addded aqueous saturated sodium sulfite solution. The reaction mixture was adjusted to pH = 2 by addition of 12 M hydrochloric acid, and then extracted with ethyl acetate. The resulted organic layer was dried over anhydrous magnesium sulfate, and then the solvent was removed under reduced pressure. The resulted solid was washed with a mixed solution of diisopropyl ether/ethyl acetate (v/v = 1/1) to give the titled compound (10 g).
¹H-NMR (DMSO-d₆) δ: 7.97 (1H, s), 7.45 (2H, s), 7.31 (1H, br s), 6.91 (1H, br s), 5.53 (2H, s), 2.02 (6H, s).
LC-MS (MH+): 309.

### Step 5-6: N-(1-(4-Bromo-2,6-dimethylphenyl)-4-carbamoyl-1H-pyrazol-3-yl)pyrimidine-4-carboxamide

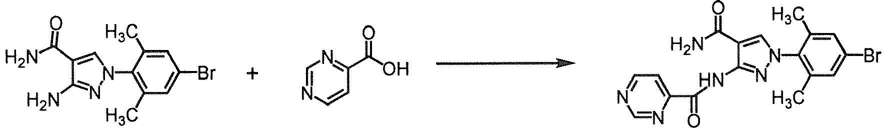

To a mixture of pyrimidine-4-carboxylic acid (60 mg) and N,N-dimethylformamide (1.5 mL) were added HATU (180 mg) and N,N-diisopropylethylamine (160 mg), and the mixture was stirred at room temperature for 5 minutes. Then, thereto was added 3-amino-1-(4-bromo-2,6-dimethylphenyl)-1H-pyrazole-4-carboxamide (75 mg), and the mixture was stirred at 50°C for 2 hours. The reaction mixture was let cool to room temperature and diluted with ethyl acetate, and then washed sequentially with water and brine. The resulted organic layer was dried over anhydrous magnesium sulfate, and then the solvent was removed under reduced pressure to give a crude product of the titled compound (100 mg).
LC-MS (MH+): 415.

### Step 5-7: 2-(4-Bromo-2,6-dimethylphenyl)-6-(pyrimidine-4-yl)-2,5-dihydro-4H-pyrazolo[3,4-d]pyrimidin-4-one

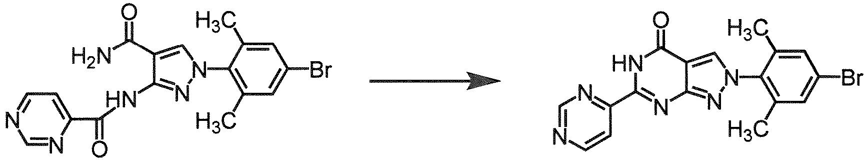

Under an argon atmosphere, to a mixture of a crude product of N-(1-(4-bromo-2,6-dimethylphenyl)-4-carbamoyl-1H-pyrazol-3-yl)pyrimidine-4-carboxamide (100 mg), ethanol (1.0 mL), and water (1.0 mL) was added 1,8-diazabicyclo[5.4.0]undec-7-ene (1.0 mL), and the mixture was stirred at 110°C for 2 hours. The reaction mixture was let cool to room temperature, and thereto was added water. Then, the mixture was extracted with ethyl acetate. The resulted organic layer was washed sequentially with water and brine and dried over anhydrous magnesium sulfate, and then the solvent was removed under reduced pressure. The residue was purified by column chromatography (Developing solvent: 50 vol% to 100 vol% of ethyl acetate/hexane) to give the titled compound (47 mg).
¹H-NMR (CDCl₃) δ: 10.62 (1H, br s), 9.36 (1H, d, *J* = 1.4 Hz), 9.03 (1H, d, J = 5.3 Hz), 8.54 (1H, dd, J = 5.2, 1.5 Hz), 8.18 (1H, s), 7.38 (2H, s), 2.06 (6H, s).
LC-MS (MH+): 397.

### [Preparation example 6]: Synthesis of 2-(6-chloro-2,3-dihydro-1H-inden-5-yl)-6-(1-methyl-1H-pyrazol-3-yl)-2,5-dihydro-4H-pyrazolo[3,4-d]pyrimidin-4-one (Example 59)

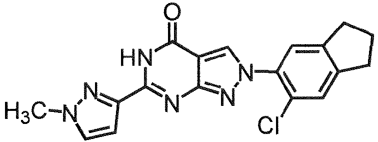

### Step 6-1: (6-Chloro-2,3-dihydro-1H-inden-5-yl)hydrazine hydrochloride

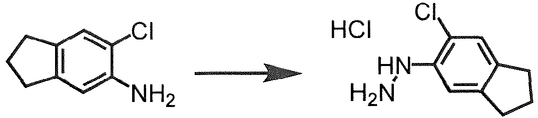

To a mixture of 6-chloro-2,3-dihydro-1H-inden-5-amine (1.0 g) and 6 M hydrochloric acid (5.0 mL) was added concentrated hydrochloric acid (3.0 mL), and then the mixture was cooled to 0°C. To the reaction mixture was added dropwise and slowly an aqueous solution (1.0 mL) of sodium nitrite (0.43 g) over 3 minutes. Then, the mixture was stirred at the same temperature for 70 minutes. To the reaction mixture was added dropwise and slowly a mixture of tin (II) chloride dihydrate (2.8 g) and concentrated hydrochloric acid (2.3 mL) over 5 minutes. The mixture was stirred at the same temperature for 1 hour, and then stirred at room temperature for 2 hours. The resulted solid was collected by filtration, and then washed sequentially with 2M hydrochloric acid and diisopropyl ether to give the titled compound (1.2 g).
¹H-NMR (DMSO-D₆) δ: 10.12 (3H, br s), 7.86 (1H, br s), 7.28 (1H, s), 7.00 (1H, s), 2.83-2.81 (4H, m), 2.06-1.99 (2H, m).

### Step 6-2: Lithium 6-hydroxy-2-(1-methyl-1H-pyrazol-3-yl)pyrimidin-4-olate

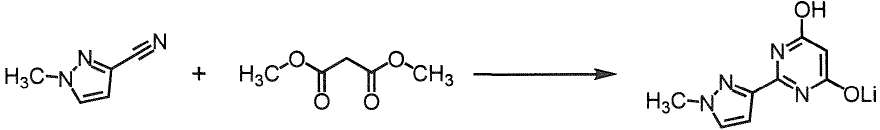

Under an argon atmosphere, to a mixture of 1-methyl-1H-pyrazole-3-carbonitrile (53 g) and tetrahydrofuran (11 mL) was added a 1.2 M solution (500 mL) of lithium bis(trimethylsilyl)amide in tetrahydrofuran at 0°C, and the mixture was stirred at room temperature for 2 hours. The reaction mixture was cooled to 5°C, and then thereto were added dimethyl malonate (110 mL) and methanol (320 mL). The mixture was stirred at 80°C for 24 hours. The reaction mixture was cooled to room temperature, and then thereto was added tetrahydrofuran (320 mL). The mixture was stirred at room temperature for 1 hour. The resulted solid was collected by filtration, and then washed sequentially with a mixed solution of methanol/tetrahydrofuran (v/v = 1/2, 150 mL) and tetrahydrofuran (200 mL) to give the titled compound (100 g).
¹H-NMR (CD₃OD) δ: 7.64 (1H, d, J = 2.2 Hz), 6.91 (1H, d, J = 2.2 Hz), 5.11 (1H, s), 3.97 (3H, s).

### Step 6-3: 4,6-Dichloro-2-(1-methyl-1H-pyrazol-3-yl)pyrimidine-5-carbaldehyde

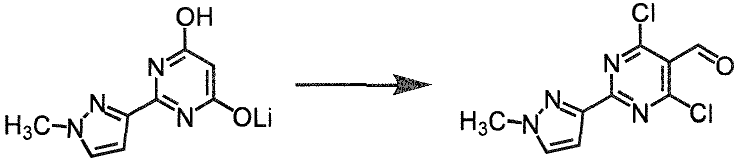

Under a nitrogen atmosphere, phosphorus oxychloride (64 mL) was added to N,N-dimethylformamide (270 mL) at 0°C, and then the mixture was stirred at room temperature for 1 hour. The mixture was cooled to 0°C, and then added dropwise and slowly to a mixture of lithium 6-hydroxy-2-(1-methyl-1H-pyrazol-3-yl)pyrimidin-4-olate (90 g) and toluene (270 mL) which was cooled to 0°C. The reaction mixture was stirred at room temperature overnight, and then cooled to 0°C again. To the reaction mixture was added dropwise and slowly phosphorus oxychloride (380 mL), and then the mixture was stirred at 80°C for 6 hours. The reaction mixture was let cool to room temperature, and then was added dropwise and slowly to a mixture of disodium hydrogenphosphate (770 g) and water (3.0 L) which was cooled below 10°C. The mixture was stirred at the same temperature for 30 minutes, and then stirred at room temperature for 1 hour. The resulted solid was collected by filtration, and then washed with water to give the titled compound (79 g).
¹H-NMR (DMSO-D₆) δ: 10.28 (1H, s), 7.92 (1H, d, *J* = 2.5 Hz), 7.03 (1H, d, *J* = 2.5 Hz), 4.00 (3H, s).

### Step 6-4: 4-Chloro-2-(6-chloro-2,3-dihydro-1H-inden-5-yl)-6-(1-methyl-1H-pyrazol-3-yl)-2H-pyrazolo[3,4-d]pyrimidine

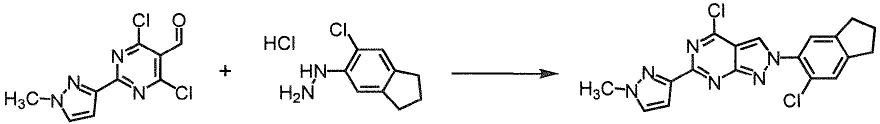

To a mixture of (6-chloro-2,3-dihydro-1H-inden-5-yl)hydrazine hydrochloride (100 mg), N,N-diisopropylethylamine (0.40 mL), tetrahydrofuran (1.5 mL), and water (0.50 mL) was added 4,6-dichloro-2-(1-methyl-1H-pyrazol-3-yl)pyrimidine-5-carbaldehyde (120 mg), and the mixture was stirred at room temperature for 1 hour. To the reaction mixture was added water, and then the mixture was extracted with ethyl acetate. The resulted organic layer was washed with brine and dried over anhydrous magnesium sulfate, and then the solvent was removed under reduced pressure. The residue was purified by column chromatography (Developing solvent: 1 vol% to 100 vol% of ethyl acetate/hexane) to give the titled compound (55 mg).
¹H-NMR (DMSO-D₆) δ: 9.25 (1H, s), 7.85 (1H, d, *J* = 2.2 Hz), 7.66-7.64 (2H, m), 6.99 (1H, d, *J* = 2.2 Hz), 3.98 (3H, s), 3.02-2.93 (4H, m), 2.15-2.10 (2H, m).

### Step 6-5: 2-(6-Chloro-2,3-dihydro-1H-inden-5-yl)-6-(1-methyl-1H-pyrazol-3-yl)-2,5-dihydro-4H-pyrazolo[3,4-d]pyrimidin-4-one

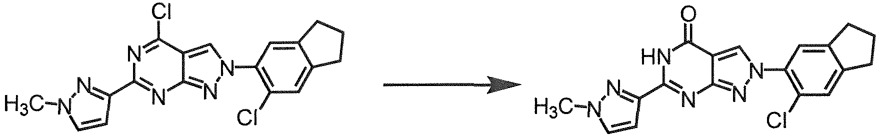

Under an argon atmosphere, to a mixture of 4-chloro-2-(6-chloro-2,3-dihydro-1H-inden-5-yl)-6-(1-methyl-1H-pyrazol-3-yl)-2H-pyrazolo[3,4-d]pyrimidine (55 mg), acetonitrile (0.55 mL), and water (0.28 mL) was added trifluoroacetic acid (0.033 mL), and the mixture was stirred at 60°C for 1 hour. To the reaction mixture was added water, and then the mixture was extracted with ethyl acetate. The resulted organic layer was washed with brine and dried over anhydrous magnesium sulfate, and then the solvent was removed under reduced pressure. The residue was purified by column chromatography (Developing solvent: 1 vol% to 100 vol% of ethyl acetate/hexane), and then treated with a mixed solution of ethyl acetate/hexane (v/v = 1/5, 12 mL) to give a solid, the titled compound (44 mg).
¹H-NMR (DMSO-D₆) δ: 11.28 (1H, br s), 8.85 (1H, s), 7.90 (1H, d, *J* = 2.5 Hz), 7.57 (2H, d, *J* = 11.5 Hz), 6.96 (1H, d, *J* = 2.5 Hz), 3.98 (3H, s), 2.98-2.91 (4H, m), 2.14-2.07 (2H, m). LC-MS (MH+): 367.

Example compounds in addition to those described above were obtained in a similar manner to the above Preparation methods and Preparation examples, or if necessary, by known methods. The structure and physical property data of each Example compound are shown in the following tables.

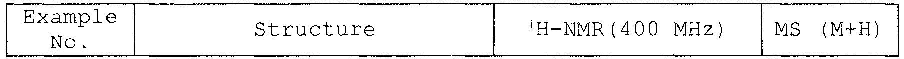

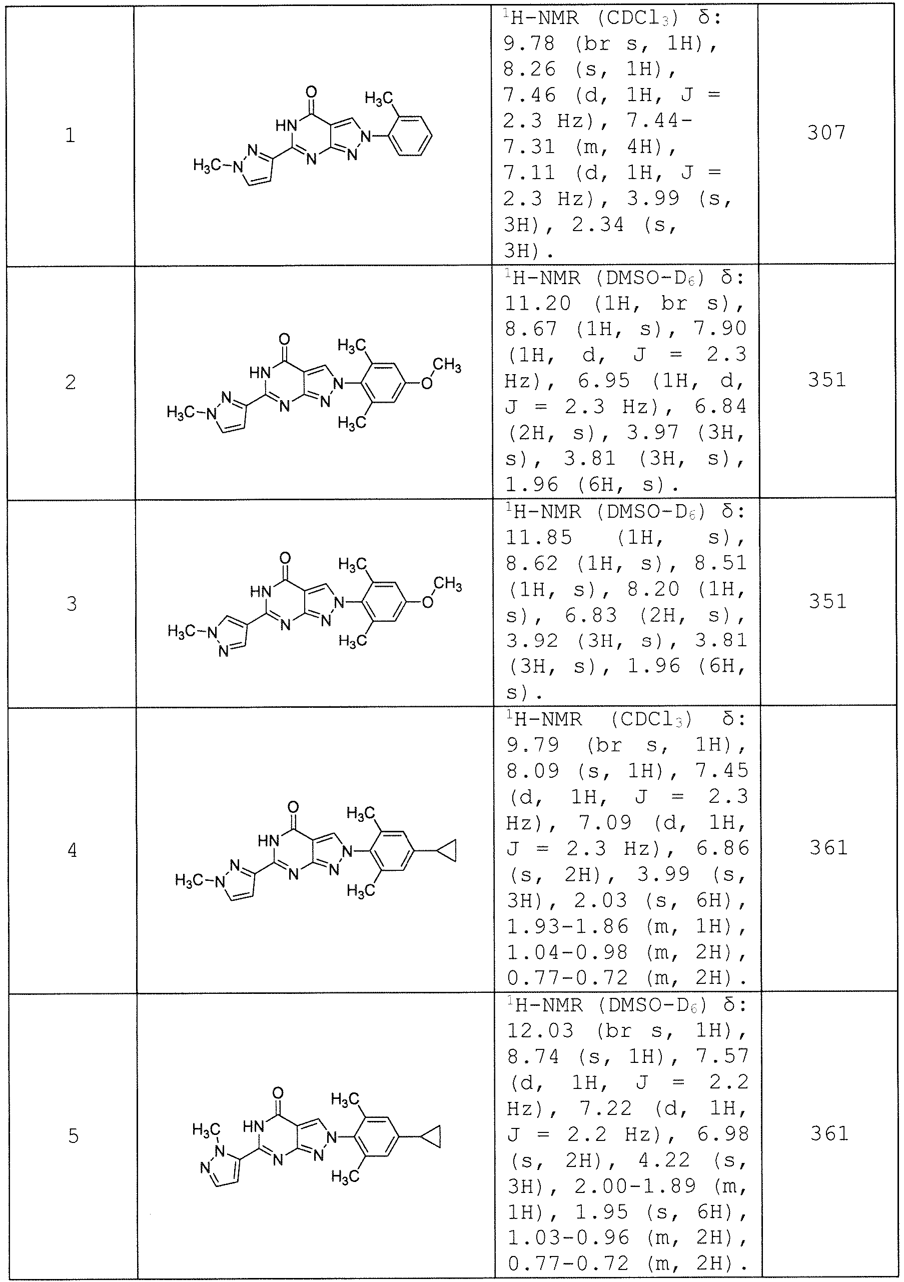

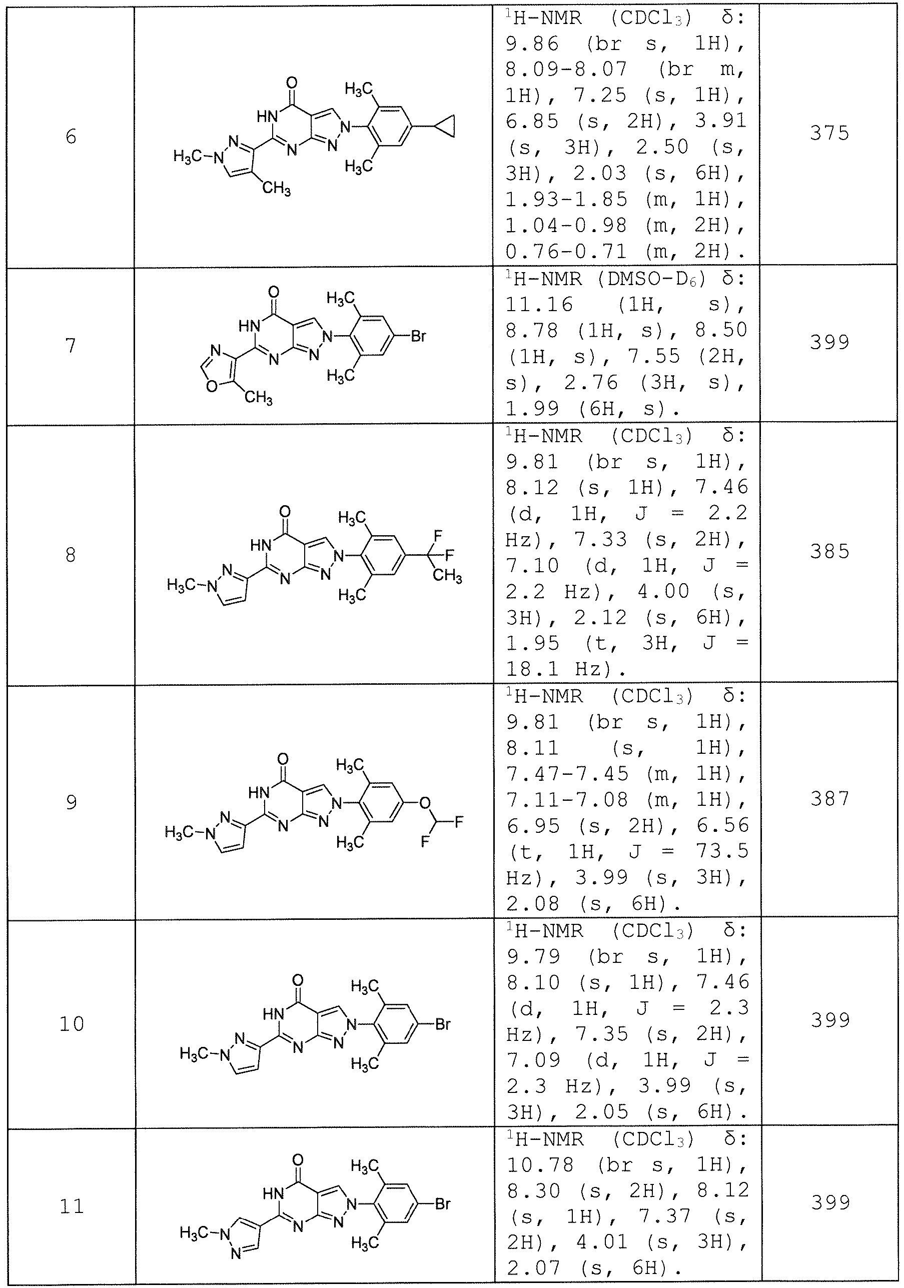

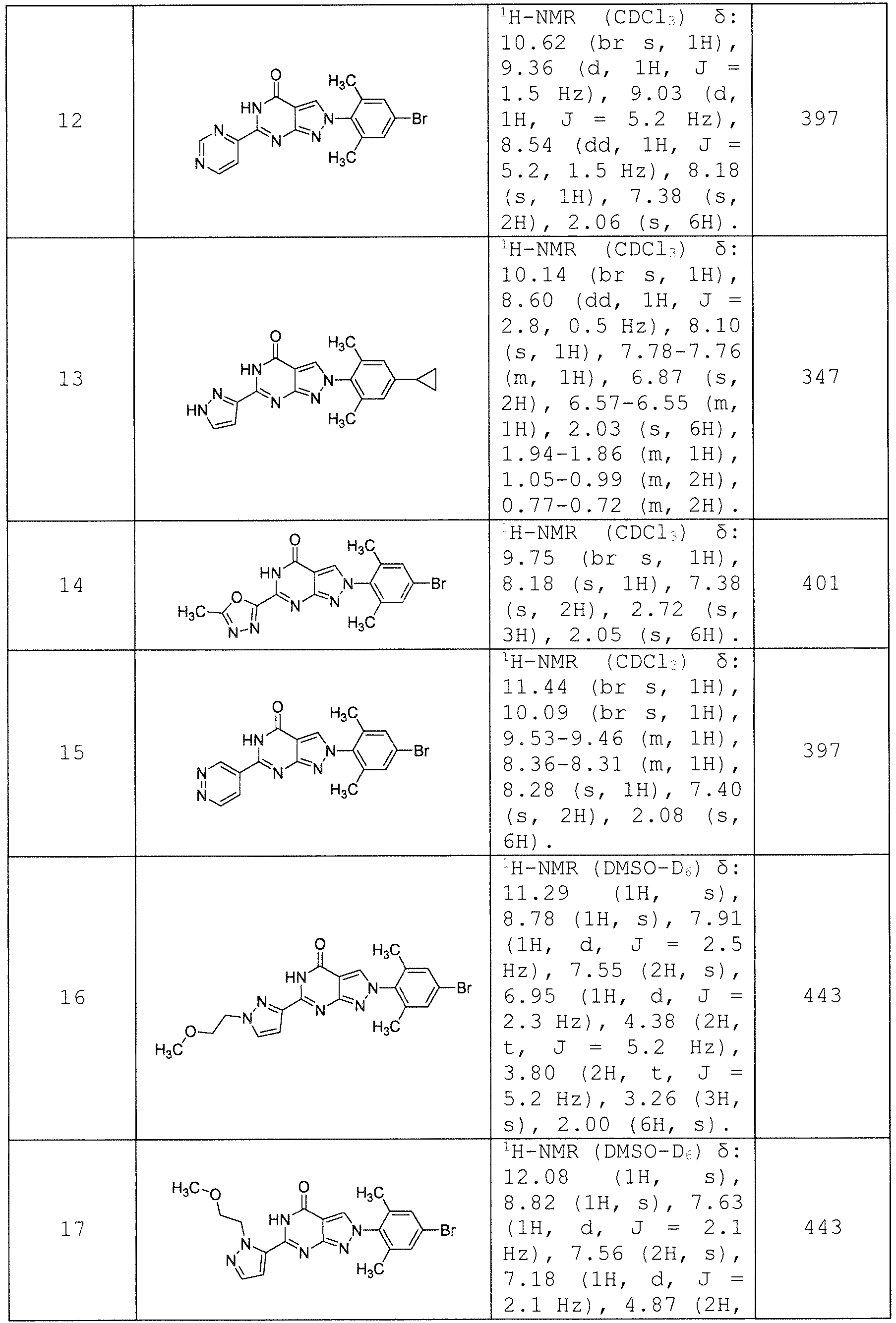

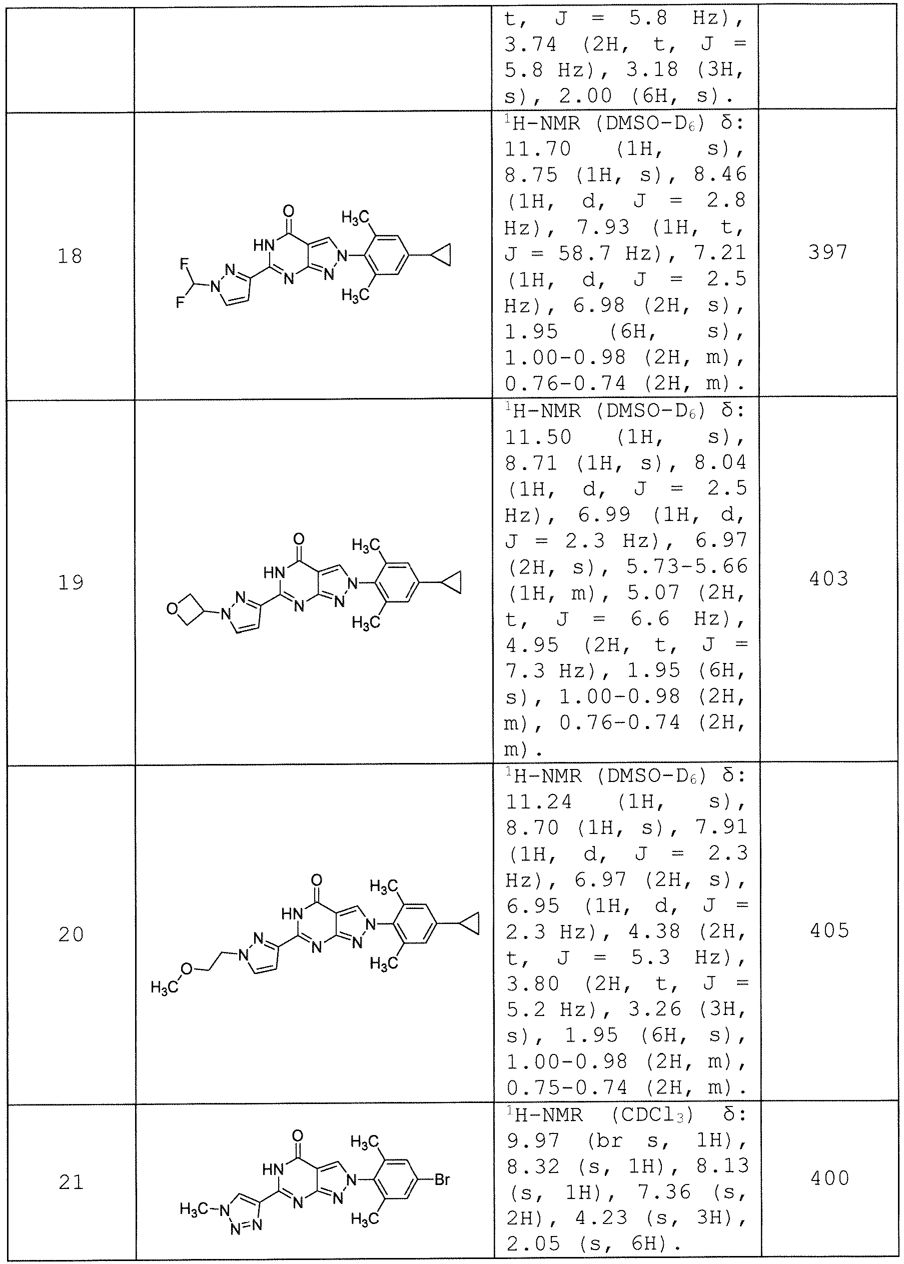

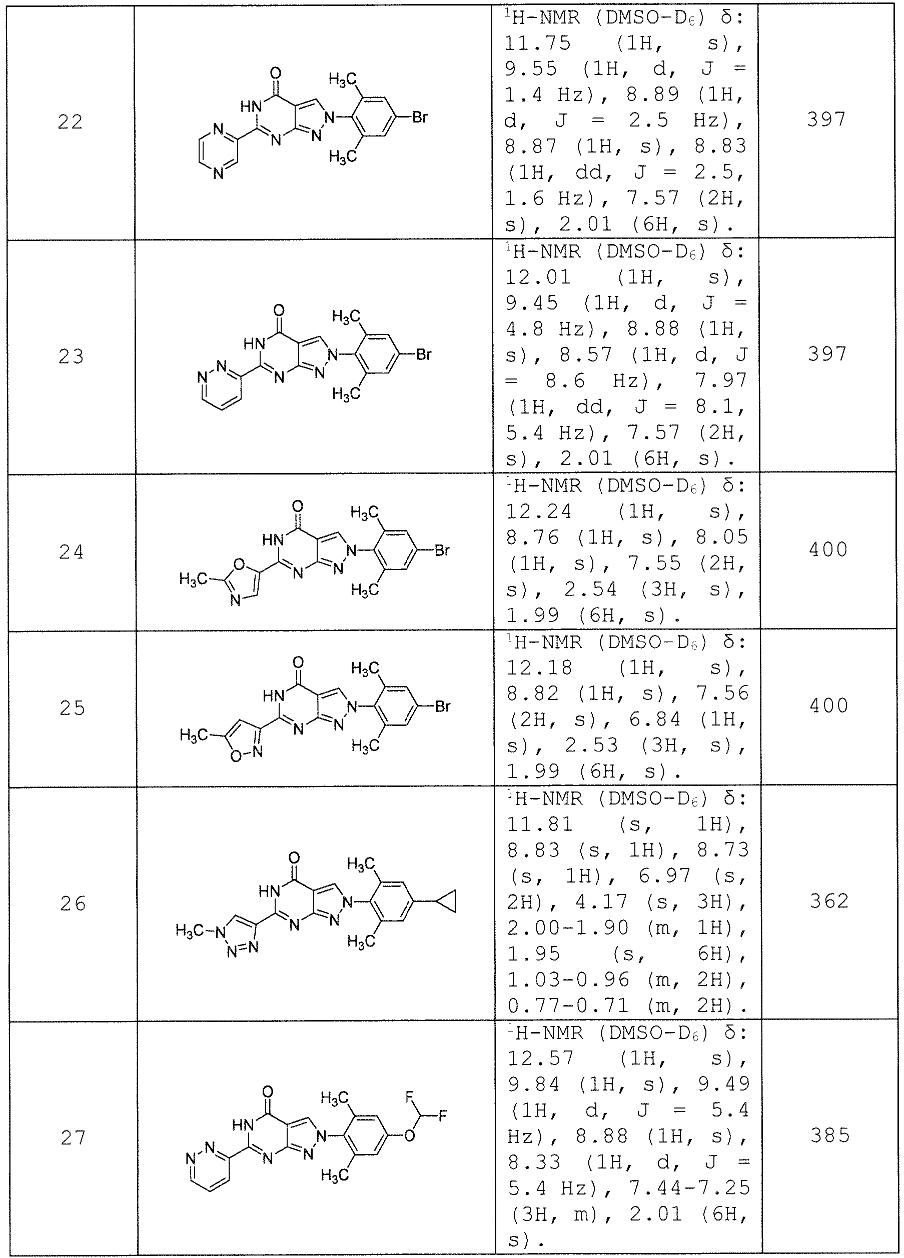

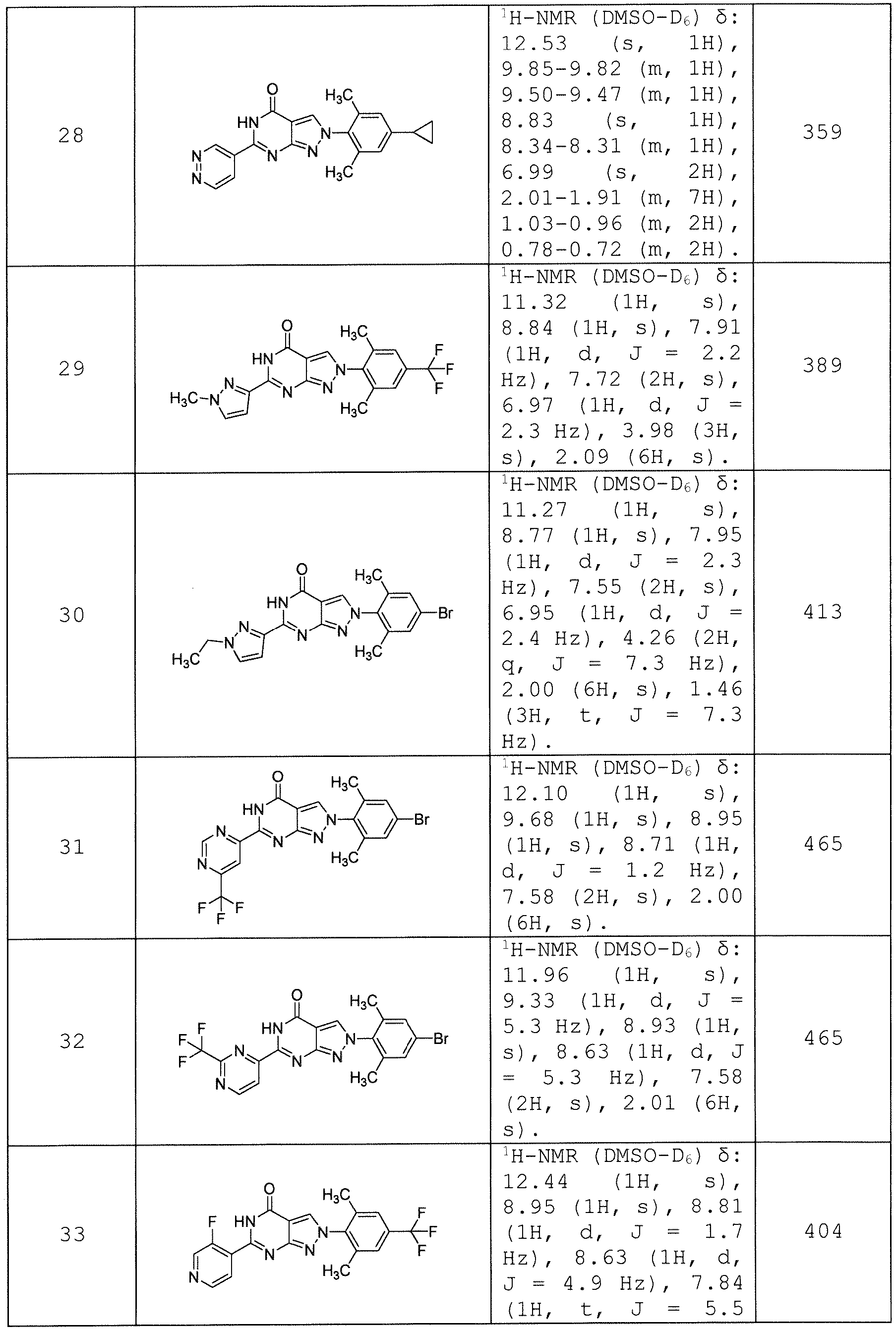

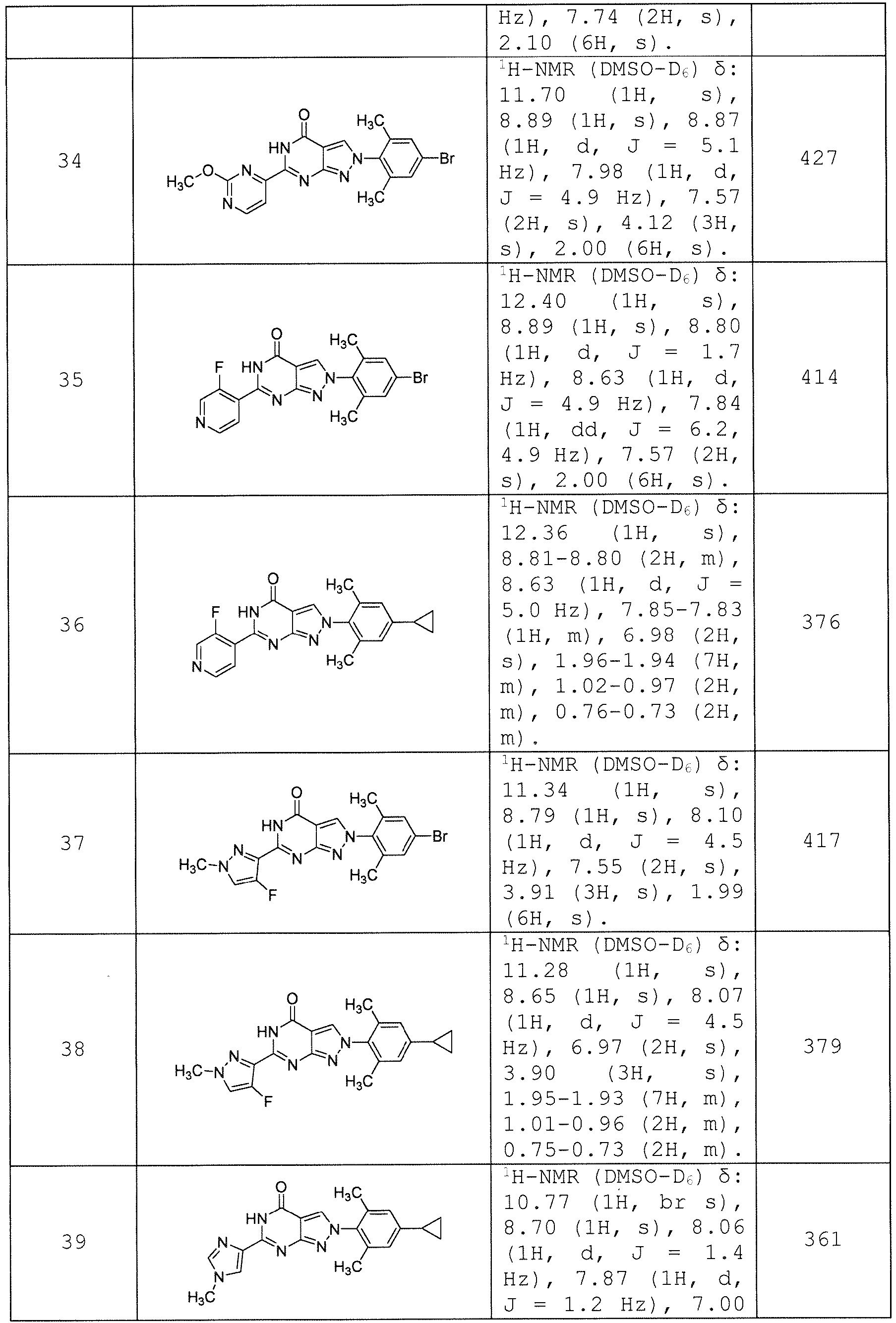

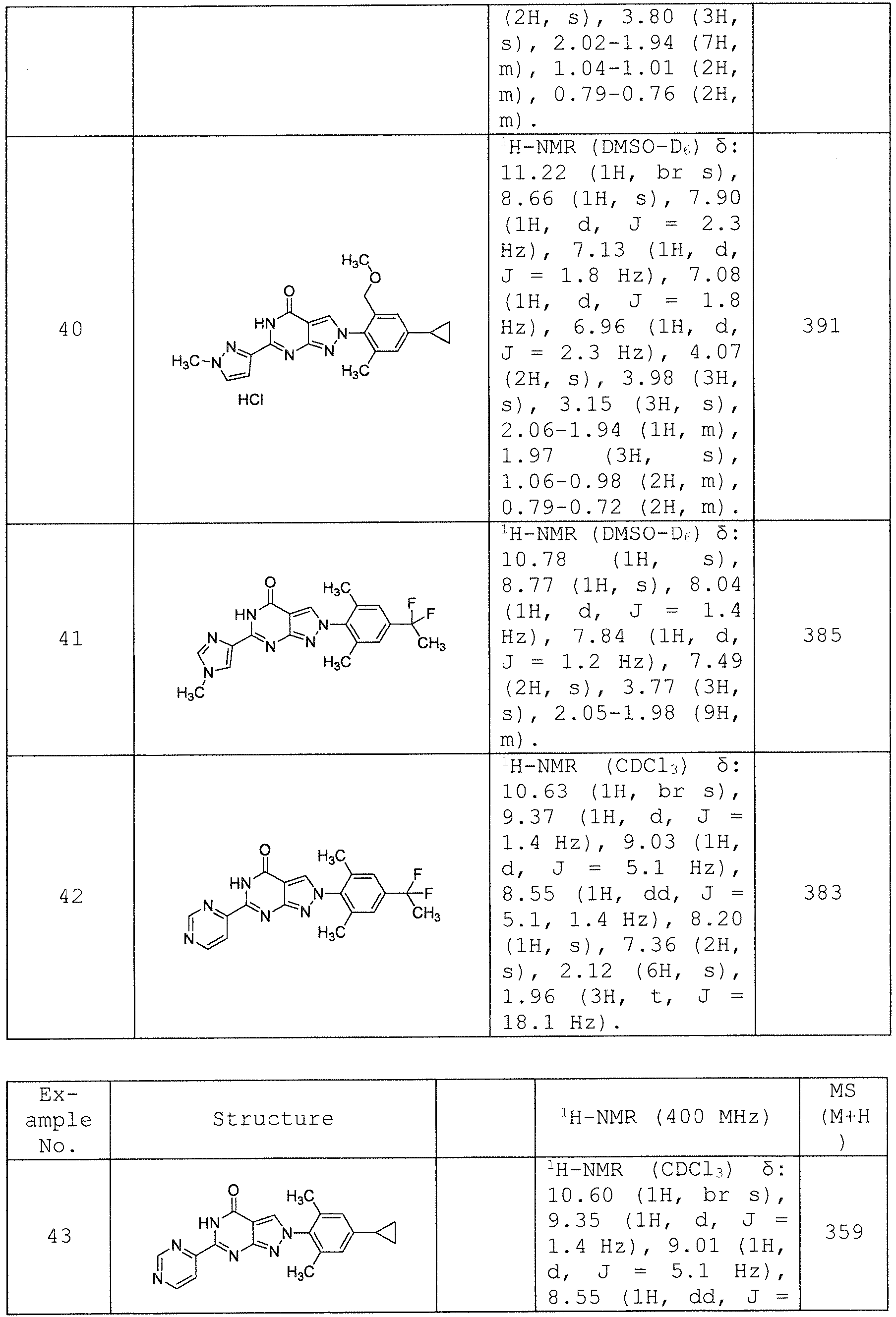

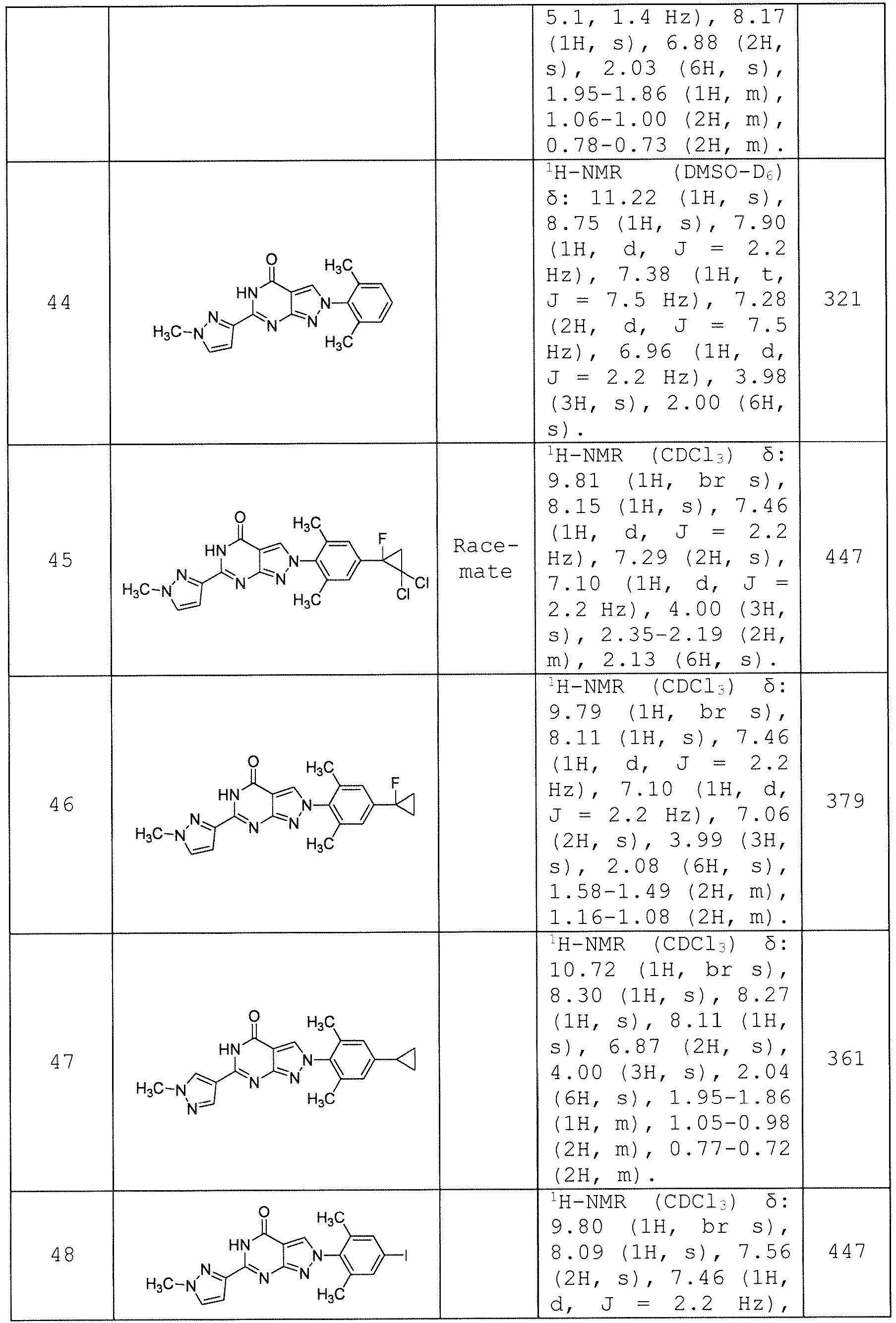

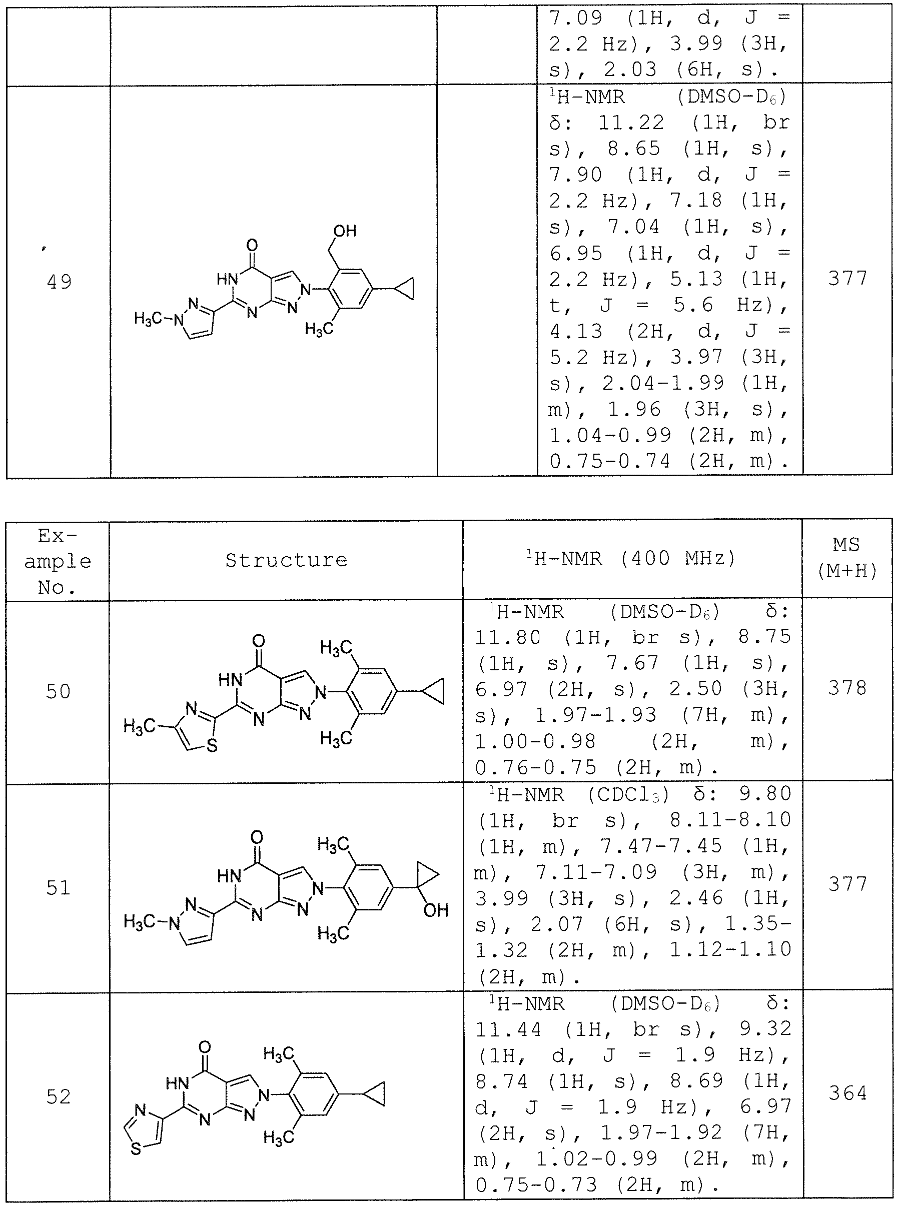

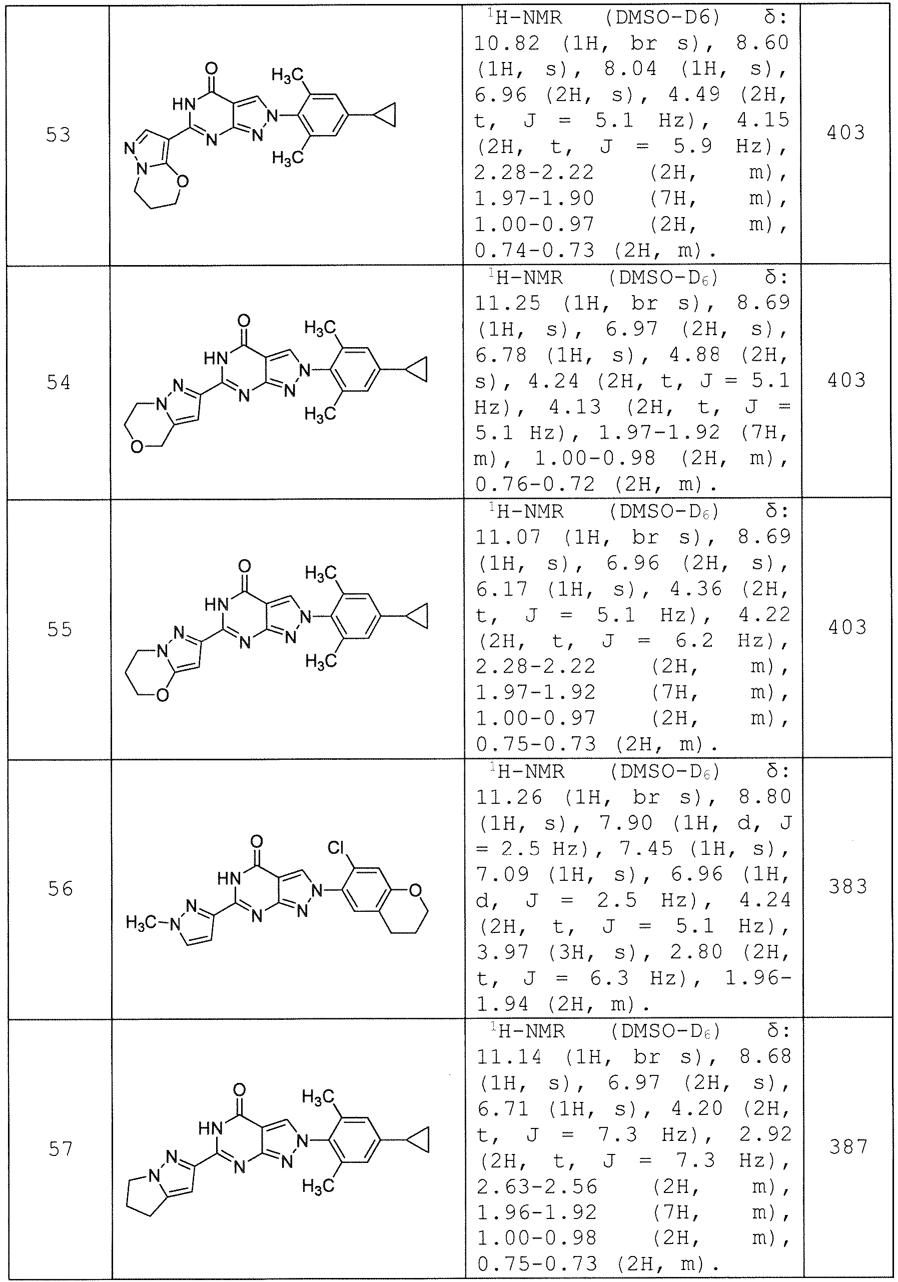

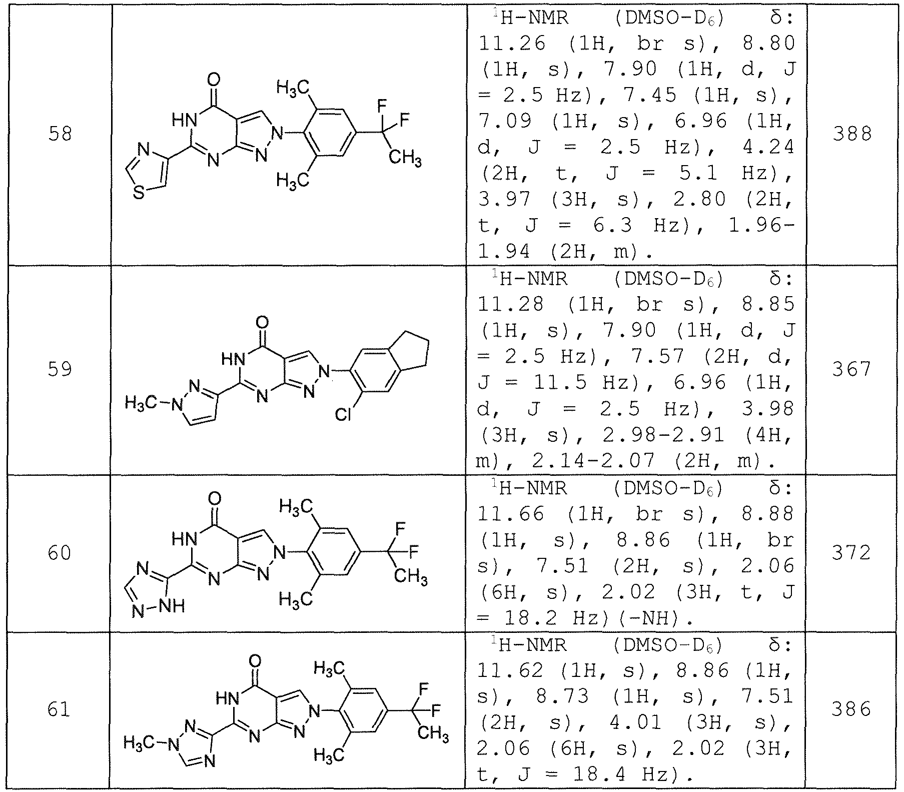

### Test example 1: Evaluation of NLRP3 inflammasome inhibitory activity

The NLRP3 inflammasome inhibitory activity of test compounds were evaluated on the basis of the inhibitory activity of the IL-1β production in THP1-Null cells (Product Number: thp-null, InvivoGen). Cells were maintained for culture in RPMI-1640 media containing 10% (v/v) fetal bovine serum, 25 mmol/L HEPES, 100 U/mL penicillin, 100 µg/mL streptomycin, 100 µg/mL normocin, and 200 µg/mL hygromycin B (set at 37°C, 5% CO₂/95% air).

Cells were suspended with media for assay containing 0.5 µmol/L PMA (RPMI-1640 media containing 10% (v/v) fetal bovine serum, 100 U/mL penicillin, and 100 µg/mL streptomycin), and the suspended cells were seeded on Corning (registered trademark) 384-well Flat Clear Bottom Black Polystyrene TC-treated Microplates (25,000 cells/25 µL/well), followed by incubation (set at 37°C, 5% CO₂/95% air) overnight. The supernatant of the culture was removed, and thereto was added media for assay (25 µL/well) containing 1 µg/mL Lipopolysaccharides (Product Number: L2654, Sigma-Aldrich (registered trademark)). Then, the culture was further incubated for 3 hours (set at 37°C, 5% CO₂/95% air). The supernatant of the culture was removed. Then, a vehicle solution prepared from Opti-MEM (trademark) medium (Product Number: 31985-070, Invitrogen) was added to blank-setting wells and control-setting wells (20 µL/well), followed by incubation for 15 minutes (set at 37°C, 5% CO₂/95% air). A solution containing a test compound (20 µL/well) was added to test compound-setting wells. Further, Opti-MEM (trademark) medium containing Nigericin (Product Number: N7143, Sigma-Aldrich (registered trademark)) was added to the control-setting wells and test compound-setting wells (5 µL/well), followed by incubation for 1.5 hours (set at 37°C, 5% CO₂/95% air). The final concentration of Nigericin was adjusted to be 7.5 µmol/L. 5 µL/well of Opti-MEM (trademark) medium was added to the blank-setting wells. The supernatant of the culture was cryonically stored (set at -20°C) until measurement of IL-1β.

The amount of IL-1β in the culture supernatant was quantitated with AlphaLISA (registered trademark) Human IL-1β Detection Kit (Product Number: AL220C, Perkin Elmer). Fluorescence intensity was measured with a microplate reader EnSpier (Model number: 2300-00J, Perkin Elmer) or EnSight (Model number: HH34000000, Perkin Elmer) according to procedure manuals attached thereto. Inhibition rates of the test compound-setting wells were calculated on the basis of 100% for the blank-setting wells and 0% for the control-setting wells. IC₅₀ values (i.e., 50% inhibitory concentrations) of the test compounds were calculated by logistic regression analysis. The result of each Example compound is shown in the following tables.

| Example No. | IC₅₀ (µM) |
|---|---|
| 1 | 3.8 |
| 2 | 0.064 |
| 3 | 0.11 |
| 4 | 0.015 |
| 5 | 0.023 |
| 6 | 0.05 |
| 7 | 0.11 |
| 8 | 0.033 |
| 9 | 0.049 |
| 10 | 0.02 |
| 11 | 0.018 |
| 12 | 0.0078 |
| 13 | 0.029 |
| 14 | 0.39 |
| 15 | 0.015 |
| 16 | 0.047 |
| 17 | 0.22 |
| 18 | 0.055 |
| 19 | 0.062 |
| 20 | 0.023 |
| 21 | 0.013 |
| 22 | 0.068 |
| 23 | 0.022 |
| 24 | 0.053 |
| 25 | 0.015 |
| 26 | 0.012 |
| 27 | 0.062 |
| 28 | 0.011 |
| 29 | 0.069 |
| 30 | 0.04 |
| 31 | 0.94 |
| 32 | 0.077 |
| 33 | 0.022 |
| 34 | 0.027 |
| 35 | 0.024 |
| 36 | 0.016 |
| 37 | 0.036 |
| 38 | 0.015 |
| 39 | 0.023 |
| 40 | 0.0063 |
| 41 | 0.04 |
| 42 | 0.014 |
| 43 | 0.0043 |
| 44 | 0.88 |
| 45 | 34% inhibition at 2.4 µM |
| 46 | 0.0031 |
| 47 | 0.0067 |
| 48 | 0.0025 |
| 49 | 0.037 |

| Example No. | IC₅₀ (µM) |
|---|---|
| 50 | 0.006 |
| 51 | 35% inhibition at 0.3 µM |
| 52 | 0.002 |
| 53 | 0.24 |
| 54 | 0.009 |
| 55 | 0.024 |
| 56 | 0.084 |
| 57 | 0.009 |
| 58 | 0.003 |
| 59 | 0.034 |
| 60 | 0.39 |
| 61 | 0.49 |

Formulation examples of the present invention include the following formulations, but are not intended to be limited thereto.

### Formulation Example 1: Preparation of a capsule

| | | |
|---|---|---|
| (1) | A compound of Example 1 | 30 mg |
| (2) | Microcrystalline cellulose | 10 mg |
| (3) | Lactose | 19 mg |
| (4) | Magnesium stearate | 1 mg |

Ingredients (1), (2), (3), and (4) are mixed to be filled in a gelatin capsule.

### Formulation Example 2: Preparation of a tablet

| | | |
|---|---|---|
| (1) | A compound of Example 1 | 10 g |
| (2) | Lactose | 50 g |
| (3) | Cornstarch | 15 g |
| (4) | Carmellose calcium | 44 g |
| (5) | Magnesium stearate | 1 g |

The total amounts of Ingredients (1), (2), and (3) and 30 g of Ingredient (4) are combined with water, dried in vacuo, and then granulated. The resulted granules are mixed with 14 g of Ingredient (4) and 1 g of Ingredient (5), and tableted with a tabletting machine. In this manner, 1,000 tablets of which each tablet comprises 10 mg of the compound of Example 1 are obtained.

### INDUSTRIAL APPLICABILITY

A compound of Formula [I], or a pharmaceutically acceptable salt thereof, or a compound of Formula [IA], or a pharmaceutically acceptable salt thereof, has an NLRP3 inflammasome inhibitory activity, and thus is expected to be useful for treating or preventing a disease selected from the group consisting of multiple sclerosis, chronic kidney disease, inflammatory bowel disease (for example, ulcerative colitis and Crohn's disease), arteriosclerosis, Cryopyrin-associated periodic syndrome (for example, familial cold autoinflammatory syndrome, Muckle-Wells syndrome, chronic infantile neurologic cutaneous and articular syndrome, and Neonatal onset multisystem inflammatory disease), nonalcoholic steatohepatitis, gout, gouty arthritis, rheumatoid arthritis, contact dermatitis, dry eye, ischemic heart disease (for example, acute myocardial infarction), systemic lupus erythematosus, systemic juvenile idiopathic arthritis, recurrent pericarditis, adult onset Still's disease (for example, hemophagocytic lymphohistiocytosis and macrophage activation syndrome), Schnitzler syndrome, deficiency of the IL-1 receptor antagonist, familial Mediterranean fever, mevalonate kinase deficiency, hyper IgD syndrome, Behcet's disease, lung cancer, psoriasis, hypertension, diabetic retinopathy, Alzheimer's disease, mild cognitive impairment, Parkinson's disease, Huntington's disease, amyotrophic lateral sclerosis, traumatic brain injury, cerebral infarct, intracerebral bleeding, epilepsy, depressive illness, autism spectrum disorder, spinal cord injury, septic encephalopathy, neuropathic pain, COVID-19, frontotemporal dementia, age-related macular degeneration, diabetic macular edema, hereditary transient corneal endotheliitis, and TNF receptor-associated periodic syndrome.

## Claims

1. A compound of Formula [IA]: 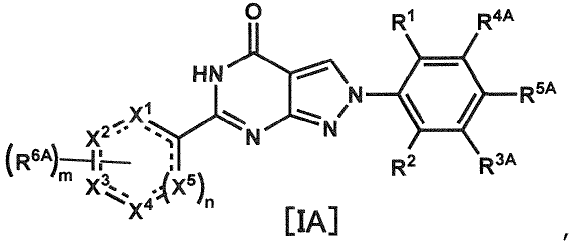
or a pharmaceutically acceptable salt thereof,
wherein a bond represented by: 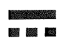
is a single bond or a double bond;
R¹ and R² are each independently
(1) hydrogen,
(2) hydroxy,
(3) cyano,
(4) C₁₋₆ alkyl, wherein the alkyl may be optionally substituted with 1 or 2 substituents independently selected from the group consisting of:
(a) hydroxy,
(b) C₁₋₄ alkoxy, and
(c) C₃₋₆ cycloalkyl,
(5) C₁₋₆ alkoxy, wherein the alkoxy may be optionally substituted with C₃₋₆ cycloalkyl,
(6) halogen,
(7) C₁₋₄ haloalkyl,
(8) -CHO,
(9) -O-C₁₋₄ haloalkyl,
(10) -O-C₃₋₆ cycloalkyl,
(11) -CO-C₁₋₄ alkyl,
(12) -CO-C₃₋₆ cycloalkyl,
(13) -NR⁷R⁸, wherein R⁷ and R⁸ are each independently hydrogen or 2,4-dimethoxybenzyl, or alternatively, R⁷ and R⁸ may be combined together with the nitrogen atom to which they attach and the -NR⁷R⁸ group may form 5- to 6-membered heterocycloalkyl comprising 1 or 2 heteroatoms independently selected from the group consisting of nitrogen and oxygen atom, or
(14) C₃₋₆ cycloalkyl,
R^{3A} and R^{4A} are each independently
(1) hydrogen,
(2) C₁₋₄ alkyl, or
(3) C₁₋₄ haloalkyl,
R^{5A} is
(1) hydrogen,
(2) cyano,
(3) C₁₋₆ alkyl,
(4) C₂₋₆ alkenyl,
(5) C₂₋₅ alkynyl,
(6) C₁₋₄ alkoxy,
(7) halogen,
(8) C₁₋₆ haloalkyl,
(9) C₂₋₆ haloalkenyl,
(10) -O-C₁₋₄ haloalkyl,
(11) C₃₋₆ cycloalkyl, wherein the cycloalkyl may be optionally substituted with 1 to 3 substituents independently selected from the group consisting of:
(a) hydroxy,
(b) halogen, and
(c) C₁₋₄ haloalkyl,
(12) C₅₋₆ cycloalkenyl, or
(13) 4- to 6-membered heterocycloalkyl comprising 1 or 2 heteroatoms independently selected from the group consisting of nitrogen and oxygen atom, or alternatively
R^{5A} may be combined together with R^{3A} or R^{4A} and the carbon atom to which they attach to form:
(1) C₅₋₆ cycloalkene, or
(2) 5- to 7-membered heterocycloalkene comprising 1 or 2 oxygen atoms,
R^{6A} is independently
(1) C₁₋₄ alkyl, wherein the alkyl may be optionally substituted with C₁₋₄ alkoxy,
(2) C₁₋₄ alkoxy,
(3) halogen,
(4) C₁₋₄ haloalkyl, or
(5) 4- to 6-membered heterocycloalkyl comprising 1 or 2 heteroatoms independently selected from the group consisting of nitrogen and oxygen atom,
m is 0, 1, or 2,
provided that when m is 2, then two adjacent R^{6A}s may be combined together with the two adjacent atoms among X¹, X², X³, X⁴, and X⁵ to which they attach to form 5- to 7-membered heterocycloalkane or heterocycloalkene comprising 1 or 2 heteroatoms independently selected from the group consisting of nitrogen and oxygen atom,
n is 0 or 1,
provided that when n is 0, then X¹, X², X³, and X⁴ are each independently carbon, nitrogen, oxygen, or sulfur atom, wherein a total number of nitrogen, oxygen, and sulfur atoms as X¹, X², X³, or X⁴ is 1, 2, or 3, and a total number of oxygen and sulfur atoms is 0 or 1, and X¹, X², X³, and X⁴ are combined together with the carbon atom that is adjacent to X¹ and X⁴ to form heteroaryl, and
provided that when n is 1, then X¹, X², X³, X⁴, and X⁵ are each independently carbon or nitrogen atom, wherein a total number of nitrogen atoms as X¹, X², X³, X⁴, or X⁵ is 1 or 2, and X¹, X², X³, X⁴, and X⁵ are combined together with the carbon atom that is adjacent to X¹ and X⁵ to form heteroaryl.

2. The compound according to claim 1, or a pharmaceutically acceptable salt thereof, wherein R^{3A} and R^{4A} are hydrogen.

3. The compound according to claim 1 or 2, or a pharmaceutically acceptable salt thereof, wherein the compound is represented by Formula [IIA]: 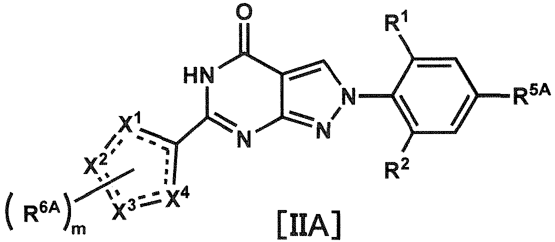 wherein R¹, R², R^{5A}, R^{6A}, X¹, X², X³, X⁴, and m are defined as those defined in claim 1.

4. The compound according to any one of claims 1 to 3, or a pharmaceutically acceptable salt thereof, wherein X¹, X², X³, and X⁴ are combined together with the carbon atom that is adjacent to X¹ and X⁴ to form pyrazolyl, imidazolyl, oxazolyl, isoxazolyl, thiazolyl, oxadiazolyl, or triazolyl.

5. The compound according to any one of claims 1 to 4, or a pharmaceutically acceptable salt thereof, wherein X¹, X², X³, and X⁴ are combined together with the carbon atom that is adjacent to X¹ and X⁴ to form pyrazolyl, imidazolyl, or thiazolyl.

6. The compound according to any one of claims 1 to 5, or a pharmaceutically acceptable salt thereof, wherein X¹, X², X³, and X⁴ are combined together with the carbon atom that is adjacent to X¹ and X⁴ to form a group of formula (1): 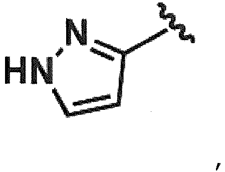 a group of formula (2): 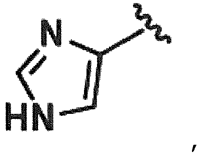 or
a group of formula (3):

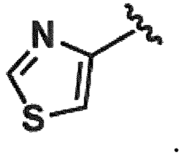

7. The compound according to claim 1 or 2, or a pharmaceutically acceptable salt thereof, wherein the compound is represented by Formula [IIIA]: 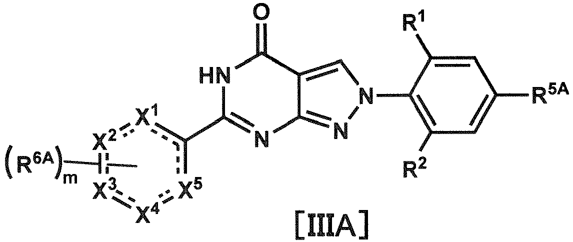 wherein R¹, R², R^{5A}, R^{6A}, X¹, X², X³, X⁴, X⁵, and m are defined as those defined in claim 1.

8. The compound according to any one of claim 1, 2, or 7, or a pharmaceutically acceptable salt thereof, wherein X¹, X², X³, X⁴, and X⁵ are combined together with the carbon atom that is adjacent to X¹ and X⁵ to form pyridyl, pyridazinyl, pyrimidyl, or pyrazinyl.

9. The compound according to any one of claim 1, 2, 7, or 8, or a pharmaceutically acceptable salt thereof, wherein X¹, X², X³, X⁴, and X⁵ are combined together with the carbon atom that is adjacent to X¹ and X⁵ to form pyridazinyl or pyrimidyl.

10. The compound according to any one of claim 1, 2, 7, 8, or 9, or a pharmaceutically acceptable salt thereof, wherein X¹, X², X³, X⁴, and X⁵ are combined together with the carbon atom that is adjacent to X¹ and X⁵ to form a group of formula (1'): 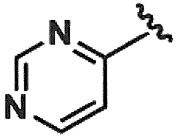 or
a group of formula (2'):

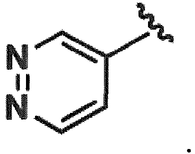

11. The compound according to any one of claims 1 to 10, or a pharmaceutically acceptable salt thereof, wherein m is 0 or 1.

12. The compound according to any one of claims 1 to 11, or a pharmaceutically acceptable salt thereof, wherein at least one of R¹ and R² is:
C₁₋₆ alkyl, wherein the alkyl may be optionally substituted with 1 or 2 substituents independently selected from the group consisting of:
(a) hydroxy,
(b) C₁₋₄ alkoxy, and
(c) C₃₋₆ cycloalkyl, or
halogen.

13. A compound selected from the group consisting of the following structural formulae: 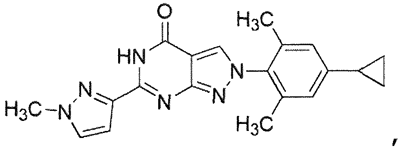 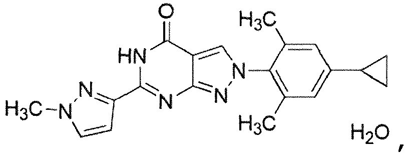 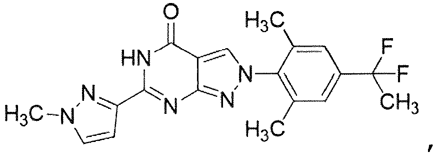 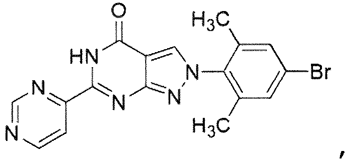 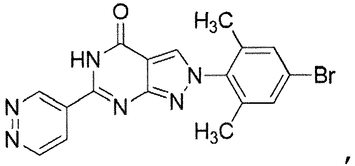 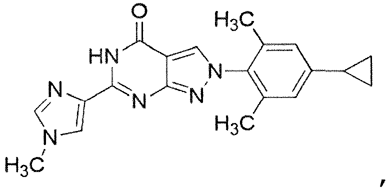 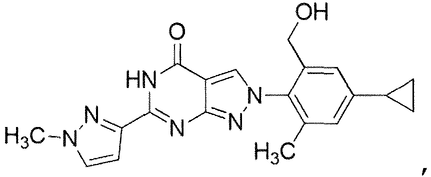 and 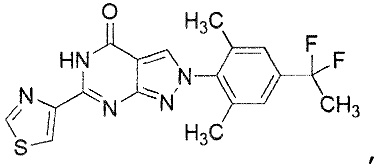 or a pharmaceutically acceptable salt thereof.

14. A pharmaceutical composition comprising a compound according to any one of claims 1 to 13, or a pharmaceutically acceptable salt thereof, and a pharmaceutically acceptable carrier.

15. An NLRP3 inflammasome inhibitor comprising a compound according to any one of claims 1 to 13, or a pharmaceutically acceptable salt thereof.

16. A medicament for treating or preventing a disease selected from the group consisting of multiple sclerosis, inflammatory bowel disease, arteriosclerosis, Cryopyrin-associated periodic syndrome, nonalcoholic steatohepatitis, gout, ischemic heart disease, Alzheimer's disease, Parkinson's disease, Huntington's disease, amyotrophic lateral sclerosis, and traumatic brain injury, comprising a compound according to any one of claims 1 to 13, or a pharmaceutically acceptable salt thereof.

17. The medicament according to claim 16, wherein the inflammatory bowel disease is ulcerative colitis or Crohn's disease.

18. The medicament according to claim 16, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

19. The medicament according to claim 16, wherein the disease is selected from the group consisting of multiple sclerosis, Alzheimer's disease, Parkinson's disease, Huntington's disease, amyotrophic lateral sclerosis, and traumatic brain injury.

20. A method for inhibiting NLRP3 inflammasome, comprising administering a therapeutically effective amount of a compound according to any one of claims 1 to 13, or a pharmaceutically acceptable salt thereof, to a mammal.

21. A method for treating or preventing a disease selected from the group consisting of multiple sclerosis, inflammatory bowel disease, arteriosclerosis, Cryopyrin-associated periodic syndrome, nonalcoholic steatohepatitis, gout, ischemic heart disease, Alzheimer's disease, Parkinson's disease, Huntington's disease, amyotrophic lateral sclerosis, and traumatic brain injury, comprising administering a therapeutically effective amount of a compound according to any one of claims 1 to 13, or a pharmaceutically acceptable salt thereof, to a mammal.

22. The method according to claim 21, wherein the inflammatory bowel disease is ulcerative colitis or Crohn's disease.

23. The method according to claim 21, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

24. The method according to claim 21, wherein the disease is selected from the group consisting of multiple sclerosis, Alzheimer's disease, Parkinson's disease, Huntington's disease, amyotrophic lateral sclerosis, and traumatic brain injury.

25. Use of a compound according to any one of claims 1 to 13, or a pharmaceutically acceptable salt thereof, in the manufacture of an NLRP3 inflammasome inhibitor.

26. Use of a compound according to any one of claims 1 to 13, or a pharmaceutically acceptable salt thereof, in the manufacture of a medicament for treating or preventing a disease selected from the group consisting of multiple sclerosis, inflammatory bowel disease, arteriosclerosis, Cryopyrin-associated periodic syndrome, nonalcoholic steatohepatitis, gout, ischemic heart disease, Alzheimer's disease, Parkinson's disease, Huntington's disease, amyotrophic lateral sclerosis, and traumatic brain injury.

27. The use according to claim 26, wherein the inflammatory bowel disease is ulcerative colitis or Crohn's disease.

28. The use according to claim 26, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

29. The use according to claim 26, wherein the disease is selected from the group consisting of multiple sclerosis, Alzheimer's disease, Parkinson's disease, Huntington's disease, amyotrophic lateral sclerosis, and traumatic brain injury.

30. A compound according to any one of claims 1 to 13, or a pharmaceutically acceptable salt thereof, for use in inhibiting NLRP3 inflammasome.

31. A compound according to any one of claims 1 to 13, or a pharmaceutically acceptable salt thereof, for use in treating or preventing a disease selected from the group consisting of multiple sclerosis, inflammatory bowel disease, arteriosclerosis, Cryopyrin-associated periodic syndrome, nonalcoholic steatohepatitis, gout, ischemic heart disease, Alzheimer's disease, Parkinson's disease, Huntington's disease, amyotrophic lateral sclerosis, and traumatic brain injury.

32. The compound, or a pharmaceutically acceptable salt thereof, for use according to claim 31, wherein the inflammatory bowel disease is ulcerative colitis or Crohn's disease.

33. The compound, or a pharmaceutically acceptable salt thereof, for use according to claim 31, wherein the Cryopyrin-associated periodic syndrome is familial cold autoinflammatory syndrome, Muckle-Wells syndrome, chronic infantile neurologic cutaneous and articular syndrome, or neonatal onset multisystem inflammatory disease.

34. The compound, or a pharmaceutically acceptable salt thereof, for use according to claim 31, wherein the disease is selected from the group consisting of multiple sclerosis, Alzheimer's disease, Parkinson's disease, Huntington's disease, amyotrophic lateral sclerosis, and traumatic brain injury.
